(19) Europäisches Patentamt
European Patent Office
Office européen des brevets

(11) EP 3 074 032 B1

(12) **EUROPEAN PATENT SPECIFICATION**

(45) Date of publication and mention of the grant of the patent:
**26.12.2018 Bulletin 2018/52**

(21) Application number: **14821058.6**

(22) Date of filing: **26.11.2014**

(51) Int Cl.:
***A61K 38/17*** *(2006.01)*

(86) International application number:
**PCT/DK2014/050402**

(87) International publication number:
**WO 2015/078477 (04.06.2015 Gazette 2015/22)**

# (54) FATTY ACID DERIVATIVES OF DIMERIC INHIBITORS OF PSD-95

FETTSÄUREDERIVATE VON DIMEREN PSD-95-INHIBITOREN

DÉRIVÉS ACIDES GRAS D'INHIBITEURS DIMÈRES DE PSD-95

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.12.2013 DK 201370735**

(43) Date of publication of application:
**05.10.2016 Bulletin 2016/40**

(73) Proprietor: **University of Copenhagen**
**1165 Copenhagen K (DK)**

(72) Inventors:
- **STRØMGAARD, Kristian**
  **Brookline, MA 02445 (US)**
- **BACH, Anders**
  **DK-2500 Valby (DK)**
- **NISSEN, Klaus Bertram**
  **DK-5792 Årslev (DK)**

(74) Representative: **Høiberg P/S**
**Adelgade 12**
**1304 Copenhagen K (DK)**

(56) References cited:
**WO-A2-2010/004003**

- **A. BACH ET AL: "A high-affinity, dimeric inhibitor of PSD-95 bivalently interacts with PDZ1-2 and protects against ischemic brain damage", PROCEEDINGS OF THE NATIONAL ACADEMY OF SCIENCES, vol. 109, no. 9, 28 February 2012 (2012-02-28), pages 3317-3322, XP055023131, ISSN: 0027-8424, DOI: 10.1073/pnas.1113761109**
- **JESPER T. ANDREASEN ET AL: "UCCB01-125, a dimeric inhibitor of PSD-95, reduces inflammatory pain without disrupting cognitive or motor performance: Comparison with the NMDA receptor antagonist MK-801", NEUROPHARMACOLOGY, vol. 67, 1 April 2013 (2013-04-01), pages 193-200, XP055167375, ISSN: 0028-3908, DOI: 10.1016/j.neuropharm.2012.11.006**
- **LIM SUNG IN ET AL: "Site-specific fatty acid-conjugation to prolong protein half-lifein vivo", JOURNAL OF CONTROLLED RELEASE, vol. 170, no. 2, 2 June 2013 (2013-06-02), pages 219-225, XP028676591, ISSN: 0168-3659, DOI: 10.1016/J.JCONREL.2013.05.023**
- **KLAUS B. NISSEN ET AL: "Design, Synthesis, and Characterization of Fatty Acid Derivatives of a Dimeric Peptide-Based Postsynaptic Density-95 (PSD-95) Inhibitor", JOURNAL OF MEDICINAL CHEMISTRY, 15 January 2015 (2015-01-15), XP055167308, ISSN: 0022-2623, DOI: 10.1021/jm501755d**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 074 032 B1

SEQUENCE LISTING

<110> University of Copenhagen

<120> Fatty acid derivatives of dimeric inhibitors of PSD-95

<130> P3416PC00

<160> 6

<170> PatentIn version 3.5

<210> 1
<211> 4
<212> PRT
<213> Artificial Sequence

<220>
<223> X is independently any amino acid

<220>
<221> SITE
<222> (1)..(4)
<223> Peptide 1

<220>
<221> SITE
<222> (1)..(1)
<223> X4 in description; amino acid residue selected from E, Q, A, N and S

<220>
<221> SITE
<222> (2)..(2)
<223> X3 in description; amino acid residue selected from S and T

<220>
<221> SITE
<222> (3)..(3)
<223> X2 in description; amino acid residue selected from A, D, E, Q, N, S, V, N-Me-A, N-Me-D, N-Me-E, N-Me-Q, N-Me-N, N-Me-S and N-Me-V

<220>
<221> SITE
<222> (4)..(4)
<223> X1 in description; amino acid residue selected from I, L and V

<400> 1

**Xaa Xaa Xaa Xaa**
**1**

<210> 2
<211> 4
<212> PRT
<213> Artificial Sequence

<220>
<223> X is independently any amino acid

<220>
<221> SITE
<222> (1)..(4)
<223> Peptide 2

<220>
<221> SITE
<222> (1)..(1)
<223> Z4 in description; amino acid residue selected from E, Q, A, N and S

<220>
<221> SITE
<222> (2).. (2)
<223> Z3 in description; amino acid residue selected from S and T

<220>
<221> SITE
<222> (3)..(3)
<223> Z2 in description; amino acid residue selected from A, D, E, Q, N, S, V, N-Me-A, N-Me-D, N-Me-E, N-Me-Q, N-Me-N, N-Me-S and N-Me-V

<220>
<221> SITE
<222> (4)..(4)
<223> Z1 in description; amino acid residue selected from I, L and V

<400> 2

```
Xaa Xaa Xaa Xaa
1
```

<210> 3
<211> 5
<212> PRT
<213> Artificial Sequence

<220>
<223> specific peptide

<220>
<221> PEPTIDE
<222> (1)..(5)
<223> Specific peptide

<400> 3

```
Ile Glu Thr Ala Val
1               5
```

<210> 4
<211> 5
<212> PRT
<213> Artificial Sequence

<220>
<223> specific peptide

<220>
<221> PEPTIDE
<222> (1)..(5)
<223> specific peptide

<400> 4

```
Ile Glu Thr Asp Val
1               5
```

<210> 5

<211> 5
<212> PRT
<213> Artificial Sequence

<220>
<223> X is independently any amino acid

<220>
<221> SITE
<222> (1)..(1)
<223> X5 in description; any amino acid residue

<220>
<221> SITE
<222> (2)..(2)
<223> X4 in description; amino acid residue selected from E, Q, A, N and S

<220>
<221> SITE
<222> (3)..(3)
<223> X3 in description; amino acid residue selected from S and T

<220>
<221> SITE
<222> (4)..(4)
<223> X2 in description; amino acid residue selected from A, D, E, Q, N, S, V, N-Me-A, N-Me-D, N-Me-E, N-Me-Q, N-Me-N, N-Me-S and N-Me-V

<220>
<221> SITE
<222> (5)..(5)
<223> X1 in description; amino acid residue selected from I, L and V

<400> 5

**Xaa Xaa Xaa Xaa Xaa**
**1               5**

<210> 6
<211> 5
<212> PRT
<213> Artificial Sequence

<220>
<223> X is independently any amino acid

<220>
<221> SITE
<222> (1)..(1)
<223> Z5 in description; any amino acid residue

<220>
<221> SITE
<222> (2).. (2)
<223> Z4 in description; amino acid residue selected from E, Q, A, N and S

<220>
<221> SITE

<222> (3)..(3)
<223> Z3 in description; amino acid residue selected from S and T

<220>
<221> SITE
<222> (4)..(4)
<223> Z2 in description; amino acid residue selected from A, D, E, Q, N, S, V, N-Me-A, N-Me-D, N-Me-E, N-Me-Q, N-Me-N, N-Me-S and N-Me-V

<220>
<221> SITE
<222> (5)..(5)
<223> Z1 in description; amino acid residue selected from I, L and V

<400> 6

```
Xaa Xaa Xaa Xaa Xaa
1               5
```

## Description

### Technical field

The present disclosure relates to compounds capable of binding to the PDZ domains of PSD-95 and their medical use as inhibitors of protein-protein interaction mediated by PSD-95.

### Background

Postsynaptic density protein-95 (PSD-95) is a protein encoded in humans by the DLG4 (disks large homolog 4) gene. PSD-95 is a member of the membrane-associated guanylate kinase (MAGUK) family and is together with PSD-93 recruited into the same NMDA receptor and potassium channel clusters.

PSD-95 is the best studied member of the MAGUK family of PDZ domain-containing proteins. Like all MAGUK family proteins, it includes three PDZ domains, an SH3 domain, and a guanylate kinase-like domain (GK) connected by linker regions. It is almost exclusively located in the postsynaptic density of neurons, and is involved in anchoring synaptic proteins. Its direct and indirect binding partners include neuroligin, neuronal nitric oxide synthase (nNOS), N-methyl-D-aspartate (NMDA) receptors, AMPA receptors, and potassium channels.

The PDZ domain is a common structural domain of 80-90 amino-acids predominantly found in scaffolding proteins of various organisms including humans. PDZ is an acronym for the first letters of three proteins-PSD-95, Drosophila disc large tumor suppressor (Dlg1), and Zonula occludens-1 protein (ZO-1)-which were the first proteins discovered comprising the domain.

In general, PDZ domains interact with other proteins by binding to their C-terminus. This is achieved by β-sheet augmentation, meaning that the β-sheet in the PDZ domain is extended by the addition of the C-terminal tail of the binding partner protein and thus forming an extended β-sheet like structure.

PDZ domains are found in a wide range of proteins both in the eukaryotic and eubacteria kingdoms, whereas there are very few examples of the protein in archaea. The three PDZ domains of PSD-95, PDZ1-3, bind peptide ligands with similar consensus sequence such as Ser/Thr-X-Val/Ile/Leu-COOH.

The structural basis for the interaction of PDZ domains with C-terminal peptides was first elucidated by an X-ray crystallographic structure of PDZ3 of PSD-95 complexed with a native peptide ligand, CRIPT (Sequence: YKQTSV). PDZ3 contains six antiparallel β-strands (βA-βF) and two α-helices (αA and αB), and the C-terminal peptide ligand binds into a groove between the βB strand and αB helix. Two residues in the peptide ligand are considered particularly important for affinity and specificity, the first ($P^0$) and the third ($P^{-2}$) amino acids as counted from the C-terminus. The side chain of the amino acid in $P^0$ position projects into a hydrophobic pocket and an amino acid with an aliphatic side chains (Val, Ile and Leu) is required. In the PDZ3-CRIPT X-ray crystal structure, the hydroxyl oxygen of Ser or Thr ($P^{-2}$) forms a hydrogen bond with the nitrogen of an imidazole side chain of His372, and this interaction has been shown to be an important determinant for the affinity of the PDZ domain/ligand interaction. A conserved Gly-Leu-Gly-Phe (position 322-325 in PDZ3) motif and a positively charged residue (Arg318 in PSD-95 PDZ3) of PDZ domains mediate binding to the C-terminal carboxylate group.

The PDZ1 and PDZ2 domains of PSD-95 interact with several proteins including the simultaneous binding of the NMDA-type of ionotropic glutamate receptors and the nitric oxide (NO) producing enzyme nNOS. NMDA receptors are the principal mediators of excitotoxicity, i.e. glutamate-mediated neurotoxicity, which is implicated in neurodegenerative diseases and acute brain injuries, and although antagonists of the NMDA receptor efficiently reduce excitotoxicity by preventing glutamate-mediated ion-flux, they also prevent physiological important processes. Thus NMDA receptor antagonists have failed in clinical trials for e.g. stroke due to low tolerance and lack of efficacy. Instead, specific inhibition of excitotoxicity can be obtained by perturbing the intracellular nNOS/PSD-95/NMDA receptor complex using PSD-95 inhibitors.

PSD-95 simultaneously binds the NMDA receptor, primarily GluN2A and GluN2B subunits, and nNOS via PDZ1 and PDZ2, respectively. Activation of the NMDA receptor causes influx of calcium ions, which activates nNOS thereby leading to NO generation. Thus, PSD-95 mediates a specific association between NMDA receptor activation and NO production, which can be detrimental for the cells if sustained for a longer period, and is a key facilitator of glutamate-mediated neurotoxicity. Inhibition of the ternary complex of nNOS/PSD-95/NMDA receptor interaction by targeting PSD-95 is known to prevent ischemic brain damage in mice, by impairing the functional link between calcium ion entry and NO production, while the physiological function, such as ion-flux and pro-survival signaling pathways of the NMDA receptor remains intact. WO 2010/004003 discloses a concept of inhibiting PSD-95 by dimeric peptide ligands linked by a polyethylene glycol linker (PEG). These dimers simultaneously bind to the PDZ1 and PDZ2 domains of PSD-95.

Lim et al. (Journal of Controlled Release, 2013, 170, 2, 219-225) relates to conjugation of fatty acids to small therapeutic peptides to prolong serum half-life, and discloses site-specific fatty acid-conjugation to a permissive site of a protein, using copper-catalyzed alkyne-azide cycioaddition, by linking a fatty acid derivative to p-ethynylphenylalanine

incorporated into a protein using an engineered pair of yeast tRNA/aminoacyl tRNA synthetase.

Dimeric ligands targeting PSD-95 are under pre-clinical evaluation as a treatment for chronic pain (Andreasen et al, Neuropharmacol, 2013, 67, 193-200; Bach et al, PNAS USA, 2012, 109, 3317-3322). However, therapeutic peptides are generally susceptible to removal from the blood and degradation by renal clearance and hepatic metabolism. Therefore there is a need for improving the pharmacokinetic properties and thus increase stability and half-life of the dimeric peptide ligands.

## Summary of the disclosure

In order to address the stated problem of providing improved pharmacokinetic properties and increased *in vivo* stability of dimeric peptide ligands of PSD-95, the present disclosure describes a new class of compounds wherein two peptide ligands are linked by a linker such as *N*PEG linker, wherein one or more fatty acids or fatty acid derivatives have been conjugated either directly to the *N*PEG linker or via a further linker.

The present inventors have therefore developed derivatives of dimeric PSD-95 ligands having improved *in vitro* plasma half-lives compared to compounds without fatty acids attached, e.g. the compounds disclosed in WO2010/004003. Furthermore, the compounds show increased residence time in a subcutaneous depot upon subcutaneous administration.

In one aspect of the present disclosure concerns a compound comprising a first peptide ($P_1$) and a second peptide ($P_2$), wherein $P_1$ and $P_2$ individually comprise at least two proteinogenic or non-proteinogenic amino acid residues, and wherein both $P_1$ and $P_2$ are conjugated to a first linker $L_1$ via their respective N-termini, and wherein $L_1$ comprises polyethylene glycol (PEG) wherein at least one oxygen atom of said PEG is substituted with a nitrogen atom to give *N*PEG, and wherein an albumin binding moiety is linked to the nitrogen atom of the *N*PEG by an amide bond, or via an optional linker $L_2$.

It has been demonstrated that the compounds of the present disclosure bind to PDZ1-2 of PSD-95 when electing $P_1$ and $P_2$ as defined herein. As PSD-95 is an important target for therapeutics, the present disclosure in one aspect concerns the compound as defined herein for use as a medicament.

More specifically, the compounds of the disclosure may in one aspect be used for the treatment or prophylaxis of an excitotoxic-related disease, or for prophylaxis and/or treatment of pain.

The compounds of the present disclosure can schematically be synthesized by a method comprising the steps of:

a) preparing a Ns-*N*PEG diacid linker,
b) preparing a peptide using Fmoc-based solid-phase peptide synthesis,
c) dimerizing Fmoc-deprotected peptide with Ns-*N*PEG diacid linker
d) coupling a fatty acid to the linker-dimer conjugate, optionally via an intermediate linker, such as an amino acid linker ($L_2$).

## Description of the drawings

Figure 1: Structure of reference ligands, UCCB01-125 and UCCB01-144. Capital letters indicate L-amino acids, except for 'N' (nitrogen), 'O' (oxygen).

Figure 2: Affinity for HSA of FA-linked dimeric ligands (**1-12**) and UCCB01-125 and UCCB01-144. Data shown as mean $\pm$SEM, n=3.

Figure 3: Affinity to PSD-95 PDZ1-2 of FA-linked dimeric ligands (**1-12**) and UCCB01-125 and UCCB01-144 as determined by FP. A) Measured in TBS; B) Measure in TBS + HSA; C) Measured in TBS + HSA corrected for fu. Data shown as mean $\pm$ SEM, n$\geq$3.

Figure 4: In vitro plasma stability of compounds (UCCB01-125, **1**, **4**, **7**, **13**). Calculated half-lives are: UCCB01-125: 1.7 h; **1:** 23.6 h; **4**, **7**, and **13:** >24 h.

Figure 5: Plasma profiles of dimeric ligands UCCB01-125 (two doses) and compound **1**, **4**, **7** and **13** after s.c. administration in rats.

Figure 6: Synthesis of FA-linked dimeric ligands (**1-12**). The reaction conditions of the synthesis illustrated in scheme 1 of this figure was as follows: (a) Fmoc-GABA-OH/Fmoc-(L)-Glu-OtBu/Fmoc-5-Ava, HATU, collidine, DMF (1h x

2), then 20% piperidine in DMF; (b) FA1/FA2/FA3/FA4, HBTU, DIPEA, DMF/DCM, 45 min, then TFA/TIPS/$H_2O$ (90/5/5); (c) 0.5M LiOH, $H_2O$/ACN (75/25), 30 min, then TFA to pH<2. Triangle indicates that E and T are side-chain protected (tert-butyl).

Figure 7: Mono saponification of octadecandioate dimethyl ester. Reaction conditions: (a) NaOH (1eq.), MeOH, 45°C, O/N.

**Detailed description of the disclosure**

The invention is defined by the appended claims. Any embodiment not falling under the scope of the appended claims does not form part of the invention.

**I. Definitions**

Amide bond: The term 'amide bond' as used herein is a chemical bond formed by a reaction between a carboxylic acid and an amine (and concomitant elimination of water). Where the reaction is between two amino acid residues, the bond formed as a result of the reaction is known as a peptide linkage (peptide bond);

Comprising: The term 'comprising' as used herein should be understood in an inclusive manner. Hence, by way of example, a composition comprising compound X, may comprise compound X and optionally additional compounds.

Dimer: The term dimer as used herein refers to two identical or non-identical chemical moieties associated by chemical or physical interaction. By way of example, the dimer can be a homodimer such as two identical peptides linked by a linker. The dimer may also be a heterodimer such as two different peptides linked by a linker. An example of a dimer is the PSD-95 inhibitor of the present disclosure which is a compound comprising two peptide or peptide analogues, that are covalently linked by means of a linker, wherein the peptides or peptide analogues are capable of binding to, or interacting with, PDZ1 and PDZ2 of PSD-95 simultaneously.

Dipeptide: The term 'dipeptide' as used herein refers to two natural or non-natural amino acids linked by a peptide bond.

Ethylene glycol moiety: The term 'ethylene glycol moiety' as used herein refers to the structural unit that constitutes a PEG or *N*PEG linker. Another name of an 'ethylene glycol moiety' is 'oxyethylene', and the chemical formula of the monomer unit is:

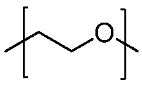

Fatty acid: The term fatty acid (abbreviated FA) as used herein typically refers to a carboxylic acid with a long aliphatic carbon chain, which can be either saturated or unsaturated. The fatty acid can be selected from Short-chain fatty acids (SCFA), Medium-chain fatty acids (MCFA), Long-chain fatty acids (LCFA) and Very long chain fatty acids (VLCFA). Short-chain fatty acids (SCFA) are fatty acids with aliphatic tails of fewer than six carbons (i.e. butyric acid). Medium-chain fatty acids (MCFA) are fatty acids with aliphatic tails of 6-12 carbons, which can form medium-chain triglycerides. Long-chain fatty acids (LCFA) are fatty acids with aliphatic tails 13 to 21 carbons. Very long chain fatty acids (VLCFA) are fatty acids with aliphatic tails longer than 22 carbons. The fatty acid of the present disclosure can be any suitable fatty acid or fatty acid derivative known by those of skill in the art.

Linker: The term 'linker' as used herein refers to one or more atoms forming a connection from one chemical entity to another. By way of example, the 'first linker' referred to herein is a PEG or *N*PEG, which joins the two PDZ-domain binding peptides by forming a link to each of their *N*-termini.

Non-proteinogenic amino acids: Non-proteinogenic amino acids also referred to as non-coded, non-standard or non-natural amino acids are amino acids which are not encoded by the genetic code. A non-exhaustive list of non-proteinogenic amino acids include α-amino-n-butyric acid, norvaline, norleucine, isoleucine, alloisoleucine, tert-leucine, α-amino-n-heptanoic acid, pipecolic acid, α,β-diaminopropionic acid, α,γ-diaminobutyric acid, ornithine, allothreonine, homocysteine, homoserine, β-alanine, β-amino-n-butyric acid, β-aminoisobutyric acid, γ-aminobutyric acid, α-aminoisobutyric acid, isovaline, sarcosine, N-ethyl glycine, N-propyl glycine, N-isopropyl glycine, N-methyl alanine, N-ethyl alanine, N-methyl β-alanine, N-ethyl β-alanine, isoserine and α-hydroxy-γ-aminobutyric acid.

*N*PEG: The term *N*PEG as used herein is a linker derivative of a PEG linker, but where one or more of the backbone oxygen atoms is replaced with a nitrogen atom.

Ns-*N*PEG diacid linker: The 'Ns-*N*PEG diacid linker' is the structure where an *N*PEG linker is protected on the nitrogen with an ortho-nitrobenzenesulfonyl (Ns) protection group on the linker nitrogen, and where the termini of the *N*PEG linker comprise carboxylic acids. This chemical reagent or building block is used to dimerize the two peptide

moieties, $P_1$ and $P_2$.

PDZ: The term 'PDZ' as used herein refers to Postsynaptic density protein-95 (PSD-95), Drosophila homologue discs large tumor suppressor (DlgA), Zonula occludens-1 protein (zo-1).

PEG: The term 'PEG' as used herein refers to a polymer of the ethylene glycol moiety discussed herein above. PEG has the chemical formula $C_{2n+2}H_{4n+6}O_{n+2}$, and the repeating structure is:

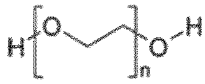

where for example 12 PEG moieties, or PEG12, corresponds to a polymer of 12 ethylene glycol moieties.

Pharmacokinetic profile: The term pharmacokinetic profile' as used herein refers to the *in vivo* characteristics of absorption into the blood stream, distribution into tissues, metabolization and excretion of the compounds described herein. An example of a parameter that is included in the pharmacokinetic profile is the *in vitro* half plasma half-life, which models the metabolization of the compounds by plasma proteases.

Proteinogenic amino acids: Proteinogenic amino acids, also referred to as natural amino acids include alanine, cysteine, selenocysteine, aspartic acid, glutamic acid, phenylalanine, glycine, histidine, isoleucine, lysine, leucine, methionine, asparagine, proline, pyrrolysine, glutamine, arginine, serine, threonine, valine, tryptophan and tyrosine.

PSD-95: The term 'PSD-95' as used herein refers to postsynaptic density protein-95.

PSD-95 inhibitor: The term 'PSD-95 inhibitor' as used herein refers to a compound that binds to PDZ1, PDZ2, or both PDZ1 and PDZ2 of PSD-95 and inhibits the protein-protein interactions facilitated by these PDZ domains in a cell. An example of an interaction that is inhibited by a PSD-95 inhibitor is the ternary complex formation between nNOS, PSD-95 and the NMDA receptor.

## II. Dimeric compounds with improved plasma half-life

Dimeric ligands targeting PSD-95 are under pre-clinical evaluation as a treatment for chronic pain and ischemic stroke (Andreasen et al, Neuropharmacol, 2013, 67, 193-200; Bach et al, PNAS USA, 2012, 109, 3317-3322). However, therapeutic peptides in general are susceptible to degradation by proteases and elimination by renal filtration and/or hepatic metabolism (Tang et al, J Pharm Sci, 2004, 93, 2184-204). Due to the limited size of the dimeric ligands, they are likely to be cleared from the blood by renal filtration, since the kidneys generally filter out compounds with a molecular weight below 60 kDa (Dennis et al, J Biol Chem 2002, 277, 35035-43). To make dimeric peptide ligands more suitable for clinical utility in a chronic setting such as neuropathic pain, the dosing regimen should be as simple as possible, preferably once-daily, to increase compliance (Claxton et al, Clin Ther 2001, 23, 1296-310). Furthermore, self-administration by the patient via appropriate routes, such as subcutaneous (s.c.) administration, is preferred over i.v. (intravenous) injection. The main limitation to s.c. administration is the requirement for a low injection volume (Dychter et al, J Infus Nurs 2012, 35, 154-60) requiring the drug to be highly potent, highly concentrated, soluble, and degraded and excreted slowly from the circulation. Furthermore, the drug should be stable in the injection depot and absorbed slowly into the circulation to protract the action of the drug (Havelund et al, Pharm Res 2004, 21, 1498-504). Thus, the pharmacokinetic profile and *in vivo* half-life of the dimeric peptide ligands should be further optimized to account for these issues.

### *Albumin binding*

Human Serum Albumin (HSA), which is the most abundant protein in human serum with 55% of the total serum protein (Elsadek et al, J Control Release 2012, 157, 4-28), offers an opportunity to solve these problems. The average blood concentration of HSA is 520-830 μM, and the molecular weight is approximately 66.5 kDa (Kragh-Hansen et al, Biol Pharm Bull 2002, 25, 695-704). HSA is abundant in blood, muscular tissue and skin (Sleep et al, Biochim Biophys Acta 2013, 1830, 5526-34), but not present inside neurons under normal conditions. It may however enter neurons in disease states where the brain-blood barrier (BBB) is compromised, such as stroke (Løberg, APMIS 1993, 101, 777-83). HSA serves as a transport and depot protein for numerous endogenous ligands such as fatty acids (FAs), hemin, bilirubin and tryptophan (Simard et al, PNAS USA, 2005, 102, 17958-63; Kragh-Hansen et al, Biol Pharm Bull 2002, 25, 695-704) and drugs such as warfarin and diazepam as well as metal ions (Yamasaki et al, Biochim Biophys Acta 2013, 1830, 5435-43).

The structure of HSA has been studied extensively, and more than 90 different x-ray structures of HSA are deposited in the Protein Data Bank (PDB, accessed 26/09/2013) with 71 different ligands. HSA is a heart-shaped molecule with approximate dimensions of 80 x 80 x 30 Å and consists of three similar domains (I, II and III), that are further divided into two subdomains (a and b) (Sugio et al, Protein Eng 1999, 12, 439-46). The majority of drugs bind in

Sudlow's Site I (also called the Warfarin site) and II (also called the Diazepam site) (Elsadek et al, J Control Release 2012, 157, 4-28; Yamasaki et al, Biochim Biophys Acta 2013, 1830, 5435-43), named after the pioneering work of Sudlow and colleagues who in 1975 identified the sites by fluorescence spectroscopy (Sudlow et al., Mol Pharmacol 1975, 11, 824-32). The two drug binding sites have since been mapped and are found within subdomain IIa (with contribution from residues in subdomains IIIa and IIb) and IIIa, respectively (Yamasaki et al, Biochim Biophys Acta 2013, 1830, 5435-43). One important exception to the general binding of ligands in Sudlow's site I and II are the fatty acids (FAs).

The concept that high HSA binding of a drug increases the half-life of the drug has been known since the 1970's. However, the specific concept of using HSA to increase the half-life of therapeutic peptides and proteins has evolved more slowly, with a doubling in the number of yearly publications from 2002 (~250 publications/year) to 2010 (~500 publications/year) (Elsadek et al, J Control Release 2012, 157, 4-28). Several peptide-based HSA binding moieties have been described, including HSA-binding sequences identified by phage-display (Dennis et al, J Biol Chem 2002, 277, 35035-43), isolated from natural sources (Jonsson et al, Protein Eng Des Sel 2008, 21, 515-27 and so-called adnectins (Lipovsek et al, Protein Eng Des Sel 2011, 24, 3-9). These may however be susceptible to protease degradation, which may be particularly a problem in the current case of s.c. administration, where the developed compounds are supposed to reside in the s.c. depot for several hours.

*Overall structure*

In order to improve the pharmacokinetic profile the present inventors investigate the influence of fatty acids and linker types on HSA affinity, affinity for PSD-95 and hydrophobicity of the generated compounds. In doing so, novel compounds have been identified that provide the desired HSA-binding profile and enhanced stability in human plasma.

Thus in one aspect the present disclosure concerns a compound comprising a first peptide ($P_1$) and a second peptide ($P_2$), wherein $P_1$ and $P_2$ individually comprise at least two proteinogenic or non-proteinogenic amino acid residues, and wherein both $P_1$ and $P_2$ are conjugated to a first linker $L_1$ via their N-termini, and wherein $L_1$ comprises polyethylene glycol (PEG) wherein at least one oxygen atom of said PEG is substituted with a nitrogen atom to give *N*PEG, and wherein an albumin binding moiety is linked to the nitrogen atom of the *N*PEG by an amide bond, or via an optional linker $L_2$.

In certain embodiments of the present disclosure, said compound are of the general formula (I):

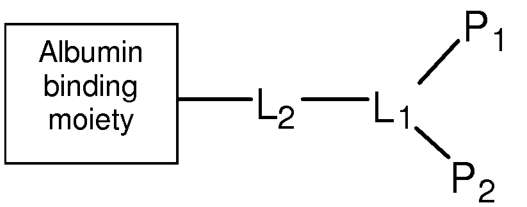

Formula (I)

While the albumin binding moiety can be any suitable chemical group binding albumin, it is preferred that the albumin binding moiety is a fatty acid (FA). In one embodiment of the present disclosure, the compound thus has the general formula (II):

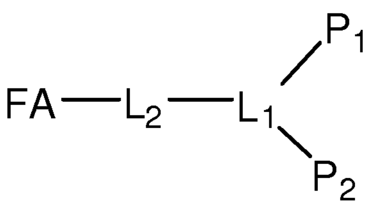

Formula (II)

The fatty acid can be any suitable fatty acid such as a saturated or an unsaturated fatty acid. As illustrated in formulas (I) and (II) above, the albumin binding moiety such as the fatty acid, may optionally be linked to the nitrogen atom of an *N*PEG linker ($L_1$) via a second linker $L_2$. In embodiments of the present disclosure wherein the second linker $L_2$ is included, that linker comprises a nitrogen atom.

In one embodiment the compound according to the present disclosure has the generic structure of formula (III) or (IV):

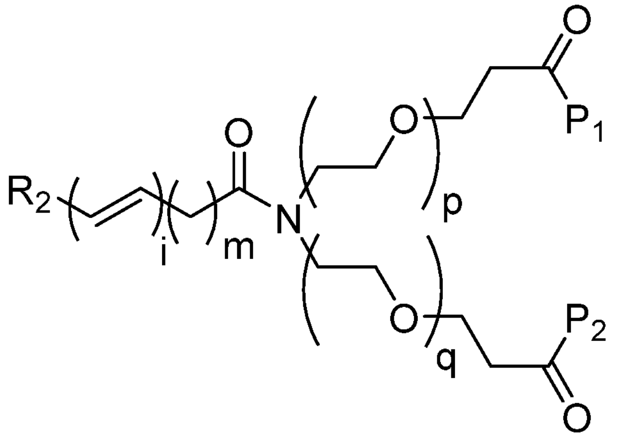

Formula (III)

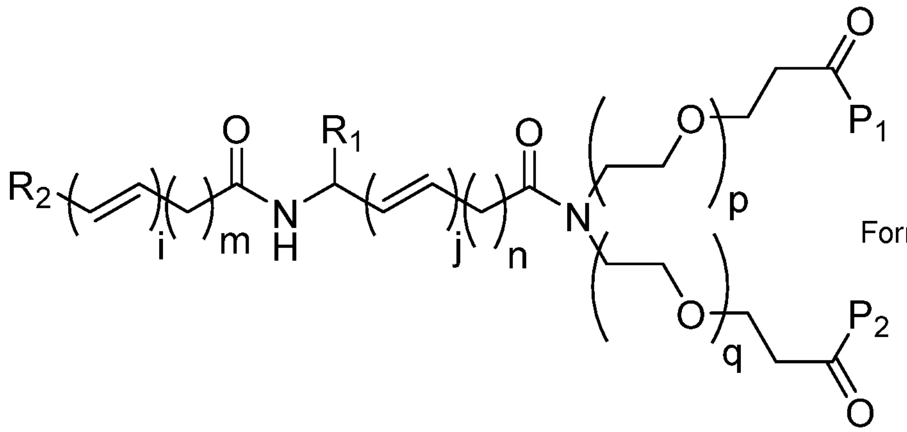

Formula (IV)

wherein

$R_1$ and $R_2$ individually are selected from the group consisting of H and COOH,
n is an integer 0 to 48,
m is an integer 1 to 48,
p is an integer 0 to 28,
q is an integer 0 to 28,
i is an integer 0 to 12,
j is an integer 0 to 12

and wherein $P_1$ and $P_2$ are individually selected from peptides comprising at least two proteinogenic or non-proteinogenic amino acid residues.

*The first linker ($L_1$)*

The properties exhibited by the fatty acid on the active peptides $P_1$ and $P_2$ are dependent on the manner in which these moieties are linked. The linking is achieved via the first linker $L_1$ and the second linker $L_2$. The first linker $L_1$, which to some extent has been described in WO 2012/156308, consists of a number of ethylene glycol moieties forming a polyethylene glycol, wherein one of the oxygen atoms has been replaced by a nitrogen atom to form an *N*PEG linker. The *N*PEG linker can be illustrated as a nitrogen atom flanked on each side by a number (p, q) of ethylene glycol moieties.

The number of ethylene glycol moieties flanking the nitrogen atom can be varied in different embodiments of the present disclosure. In one embodiment of the present disclosure, the number of ethylene glycol moieties (p) on one side is equal to the number of ethylene glycol moieties on the opposite side, i.e. p = q. In other embodiments of the present disclosure, p > q or p < q.

In one embodiment of the present disclosure, the sum of p and q is an integer between 0 and 28, such as wherein the number of ethylene glycol moieties, p is selected from 0, 1, 2, 3, 4, 5, 6, 7, 8, 9, 10, 11, 12, 13, 14, 15, 16, 17, 18, 19, 20, 21, 22, 23, 24, 25, 26, 27 or 28.

In one embodiment of the present disclosure, the number of ethylene glycol moieties, p is 1 to 4, as that range of p provides the highest affinity towards PSD-95.

In one embodiment of the present disclosure, the number of ethylene glycol moieties, q is selected from 0, 1, 2, 3, 4, 5, 6, 7, 8, 9, 10, 11, 12, 13, 14, 15, 16, 17, 18, 19, 20, 21, 22, 23, 24, 25, 26, 27 or 28 ethylene glycol moieties.

In one embodiment of the present disclosure, the number of ethylene glycol moieties, q is 1 to 4, as that range of q provides the highest affinity towards PSD-95.

In one embodiment of the present disclosure, the total number of ethylene glycol moieties p + q is between 2 and 12, as linker length within that range provides the highest affinity towards PSD-95.

In one embodiment of the present disclosure, the number of ethylene glycol moieties, p + q is 4, as linker length of that range provides the very highest affinity towards PSD-95.

In another embodiment of the present disclosure, the number of ethylene glycol moieties, p + q is 6, as linker length of that range provides a very high affinity towards PSD-95.

*The second linker ($L_2$)*

The second linker $L_2$ is optional and can be included or excluded depending on the physical or chemical properties required for a particular purpose. When present, the second linker $L_2$ comprises a nitrogen atom. The second linker $L_2$ can e.g. be selected from the group consisting of γ-Glu, γ-butyric acid (GABA), 5-amino valeric acid (5-Ava), proteinogenic amino acids, non-proteinogenic amino acids, and any compound having the general formula $H_2N$-[Q]-COOH, wherein Q is any suitable atom or atoms or molecule.

As mentioned herein above, the second linker may comprise a repetitive carbon moiety thus forming e.g. an alkyl or an alkenyl chain. The number of repetitive units (i) and/or (m) can be varied depending on the desired properties. Thus i and/or m are integers which individually can be selected from the group consisting of 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 and 48.

In one embodiment of the present disclosure, n is an integer between 1 and 3 such as in an embodiment of the present disclosure where n=1 or n=2 or n=3.

In certain embodiments of the present disclosure, i and/or j is an integer between 0 and 12, e.g. an integer selected from the group consisting of 0, 1, 2, 3, 4, 5, 6, 7, 8, 9, 10, 11 and 12.

In certain embodiments of the present disclosure, the linkers $L_1$ and $L_2$ have been elected so that the compound according to the disclosure is selected from the group consisting of:

a) KBN41 derivative

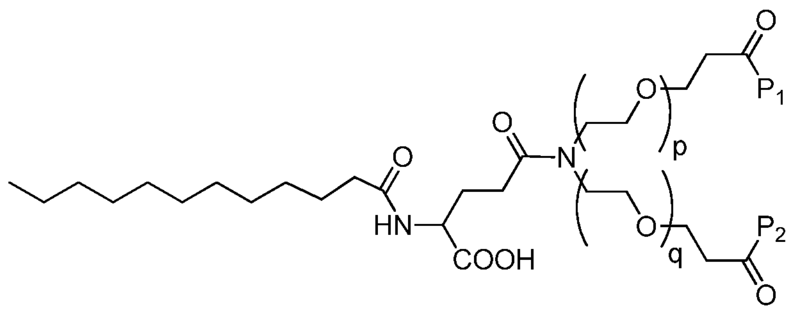

b) KBN42 Derivative

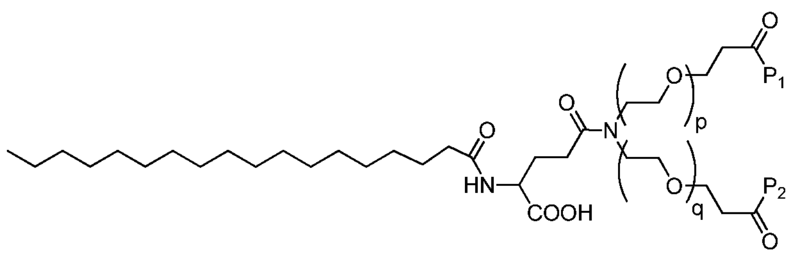

c) KBN43 Derivative

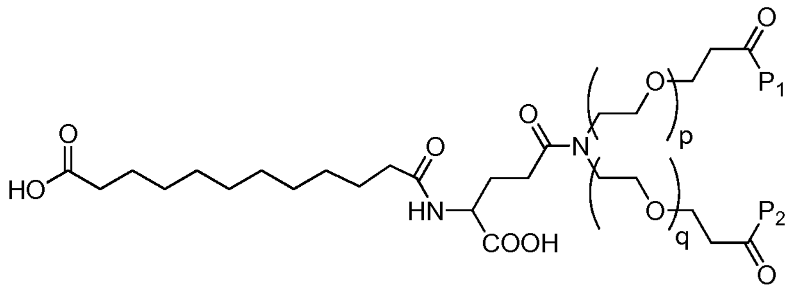

d) KBN44 Derivative

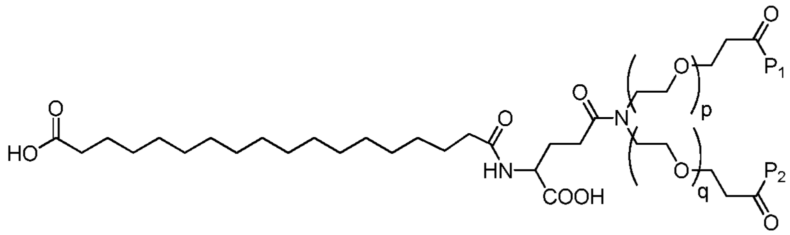

e) KBN45 Derivative

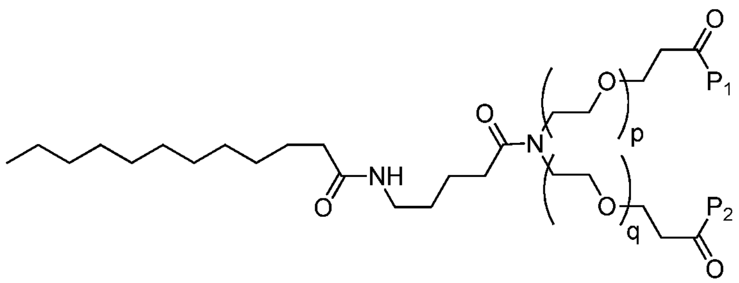

f) KBN46 Derivative

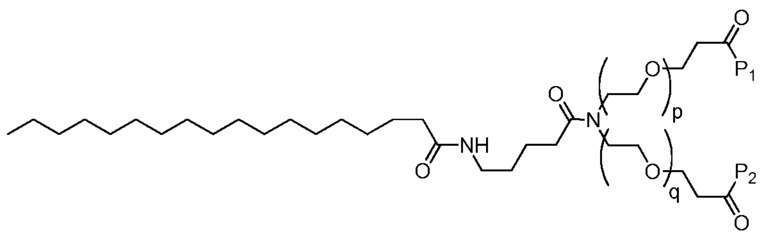

g) KBN47 Derivative

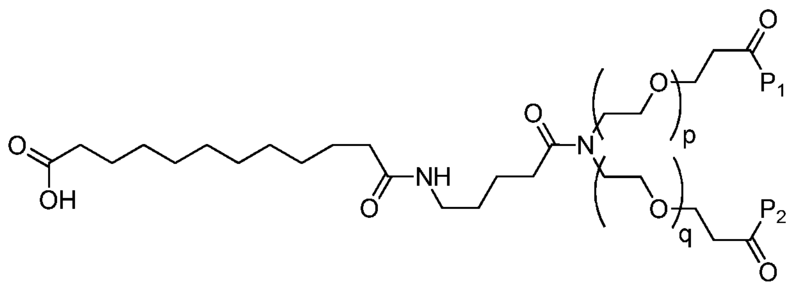

h) KBN48 Derivative

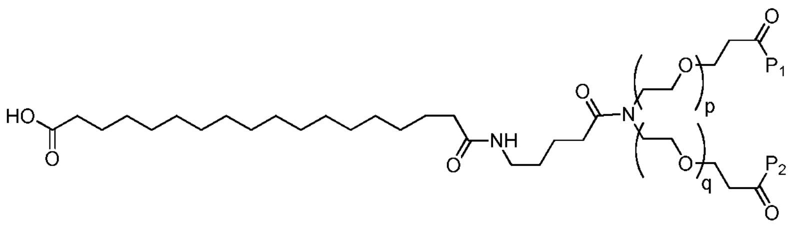

i) KBN52 Derivative

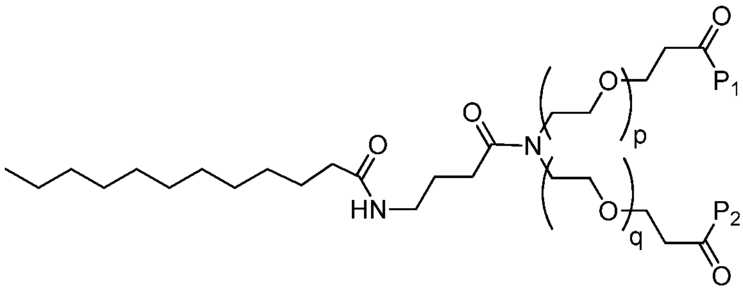

j) KBN53 Derivative

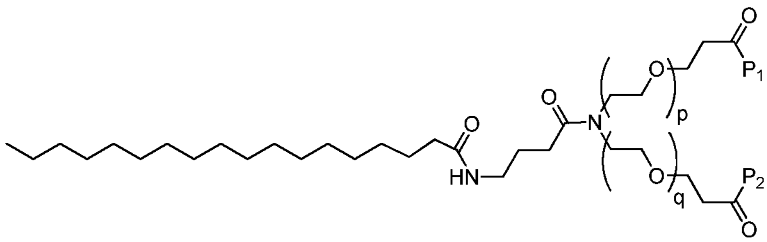

k) KBN54 Derivative

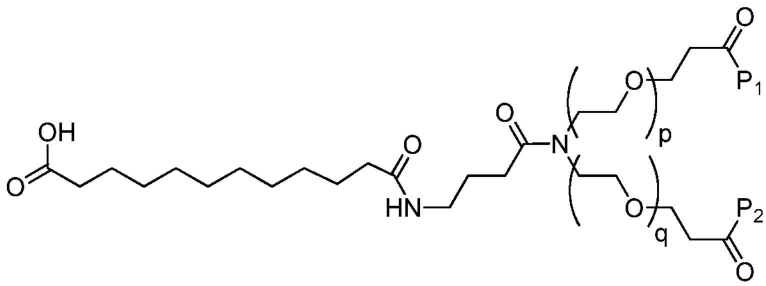

l) KBN55 Derivative

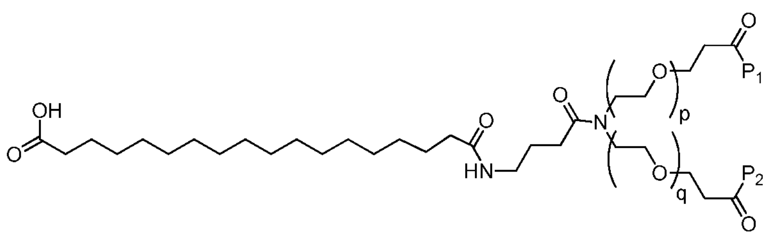

In a further embodiment of the present disclosure, the linkers have been elected so that the compound of the

disclosure is selected from the group consisting of:

a) KBN41 derivative

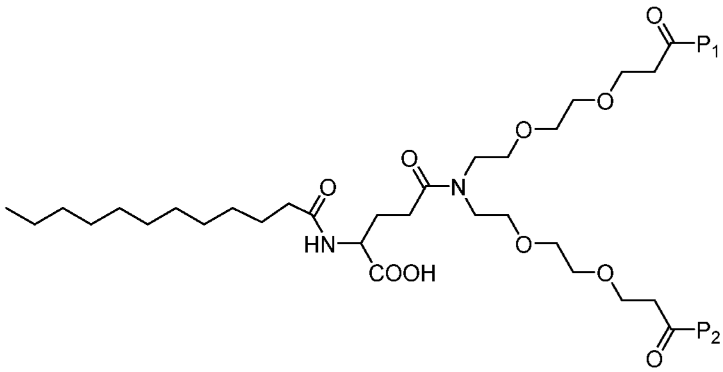

b) KBN42 Derivative

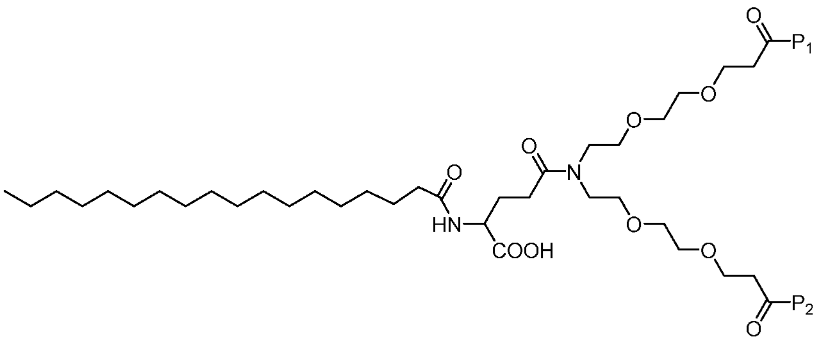

c) KBN43 Derivative

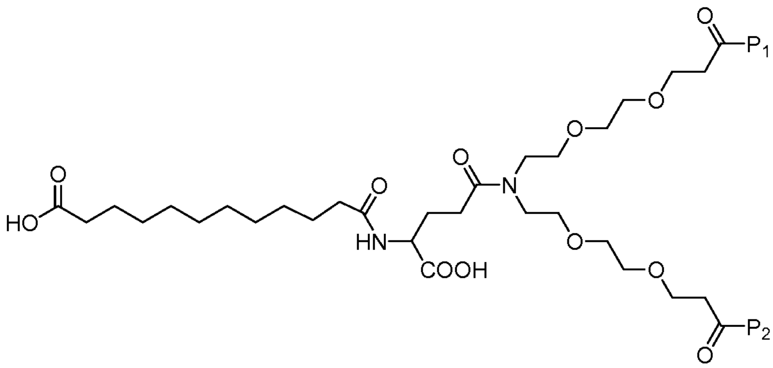

d) KBN44 Derivative

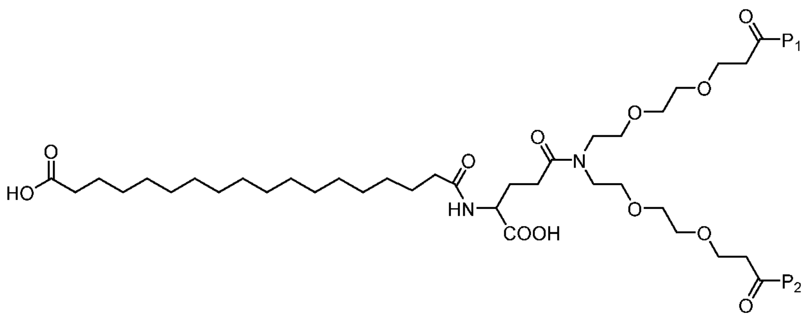

e) KBN45 Derivative

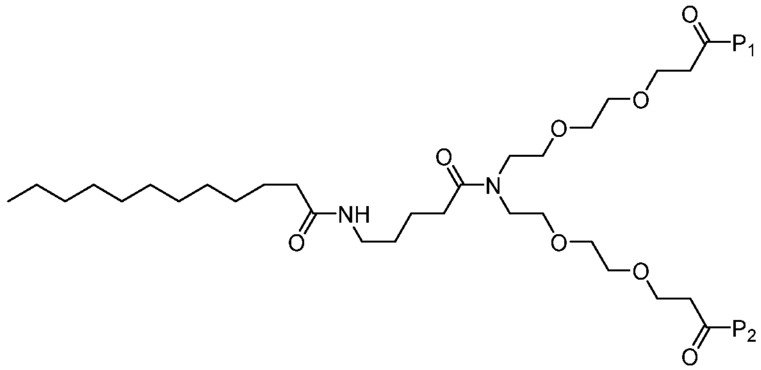

f) KBN46 Derivative

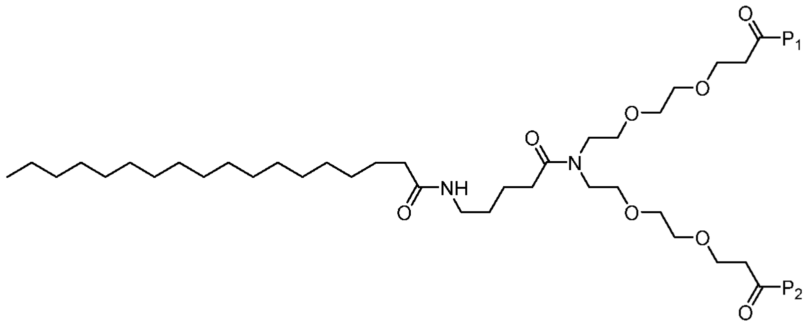

g) KBN47 Derivative

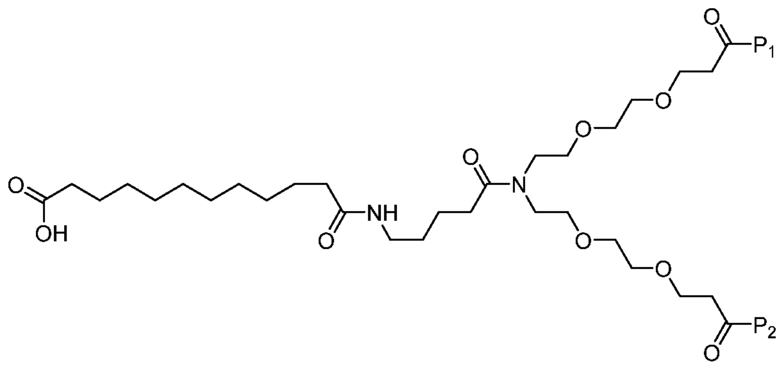

h) KBN48 Derivative

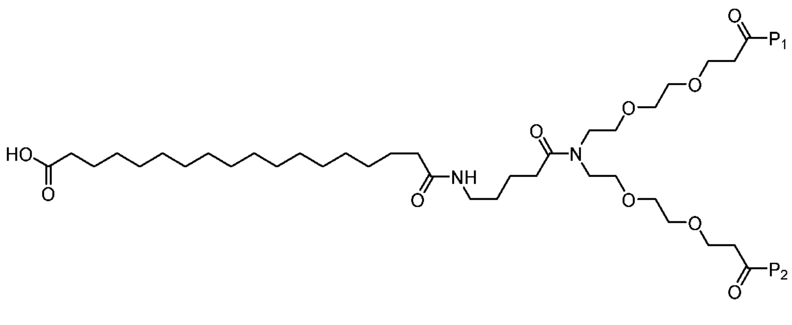

i) KBN52 Derivative

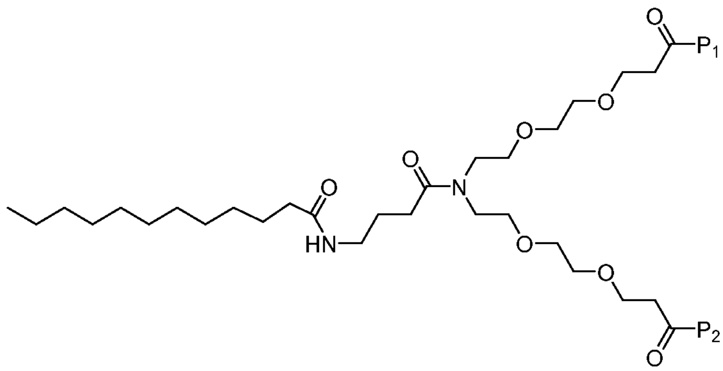

j) KBN53 Derivative

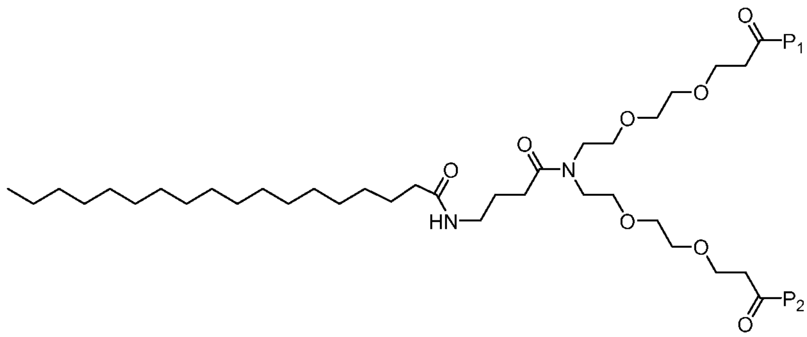

k) KBN54 Derivative

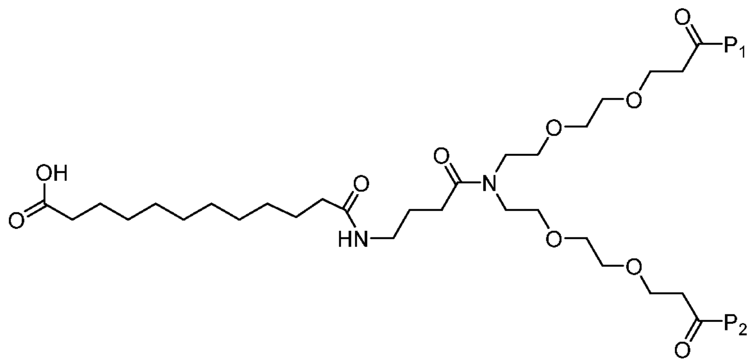

l) KBN55 Derivative

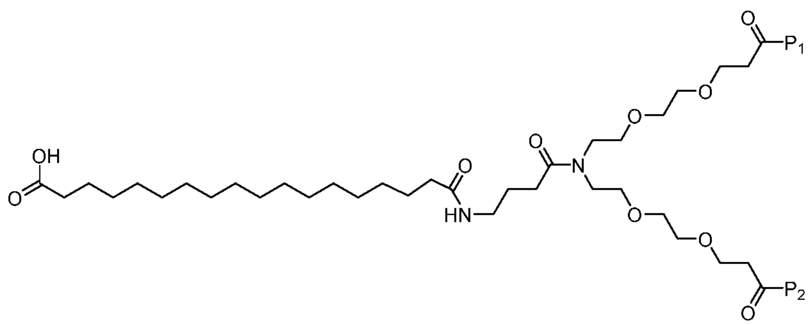

*Fatty acid (FA)*

A fatty acid is a carboxylic acid with an aliphatic tail (chain), which is either saturated or unsaturated. Most naturally occurring fatty acids have a chain of an even number of carbon atoms, from 4 to 28. Fatty acids are usually derived from triglycerides or phospholipids. When they are not attached to other molecules, they are known as free fatty acids.

Fatty acids that have carbon-carbon double bonds are known as unsaturated fatty acids while fatty acids without double bonds are known as saturated. Unsaturated fatty acids have one or more double bonds between carbon atoms. In certain embodiments of the present disclosure, the compound of the present disclosure comprises an unsaturated fatty acid. In such embodiments the indicator (i) and/or (j) of generic formulas (III), (IV), (V) or (VI) is an integer individually selected from an integer between 0 and 12, such as an integer selected from the group consisting of 0, 1, 2, 3, 4, 5, 6, 7, 8, 9, 10, 11 and 12.

Unsaturated fatty acids are available in either cis or trans configuration, or in a mixture of both.

A cis configuration means that adjacent hydrogen atoms are on the same side of the double bond. The rigidity of the double bond freezes its conformation and, in the case of the cis isomer, causes the chain to bend and restricts the conformational freedom of the fatty acid. The more double bonds the chain has in the cis configuration, the less flexibility it has. When a chain has many double bonds with cis configuration, it becomes quite curved in its most accessible conformations. For example, oleic acid, with one double bond, has a "kink" in it, whereas linoleic acid, with two double bonds, has a more pronounced bend. $\alpha$-Linolenic acid, with three double bonds, favors a hooked shape. The effect of this is that, in restricted environments, such as when fatty acids are part of a phospholipid in a lipid bilayer, or triglycerides in lipid droplets, cis double bonds limit the ability of fatty acids to be closely packed, and therefore could affect the melting temperature of the membrane or of the fat.

A trans configuration, by contrast, means that the next two hydrogen atoms are bound to opposite sides of the double bond. As a result, they do not cause the chain to bend much, and their shape is similar to straight saturated fatty acids.

It is within the scope of the present disclosure to elect any fatty acid suitable for the intended purpose including cis, trans or mixed fatty acids.

The compound according to the present disclosure may comprise any suitable fatty acid or fatty acid derivative. In one embodiment of the present disclosure, the fatty acid is a fatty acid as defined in generic formulas (III) or (IV) wherein m is an integer selected from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 and 48 such as wherein m is an integer between 10 and 16, e.g. wherein m=10 or wherein m=16, which without doubt ascertain a high degree of HSA interaction (Table 1).

The fatty acid of the disclosure may be a $C_4$-$C_{22}$ fatty acid or a fatty acid selected from the group consisting of caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, $\alpha$-linolenic acid, arachidonic acid, eicosapentaenoic acid, erucic acid and docosahexaenoic acid.

*Peptides ($P_1$ and $P_2$)*

The compound of the present disclosure comprises two peptides $P_1$ and $P_2$.

$P_1$ and $P_2$ may individually be any peptide, each comprising at least two amino acid residues and the dimeric compound of the present disclosure may thus be adapted for the intended purpose.

In a preferred embodiment of the present disclosure, the compound of the present disclosure is a PSD-95 inhibitor wherein the two peptides bind to PDZ1-2 of PSD-95. Thus, in certain embodiments the compound is a compound as defined in any one of the generic formulas (I), (II), (III) or (IV), wherein:

$P_1$ comprises the amino acid sequence $X_4X_3X_2X_1$ (SEQ ID NO: 1), and
$P_2$ comprises the amino acid sequence $Z_4Z_3Z_2Z_1$ (SEQ ID NO: 2),

wherein

a) $X_1$ and/or is an amino acid residue selected from I, L and V,
b) $X_2$ and/or $Z_2$ is an amino acid residue selected from A, D, E, Q, N, S, V, *N*-Me-A, *N*-Me-D, *N*-Me-E, *N*-Me-Q, *N*-Me-N, *N*-Me-S and *N*-Me-V,
c) $X_3$ and/or $Z_3$ is an amino acid residue selected from S and T,
d) $X_4$ and/or $Z_4$ is an amino acid residue selected from E, Q, A, N and S,

wherein $X_1$ and $Z_1$ both individually represent the ultimate C-terminal amino acid residue comprising a free carboxylic acid.

Thus, in certain embodiments the compound according to the present disclosure has the generic structure of formula (V) or (VI):

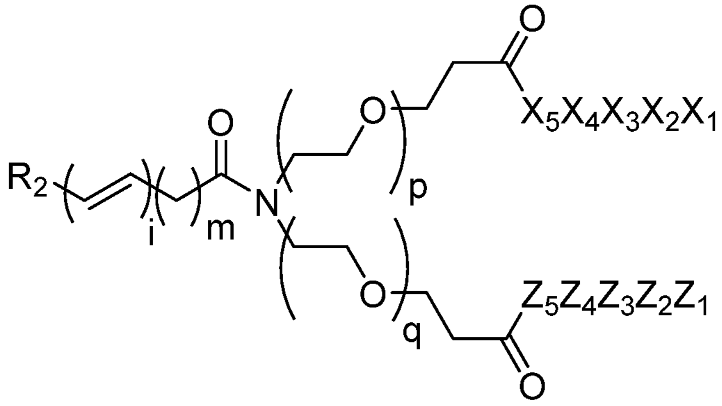

Formula (V)

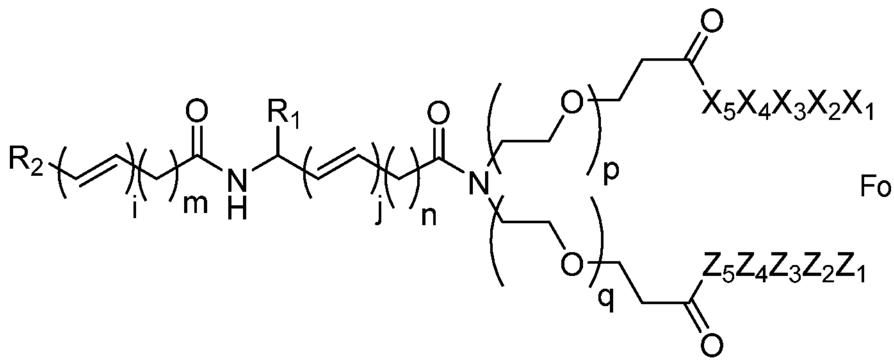

Formula (VI)

wherein

- $R_1$ and $R_2$ individually are selected from the group consisting of H and COOH,
- n is an integer 0 to 48,
- m is an integer 1 to 48, and
- p is an integer 0 to 28,
- q is an integer 0 to 28,
- i is an integer 0 to 12,
- j is an integer 0 to 12 $X_5$ and/or $Z_5$ are/is an optional amino acid residue, a peptide or a polypeptide,
- $X_4$ and/or $Z_4$ is an amino acid residue selected from E, Q, A, N and S,
- $X_3$ and/or $Z_3$ is an amino acid residue selected from S and T,
- $X_2$ and/or $Z_2$ is an amino acid residue selected from A, D, E, Q, N, S, V, *N*-Me-A, *N*-Me-D, *N*-Me-E, *N*-Me-Q, *N*-Me-N, *N*-Me-S and *N*-Me-V
- $X_1$ and/or $Z_1$ is an amino acid residue selected from I, L and V.

If $X_5$ is a single amino acid residue it is selected from proteinogenic and non-proteinogenic amino acid residues.

In one embodiment of the present disclosure, $X_5$ is an amino acid residue selected from the group consisting of I, A, L and V.

$X_5$ may also be a peptide or polypeptide having an amino acid sequence consisting of between 2 to 100 amino acid residues, wherein the C terminus of said peptide or polypeptide is an amino acid residue selected from the group consisting of I, A, L and V.

In certain embodiments of the present disclosure $X_5$ is a peptide comprising 2 to 100 residues, such as 2 to 90 amino acid residues, such as 2 to 80 amino acid residues, such as 2 to 70 amino acid residues, such as 2 to 60 amino acid residues, such as 2 to 50 amino acid residues, such as 2 to 40 amino acid residues, such as 2 to 30 amino acid residues, such as 2 to 20 amino acid residues, such as 2 to 10 amino acid residues, such as 2 to 9 amino acid residues, such as 2 to 8 amino acid residues, such as 2 to 7 amino acid residues, such as 2 to 6 amino acid residues, such as 2 to 5 amino acid residues, such as 2 to 4 amino acid residues, such as 2 to 3 amino acid residues, wherein the C terminus

is an amino acid selected from the group consisting of I, A, L and V

While the concept of the present disclosure is generally applicable as illustrated in generic formulas (I), (II), (III), (IV), (V) and (V), the present inventors have prepared a number of compounds within the present disclosure, comprising a peptide motif of $P_1$ and $P_2$ being suitable for binding to PDZ1-2 of PSD-95.

Thus in one embodiment, the compound according to the present disclosure is selected from the group consisting of:

a) KBN41 (**5**)

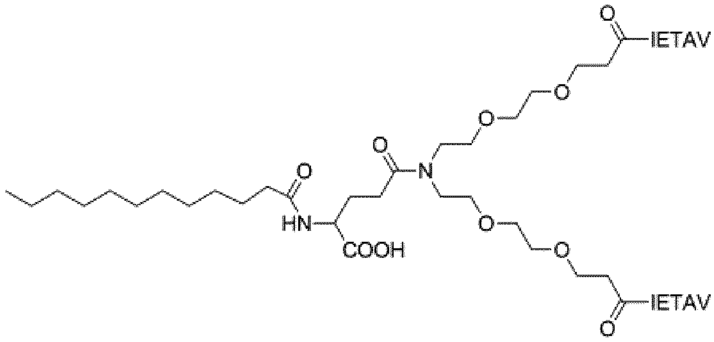

b) KBN42 (**7**)

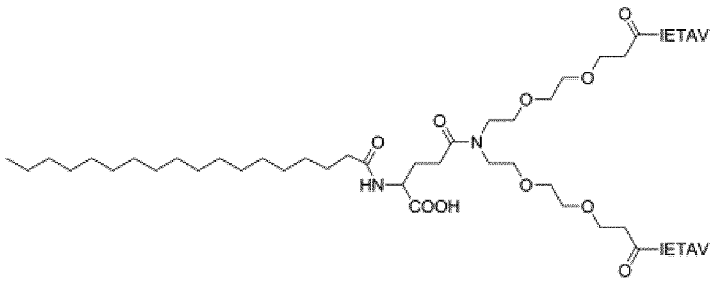

c) KBN43 (**6**)

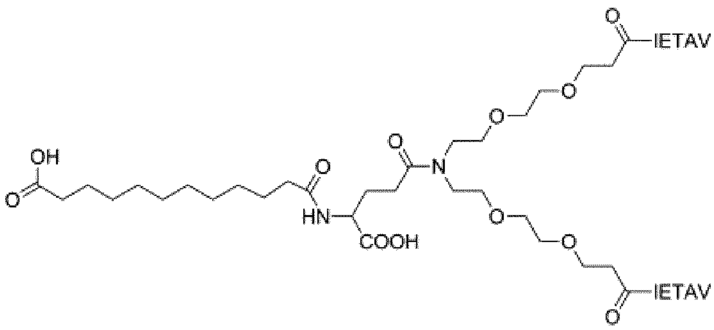

d) KBN44 (**8**)

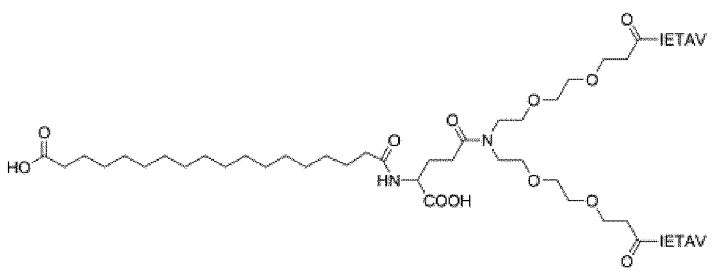

e) KBN45 (**9**)

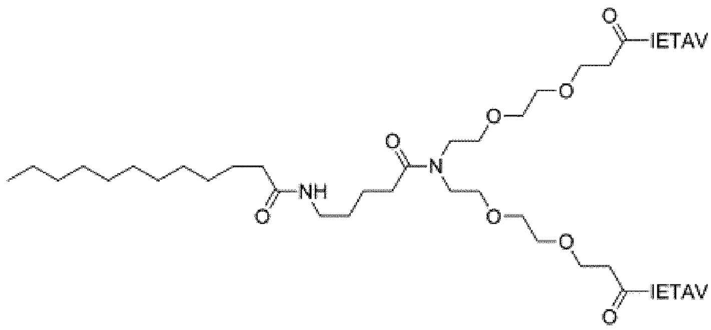

f) KBN46 (**11**)

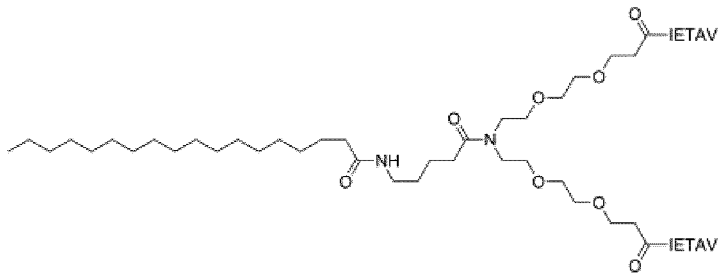

g) KBN47 (**10**)

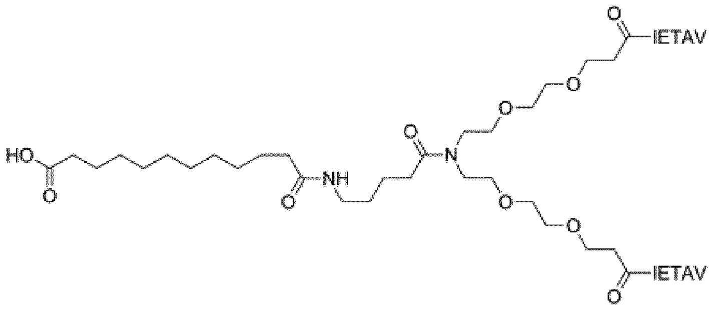

h) KBN48 (**12**)

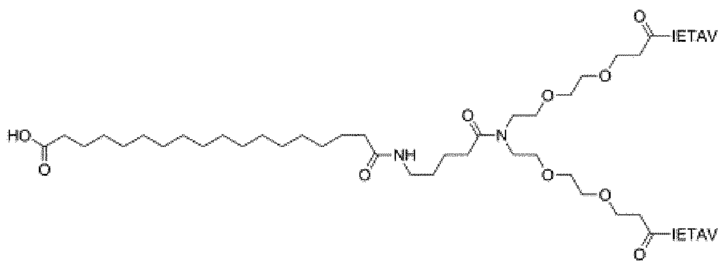

j) KBN52 (**1**)

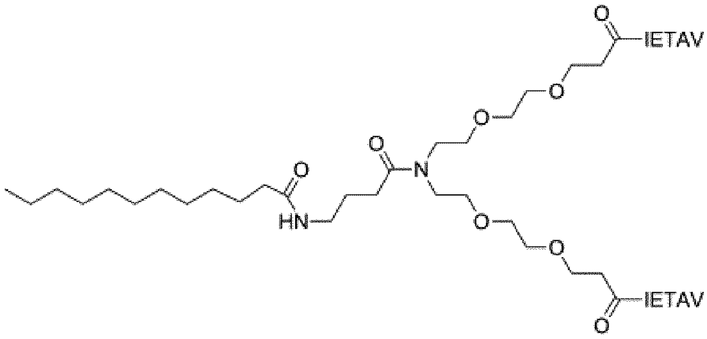

k) KBN53 (**3**)

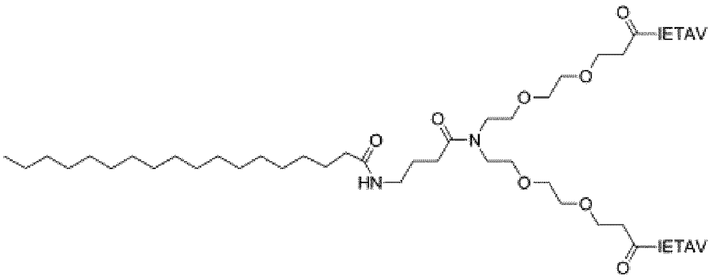

l) KBN54 (**2**)

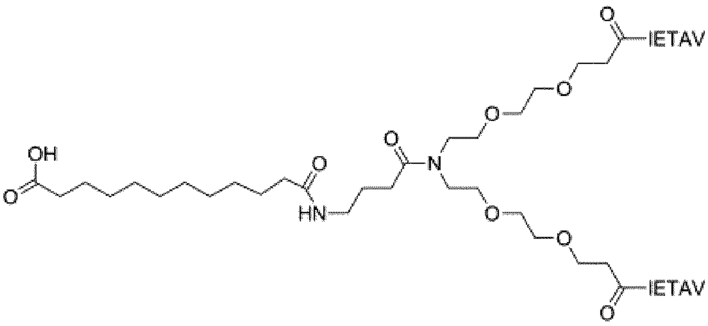

m) KBN55 (**4**)

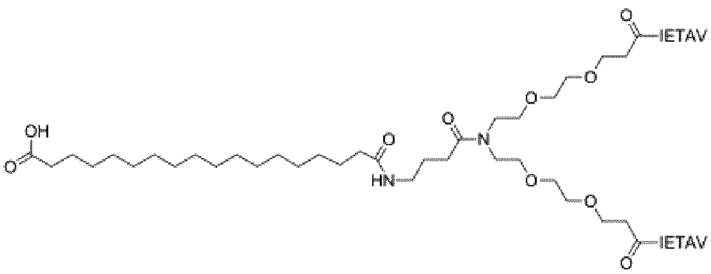

n) KBN63 (**15**)

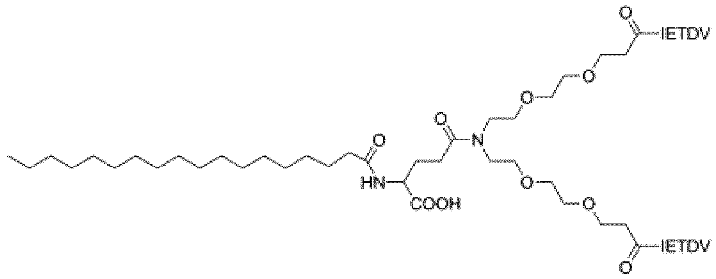

o) KBN64 (**13**)

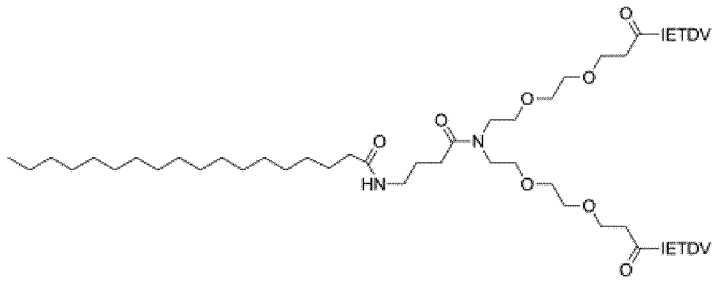

p) KBN65 (**14**)

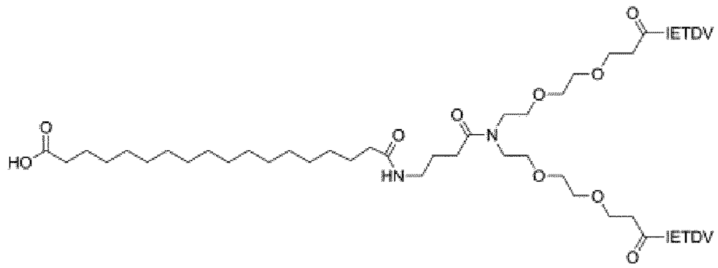

*Salt forms*

The compound as defined herein can be in the form of a pharmaceutically acceptable salt or prodrug of said compound. In one embodiment of the present disclosure the compound as defined in any one of the general formulas (I), (II), (III), (IV), (V) and (VI) can be formulated as a pharmaceutically acceptable addition salt or hydrate of said compound, such as but not limited to $K^+$, $Na^+$, as well as non-salt e.g. $H^+$.

*III. Medical use*

In one aspect the compound of the present disclosure as defined herein, is for use as a medicament.
In one embodiment of the present disclosure, the compound as defined herein is for use in the treatment or prophylaxis of pain.
In another embodiment of the present disclosure, the compound as defined herein is for use in the treatment or prophylaxis of an excitotoxic-related disease.
In a further embodiment of the present disclosure, the disease treatable by the compound of the present disclosure is ischemic or traumatic injury of the CNS.

*IV. Synthesis*

The fatty acid derivatized PSD-95 inhibitors of the present disclosure as defined herein may be manufactured by a method comprising the general steps of:

a) preparing a Ns-*N*PEG diacid linker,
b) preparing a peptide using Fmoc-based solid-phase peptide synthesis,

c) dimerizing Fmoc-deprotected peptide with Ns-*N*PEG diacid linker
d) coupling a fatty acid to the linker-dimer conjugate, optionally via an intermediate linker, such as an amino acid linker ($L_2$).

The compounds of the present disclosure can in one embodiment be synthesized as defined in the following.

Ns-*N*PEG diacid linker: The ortho-nitrobenzenesulfonyl (Ns)-protected *N*PEG linker is produced either on solid-phase or in solution.

The solid-phase procedure typically starts by loading a solid support useful for solid-phase peptide synthesis, such as 2-chlorotrityl chloride resin, with Fmoc-NH-PEG-$CH_2CH_2COOH$, using appropriate organic solvent for the specific resin (e.g. DCM, DMF, ACN, THF) and a base (e.g. DIPEA, DBU, collidine, NMM)

The Fmoc group can be removed by base (e.g. piperidine, dimethylamine, morpholine, piperazine, dicyclohexylamine, DMAP) in appropriate solvent (e.g. DMF, DCM, ACN, THF).

Ortho-nitrobenzenesulfonyl chloride can be coupled to the free amine using base (e.g. DIPEA, DBU, collidine, NMM) and appropriate solvent (e.g. THF, DCM) to get Ns-NH-PEG-$CH_2CH_2COO$-Resin.

The second part of the linker product can be connected to the resin-bound linker-part by the use of Mitsunobu-chemistry. Resin is treated with triphenylphosphine, HO-PEG-$CH_2CH_2COO$*t*Bu, solvent, and ester- or amide reagents of azodicarboxylic acid (e.g. diisopropyl azodicarboxylate, DIAD; diethyl azodicarboxylate, DEAD; 1,1'-(Azodicarbonyl)-dipiperidine, ADDP).

The final Ns-*N*PEG diacid linker is obtained by treating the resin with acid, such as trifluoroacetic acid (TFA).

The solution-phase procedure can be performed by protection of the amine group of $NH_2$-PEG-$CH_2CH_2COO$*t*Bu with Ns, followed by Mitsunobu chemistry in solution using triphenylphosphine and DIAD, DEAD, or ADDP, or similar reagents, HO-PEG-$CH_2CH_2COO$*t*Bu, and appropriate solvent (THF, DCM). Final Ns-protected *N*PEG-linker is then obtained by treatment with acids, such as TFA.

Peptide synthesis: The peptide sequence is synthesized by Fmoc-based solid-phase peptide synthesis using a solid support, such as 2-chlorotrityl chloride resin or Wang resin, Fmoc-protected amino acids, base, coupling reagents (e.g. HBTU [*N,N,N',N'*-Tetramethyl-*O*-(1*H*-benzotriazol-1-yl)uronium hexafluorophosphate], *O*-(7-azabenzotriazol-1-yl)-*N*,*N*,*N'*,*N'*-tetramethyluronium hexafluorophosphate [HATU], PyBOB, DIC/HOBt) and solvents. Alternatively to coupling reagents, activated ester of Fmoc-protected amino acids (e.g. pentafluorophenyl, succinimide) can be used.

Dimerization: The Fmoc-deprotected resin-bound peptide is dimerized with the Ns-*N*PEG diacid linker by an on-resin dimerization process by repetitive treatments of the resin with the Ns-*N*PEG diacid linker in sub-stoichiometric amounts (e.g. 1/6), base, coupling reagent, and appropriate solvents (e.g. DMF, DCM, THF). Alternatively to coupling reagents, activated ester of the Ns-*N*PEG linker can be used.

The dimerization process can also be formed in solution using either the activated ester (e.g. pentafluorophenyl, succinimide) of the Ns-*N*PEG linker together with 1-Hydroxy-7-azabenzotriazole (HOAt) or Hydroxybenzotriazole (HOBt) and appropriate side chain-protected peptide (e.g. tert-butyl) in solvent (e.g. ACN, DMF, DCM, THF). Also, dimerization in solution can be performed using the Ns-*N*PEG diacid linker, coupling reagents (e.g. HBTU, HATU etc), base and solvents.

The Ns-group is removed by mercaptoethanol and 1,8-diazabicyclo[5.4.0]undec-7-ene (DBU) or by sodium thiophenolate.

Linker and fatty acid conjugation: The amino acid linker can be coupled to the free nitrogen of the *N*PEG-dimerized and resin-bound peptide by consecutive couplings of the Fmoc-protected linker (e.g. Fmoc-Glu-OtBu, Fmoc-GABA, Fmoc-5-Ava-OH) using coupling reagent and base for activation. Alternatively to coupling reagents, activated ester of Fmoc-protected amino acid linkers can be used. Fmoc groups are subsequently removed by deprotection methods.

The fatty acid is coupled to the linker-dimer conjugate using coupling reagent and base for activation. Alternatively, coupled as activated esters. If fatty acid contains carboxylic groups, in addition to the carboxylic group that reacts with the amine of the linker-dimer conjugate, these can be protected as esters, e.g. as methyl ester.

The fatty acid-linked dimeric ligands can be cleaved from the resin with concomitant side-chain deprotection using acids such as TFA or HCl.

Ester protection groups can be removed by stirring the cleaved products in aqueous base (e.g. NaOH, LiOH) and acetonitrile followed by acidification with TFA or HCl.

The final compound of the present disclosure is obtained by lyophilization and purification by HPLC or similar chromatographic methods.

In a further embodiment of the present disclosure, the synthesis of the compounds of the present disclosure is performed as defined in example 1.

## Examples

### Example 1: Synthesis

The resin-bound *N*PEG4 IETAV (SEQ ID NO: 3) dimeric ligand (**I1**) was synthesized as previously described (Bach et al, PNAS USA, 2012, 109, 3317-3322). From this, the appropriately protected linkers (Fmoc-GABA-OH, Fmoc-(L)-Glu-OtBu, and Fmoc-5-Ava, respectively) were attached to the nitrogen in the *N*PEG4 linker by two subsequent couplings using HATU as the coupling reagent followed by deprotection with piperidine/DMF to give Intermediates **I2-4** (Figure 6). The fatty acids (FA1-4, Figure 6) were readily attached to the liberated nitrogen in the linkers using solid phase peptide synthesis conditions and cleaved from the resin with concomitant deprotection of the side-chain protecting groups. The terminal methyl protecting group of the mono-protected FA building blocks (dodecanedioic acid methyl ester and octadecanedioic acid methyl ester, FA2 and FA4) were then removed by saponification of the cleaved product followed by acidification (Figure 6). After lyophilization, the crude products were dissolved in 100% DMSO and purified by large-scale C18 RP-HPLC. The semi-pure fractions (50-90% purity) were lyophilized, re-dissolved in DMSO/ACN/$H_2O$ and purified by preparative C4 RP-HPLC (>95% pure).

Figure 6 illustrates the synthesis of FA-linked dimeric ligands (**1-12**). The reaction conditions of scheme 1 was as follows: (a) Fmoc-GABA-OH/Fmoc-(L)-Glu-OtBu/Fmoc-5-Ava, HATU, collidine, DMF (1h x 2), then 20% piperidine in DMF; (b) FA1/FA2/FA3/FA4, HBTU, DIPEA, DMF/DCM, 45 min, then TFA/TIPS/$H_2O$ (90/5/5); (c) 0.5M LiOH, $H_2O$/ACN (75/25), 30 min, then TFA to pH<2. Triangle indicates that E and T are side-chain protected (tert-butyl).

The octadecanedioate monomethyl ester (FA4) was not commercially available and was synthesized by mono-saponification of the corresponding dimethyl ester with one eq. of NaOH as described previously (Jonassen et al, Pharm Res, 2012, 29, 2104-2114) (Figure 7).

In conclusion, example 1 demonstrates that the compounds of the present disclosure can be synthesized and obtained in pure form.

### Example 2: Method for determining affinity to HSA

The synthesized FA-linked dimeric ligands (**1-12**) were evaluated for their HSA affinity using a Transil$^{XL}$ HSA binding assay kit (Sovicell GMBH, Leipzig, Germany). The dimeric ligands, UCCB01-125 (Bach et al, Angew. Chem, Int. Ed, 2009, 48, 9685-9689) and UCCB01-144 (Bach et al, PNAS USA, 2012, 109, 3317-3322) were also tested for comparison (Table 1, Figure 1).

The assay kit consisted of prefilled wells with increasing concentrations of immobilized HSA as well as two control wells without HSA. The HSA had been immobilized in a random fashion to ensure that all binding sites on HSA were available. To conduct the assay, the wells were incubated with a known concentration of the tested compounds, and the unbound amount of compound was quantified using analytical RP-HPLC (C8 column). The fraction of unbound drug ($f_u$) for each data point was calculated from the RP-HPLC data by comparison to a control sample without HSA. The ratio of HSA-bound drug ($f_b$=1-$f_u$) to unbound drug for each data point was then plotted against the total HSA concentration ($C_{HSA}$) in the well and fitted to a linear model as given by equation 1, to give the 1/$K_D$ as the slope of the fitted curve.

$$\text{Equation 1: } \frac{f_b}{f_u} = \frac{1}{K_D} c_{HSA}$$

In eq. 1, it is assumed that the concentration of drug-bound HSA ([HSA-D]) is much lower than the total concentration of HSA in the well ([HSA-D]<<$C_{HSA}$). The assay kit has been designed such that the assumption is valid for compounds where $f_u$>1%. The calculated $K_D$-values were used to calculate $f_b$ at physiological concentration of HSA (588 μM) using equation 2.

$$f_b = 1 - \frac{1}{1 + \frac{c_{HSA}}{K_D^{HSA}}}$$

Equation 2:

In conclusion, example 3 demonstrates that and how, binding of the compounds of the present disclosure to human serum albumin (HSA) can be determined.

**Example 3: Affinity to HSA**

Dimeric ligands UCCB01-125 and UCCB01-144, which do not contain FAs, showed HSA affinities of 154.3 μM and 317.5 μM respectively, corresponding to HSA bound fractions ($f_b$) of 75% and 65% respectively (Table 1). However, all FA-linked dimeric ligands (**1-12**) showed a much higher affinity towards HSA compared to the ligands without FA and accordingly higher $f_b$ values (Table 1). Thus, this clearly demonstrates that HSA binding is greatly enhanced as a result of conjugating FA to the dimeric ligands.

Compounds containing the longer 5-Ava linker generally have slightly lower affinity for HSA than compounds with the shorter linkers (GABA, γGlu) (Table 1, Figure 2). The additional acid moiety in the γGlu linker (R1) does not seem to have any influence on the affinity for HSA of the FA-linked dimeric ligands synthesized here (Table 1, Figure 2). This is in contrast to what has been observed in other protein-ligand systems (Hackett et al, Adv Drug Deliv Rev, 2013, 65, 1331-1339) and indicates that the free carboxylate of the γGlu linker is not an essential feature for binding to HSA for the present compounds.

The dimeric ligands linked to the long FAs (m=16) show superior HSA affinity compared to the dimeric ligands linked to the shorter FAs (m=10) (Table 1); and a terminal carboxyl group (R2) has a negative influence on the HSA affinity (Table 1 and Figure 2).

Table 1: HSA binding and calculated fraction of bound compound for FA-linked dimeric ligands (**1-12**) and dimeric ligands UCCB01-125 and UCCB01-144[a]

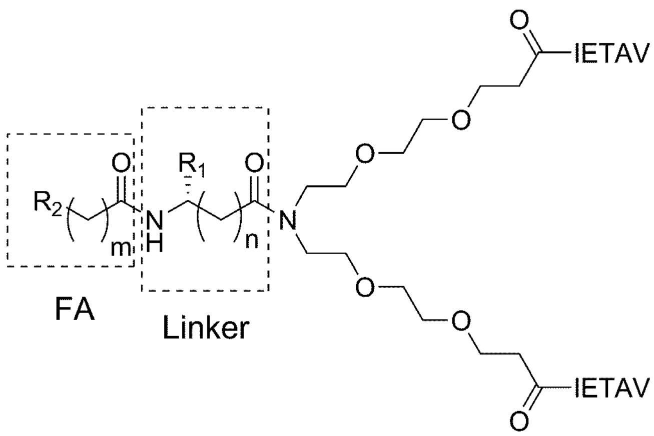

| Compound | Linker | FA | m | $R_2$ | $K_D$(HSA) (μM) | $f_b$(%) |
|---|---|---|---|---|---|---|
| **1** | GABA, n=2, $R_1$=H | C12:0 | 10 | $CH_3$ | 26.6 ± 1.1 | 95.7 ± 0.2 |
| **2** | | C11:0-COOH | 10 | COOH | 49.3 ± 4.7 | 92.3 ± 0.7 |
| **3** | | C18:0 | 16 | $CH_3$ | 4.8 ± 1.1 | 99.2 ± 0.2 |
| **4** | | C17:0-COOH | 16 | COOH | 19.4 ± 3.6 | 96.5 ± 0.6 |
| **5** | γGlu, n=2, $R_1$=COOH | C12:0 | 10 | $CH_3$ | 25.8 ± 3.2 | 95.8 ± 0.5 |
| **6** | | C11:0-COOH | 10 | COOH | 64.6 ± 9.3 | 90.1 ± 1.3 |
| **7** | | C18:0 | 16 | $CH_3$ | 6.8 ± 1.2 | 98.9 ± 0.2 |
| **8** | | C17:0-COOH | 16 | COOH | 34.3 ± 0.3 | 94.5 ± 0.1 |
| **9** | 5-Ava, n=3, $R_1$=H | C12:0 | 10 | $CH_3$ | 55.7 ± 5.2 | 91.4 ± 0.7 |
| **10** | | C11:0-COOH | 10 | COOH | 240.0 ± 11 | 71.0 ± 1.0 |
| **11** | | C18:0 | 16 | $CH_3$ | 12.7 ± 0.9 | 97.9 ± 0.2 |
| **12** | | C17:0-COOH | 16 | COOH | 23.5 ± 3.9 | 96.2 ± 0.6 |
| **UCCB01-125** | - | - | - | - | 154.3 ± 15.8 | 77.6 ± 1.8 |

(continued)

| Compound | Linker | FA | m | $R_2$ | $K_D$(HSA) (μM) | $f_b$(%) |
|---|---|---|---|---|---|---|
| **UCCB01-144** | - | - | - | - | 317.5 ± 38.6 | 65.3 ± 2.9 |
| [a]Data shown as mean ± SEM, n=3 | | | | | | |

In conclusion, example 3 demonstrates that the compounds of the present disclosure have an increased affinity for HSA as compared to non-FA derivatized dimeric reference peptides.

**Example 4: Method for determining affinity to PDZ1-2 of PSD-95:**

Affinity to PSD-95 was measured using an in vitro fluorescence polarization (FP) assay as described by Bach et al (PNAS USA, 2012, 109, 3317-3322). First, a saturation binding curve was obtained to determine $K_D$ values for the interaction between a dimeric fluorescent probe and PSD-95 PDZ1-2. Increasing concentrations of PDZ1-2 were added to a constant concentration (0.5 nM) of the probe. The fluorescence polarization of the samples was measured at excitation/emission wavelengths of 635/670 nm and the FP values were fitted to a one site binding model using the program GraphPad Prism. Then, the affinity between the non-fluorescent dimeric ligands and PDZ1-2 were determined in a heterologous competition binding assay, where increasing concentration of ligand was added to a fixed concentration of dimeric probe (0.5 nM) and PDZ1-2 (4 nM). The FP values were fitted to a one site competition (variable slope) model in GraphPad Prism. The resulting $IC_{50}$ were converted to competition inhibition constants, $K_i$ values, as described (Nikolovska-Coleska et al, Anal Biochem, 2004, 332, 261-273). The modified FP assay was conducted as described above with 1% HSA in the assay.

In conclusion, example 4 demonstrates how to test binding of the compounds of the present disclosure, to PDZ1-2 of PSD-95.

**Example 5: Affinity to PDZ1-2 of PSD-95:**

The synthesized FA-linked dimeric ligands were evaluated for their affinity to PSD-95 PDZ1-2 in the FP assay. UCCB01-125 and UCCB01-144 were used as reference compounds (Bach et al, PNAS USA, 2012, 109, 3317-3322) (Table 2, Figure 3). We first measured the affinities using a simple tris-buffered saline (TBS) buffer (Table 2, Figure 3A); but furthermore, we investigated if HSA influenced the ability of the FA-linked dimers to bind PSD95 PDZ1-2 by conducting the FP assay with HSA present in the assay buffer (Table 2, Figure 3B). Due to binding of the probe to HSA at higher concentrations, the concentration of HSA was here set to 1% (~150 μM), approximately 4 times lower than the estimated physiological blood concentration (520-830 μM) (Kragh-Hansen et al, Biol Pharm Bull 2002, 25, 695-704).

For a traditional small-molecule drug, it is commonly accepted that the unbound fraction of the drug is free to diffuse across membranes and exerts the physiological effect by interacting with its target (Berezhkovskiy et al, J Pharm Sci 2007, 96, 249-257). I.e. if the drug is bound to another molecule, then it cannot interact with the target at the same time. To account for this, the fraction of unbound drug (fu) was calculated from equation 2 (fu=1-fb) at a HSA concentration of 150 μM, and the FP assay data (TBS + HSA) were corrected for the calculated fu (Table 2, Figure 3C).

Table 2: Affinity for PSD-95 PDZ1-2 of FA-linked dimeric ligands (**1-12**) and dimeric ligands (UCCB01-125 and UCCB01-144) as determined by FP[a], calculated fraction of unbound drug ($f_u$)[b], $f_u$-corrected FP data[a] and retention time ($R_t$) of the compounds determined by RP-HPLC (C8 column)[c].

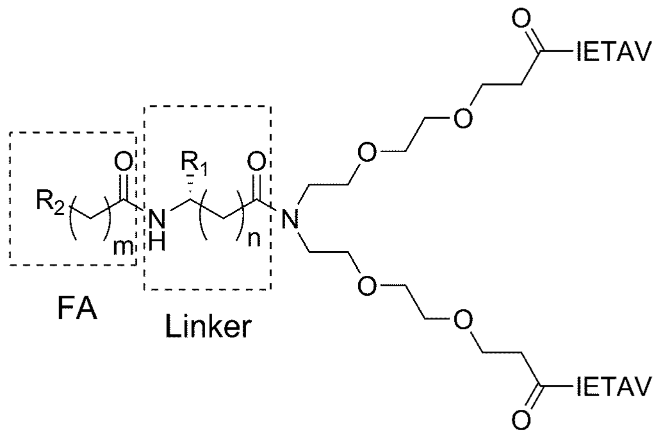

| Compound | Linker | FA | m | $R_2$ | $K_i$(PSD95) (nM) | $K_i$(PSD95) + HSA (nM) | $f_u$ (%) | $K_i$(PSD95), free ligand (nM) | $R_t$ (min) |
|---|---|---|---|---|---|---|---|---|---|
| **1** | GABA n=2, $R_1$=H | C12:0 | 10 | $CH_3$ | 13.6 ± 0.6 | 27.2 ± 2.9 | 15.1 | 3.2 ± 1.0 | 46 |
| **2** | | C11:0-COOH | 10 | COOH | 15.4 ± 1.2 | 27.9 ± 2.7 | 24.7 | 5.5 ± 0.7 | 38 |
| **3** | | C18:0 | 16 | $CH_3$ | 11.1 ± 0.6 | 1889 ± 155 | 3.1 | 57.2 ± 4.9 | 61 |
| **4** | | C17:0-COOH | 16 | COOH | 11.4 ± 0.6 | 5717 ± 298 | 11.5 | 654 ± 34 | 48 |
| **5** | γGlu, n=2, $R_1$=COOH | C12:0 | 10 | $CH_3$ | 30.2 ± 4.2 | 24.0 ± 0.6 | 14.7 | 2.4 ± 0.1 | 46 |
| **6** | | C11:0-COOH | 10 | COOH | 33.7 ± 1.6 | 26.4 ± 2.6 | 30.1 | 7.0 ± 0.9 | 37 |
| **7** | | C18:0 | 16 | $CH_3$ | 17.0 ± 0.6 | 1391 ± 184 | 4.3 | 59.1 ± 7.8 | 59 |
| **8** | | C17:0-COOH | 16 | COOH | 9.2 ± 1.6 | 3594 ± 132 | 18.6 | 669 ± 25 | 47 |
| **9** | 5-Ava, n=2, $R_1$=H | C12:0 | 10 | $CH_3$ | 20.5 ± 1.1 | 13.4 ± 1.4 | 27.1 | 2.6 ± 0.5 | 48 |
| **10** | | C11:0-COOH | 10 | COOH | 13.9 ± 2.1 | 18.5 ± 0.6 | 61.5 | 10.9 ± 0.4 | 38 |
| **11** | | C18:0 | 16 | $CH_3$ | 26.5 ± 0.2 | 1889 ± 100 | 7.8 | 16.8 ± 3.3 | 61 |
| **12** | | C17:0-COOH | 16 | COOH | 11.5 ± 1.2 | 2926 ± 335 | 8.3 | 250 ± 37 | 49 |
| **UCCB01-125** | - | - | - | - | 14.3 ± 1.1 | 9.7 ± 1.0 | 50.7 | 4.1 ± 0.5 | 28 |

(continued)

| Compound | Linker | | FA | m | $R_2$ | $K_i$(PSD95) (nM) | $K_i$(PSD95) + HSA (nM) | $f_u$ (%) | $K_i$(PSD95), free ligand (nM) | $R_t$ (min) |
|---|---|---|---|---|---|---|---|---|---|---|
| **UCCB01-144** | - | - | - | - | - | 4.3 ± 0.1 | 10.5 ± 0.9 | 67.9 | 6.7 ± 0.6 | 24 |

[a]FP data recorded in TBS and in TBS with 1% HSA.Data shown as mean ± SEM, n≥3. [b]$F_u$ calculated according to equation 2, $f_b$=1-$f_u$, [c]n=1.

The affinity for PSD-95 PDZ1-2 in TBS was comparable for all of the FA-linked dimeric ligands to the affinities of UCCB01-125 (Table 2, Figure 3A), showing that the affinity for PSD-95 PDZ1-2 was not influenced by the FA-derivatization.

The affinity for PSD-95 PDZ1-2 in TBS with 1% HSA varied significantly and systematically between the compounds (Table 2, Figure 3B). The dimeric ligands that were linked to the longer FAs (C18:0 or C17:0-COOH) generally had an apparent >50-fold lower affinity for PSD-95 PDZ1-2 than the dimeric ligands linked to the shorter FAs (C12:0 or C11:0-COOH).

When the FP data were corrected for fu (Table 2, Figure 3C), a systematic ranking of affinities for PSD-95 PDZ1-2 within each linker series was revealed. The C12:0-linked dimeric ligands (**1**, **5**, **9**) had the highest affinity, followed by C11:0-COOH (**2**, **6**, **10**), C18:0 (**3**, **7**, **11**) and C17:0-COOH (**4**, **8**, **12**).

The Fu-corrected FP data also revealed that the observed 50-fold affinity loss of the C18:0-linked dimeric ligands **3**, **7** and **11** when the FP measurement was conducted in TBS + HSA was mainly caused by a high binding of the compounds to HSA, although a 4-5 fold decrease in affinity for PSD-95 was seen for **3** and **7**, compared to the FP data recorded in TBS (**3:** $K_i$=57.2 nM vs 11.1 nM; **7:** $K_i$=59.1 nM vs 17.0 nM, Table 2) in the current case the reduction in affinity was HSA-dependent, since no decrease in affinity was seen in TBS.

The two 5-Ava-linked dimeric ligands **11** and **12** were less affected by HSA than the corresponding GABA and γGlu-linked dimeric ligands (**3**, **4**, **7** and **8**).
In conclusion, example 5 demonstrates that the compounds of the present disclosure bind to PDZ1-2 of PSD-95.

### Example 6: Hydrophobicity of FA-linked dimeric ligands

The compounds with the highest affinity for HSA (**3**, **7**, **11**) were also the most hydrophobic of the synthesized compounds as judged by the retention time ($R_t$) determined by analytical RP-HPLC (Table 2). In an attempt to increase the hydrophilicity and thus solubility of these compounds, analogues of **3** and **7** were made, where the peptide sequence was replaced with IETDV (SEQ ID NO: 4) instead of IETAV (SEQ ID NO: 3) (**13**, **15**; Table 3), as the additional charge introduced by the Asp (D) moiety could increase the hydrophilicity of the dimers. An IETDV analogue of the highest HSA affinity compound containing a terminal acid moiety (**4**) was also synthesized and tested (**14**) for comparison. These FA linked dimers were synthesized analogously to **1-12** (Figure 6) using the appropriate peptide sequence (IETDV) as starting point.

HPLC analysis revealed a minor or no decrease in $R_t$ values, and thus hydrophobicity, for IETDV-based compounds (**13-15**) relative to IETAV-based compounds (**3**, **4**, **7**); but a systematic reduction in HSA affinities were seen (Table 3). For example, **13** eluted 3 minutes earlier on the analytical RP-HPLC than the IETAV analogue (**13:** 58 min, **3:** 61 min, Table 3), but the HSA affinity was reduced (**13:** $K_D$=83.0 μM, **3:** $K_D$=4.8 μM, table 3).

Table 3: Comparison of FA-linked dimeric analogues with different peptide sequences. 3, 4 and 7 peptide sequence IETAV (SEQ ID NO: 3); 13, 14 and 15 peptide sequence IETDV (SEQ ID NO: 4).

| Compound | Linker | FA | $K_D$ (HSA) (μM) | $K_i$ (PSD-95) (nM) | $K_i$(PSD-95) + HSA (nM) | $f_u$ (%) | $K_i$(PSD-95), free ligand (nM) | $R_t$ (min) |
|---|---|---|---|---|---|---|---|---|
| **3** | GABA | C18:0 | 4.8 ± 1.1 | 11.1 ±0.6 | 1889 ± 155 | 3.1 | 57.2 ± 4.9 | 61 |
| **4** | GABA | C17:0-COOH | 19.4 ± 3.6 | 11.4 ± 0.6 | 5717 ± 298 | 11.5 | 654 ± 34 | 48 |
| **7** | γGlu | C18:0 | 6.8 ± 1.2 | 17.0 ± 0.6 | 1391 ± 184 | 4.3 | 59.1 ± 7.8 | 59 |
| **13** | GABA | C18:0 | 83.0 ± 11.0 | 8.0 ± 0.8 | 2514 ± 149 | 35.6 | 896 ± 53 | 58 |
| **14** | GABA | C17:0-COOH | 116.0 ± 7.6 | 29.3 ± 0.7 | 770 ± 27 | 43.6 | 1097 ± 65 | 47 |
| **15** | γGlu | C18:0 | 55.0 ± 3.4 | 6.7 ± 0.6 | 3806 ± 287 | 26.8 | 1022 ± 77 | 59 |

In conclusion, example 6 demonstrates that affinity for HSA is dependent not only on the fatty acid of choice, but also on the peptide sequence elected.

**Example 7: Plasma stability of compound 1, 4, 7 and 13)**

The plasma-stability of **1**, **4**, **7** and **13** was evaluated in a modified version of an *in vitro* plasma stability assay (Bach et al, Angew. Chem, Int. Ed, 2009, 48, 9685-9689). In the original procedure, the investigated compound was incubated in human plasma. Samples were then taken out at appropriate timepoints and the serum proteins were removed by precipitation with trichloroacetic acid (TCA) followed by analysis of the supernatants by RP-HPLC. The obtained peak areas were normalized to the amount at $T_0$ and fitted to a 1 st order decay model to calculate the half-life. When this method was applied to the FA-linked dimeric ligands, sample recoveries were low (<5%). This was caused by the removal of the FA-linked dimeric ligands as HSA-bound complexes during the TCA precipitation. Therefore dissolution of the sample in solid guanidine hydrochloride (GnHCl) to a final concentration of 6M was performed prior to the TCA precipitation. The purpose of this was to unfold the HSA in the sample, releasing the FA-linked dimeric ligand.

All of the FA-linked dimeric ligands were more stable in the in-vitro plasma stability assay than UCCB01-125 (Figure 4). Without being bound by theory, it is expected that this is due to the higher HSA binding of the compounds, lowering the free concentration of compound available for enzymatic digestion. The compound containing the shorter C12:0 FA (**1**) was degraded faster than the compounds containing the longer C18:0 or C17:0-COOH FA (**4**, **7**, **13**), which were highly stable. The prolonged stability is explained by the increased affinity to HSA, which prevent proteases from cleaving the dimeric peptide-based compounds, and steric hindrance mediated by the FA.

In conclusion, example 7 demonstrates a method of assessing blood plasma stability in vitro, and that the FA linked dimeric compounds of the present disclosure have increased plasma stability and half-life as compared to non-FA linked reference compounds.

**Example 8: In vivo pharmacokinetic studies**

To determine the pharmacokinetic properties of FA-linked compounds we measured the concentration of selected compounds in blood by LC-MS/MS following a single subcutaneous (s.c.) bolus injection in male Wistar rats (Figure 5). From this, it was apparent that all FA-linked dimeric ligands have longer $T_{½}$ and greater $T_{max}$ than dimeric ligand without FA (UCCB01-125) (Table 4 and Figure 5). The effect was smallest for **1**, but very noticeable for **4**, **7**, and **13** which showed $T_{1/2}$ greater than 8 hours, corresponding to a >16-fold increase relative to UCCB01-125. The increased $T_{max}$ is explained by a prolonged absorption from the injection site. Overall, these properties enable administration by s.c.depot injections and thereby slow and consistent release of compound into the blood, whereby fewer administrations are needed to maintain pharmaceutical relevant blood concentrations.

Table 4: Pharmacokinetic parameters of FA-linked dimeric ligands after s.c. injection in rats

| Compound | Linker | FA | Peptide sequence | Dose (mg/kg) | $T_{½}$ (h)[a] | $T_{max}$ (h)[a] |
|---|---|---|---|---|---|---|
| **UCCB01-125** | - | - | IETAV | 3 | 0.561 ± 0.101 | 0.5 ± 0 |
| | | | | 30 | 0.450 ± 0.068 | 0.5 ± 0 |
| **1** | GABA | C12 | IETAV | 15 | 0.768 ± 0.045 | 0.833 ± 0.167 |
| **4** | GABA | C17:0-COOH | IETAV | 15 | 8.13 ± 0.50 | 4.67 ± 0.66 |
| **7** | γGlu | C18:0 | IETAV | 10 | 10.7 ± 0.58 | 6.00 ± 1.15 |
| **13** | GABA | C18:0 | IETDV | 10 | 16.3 ± 2.81 | 8.00 ± 0 |

[a]Data given as mean ± SEM, n=3.

**Example 9: Sequences**

**SEQ ID NO: 1**

$X_4X_3X_2X_1$

wherein

$X_4$ is an amino acid residue selected from E, Q, A, N and S,
$X_3$ is an amino acid residue selected from S and T,
$X_2$ is an amino acid residue selected from A, D, E, Q, N, S, V, *N*-Me-A, *N*-Me-D, *N*-Me-E, *N*-Me-Q, *N*-Me-N,

*N*-Me-S and *N*-Me-V
$X_1$ is an amino acid residue selected from I, L and V

**SEQ ID NO: 2**

$Z_4Z_3Z_2Z_1$

wherein

$Z_4$ is an amino acid residue selected from E, Q, A, N and S,
$Z_3$ is an amino acid residue selected from S and T,
$Z_2$ is an amino acid residue selected from A, D, E, Q, N, S, V, *N*-Me-A, *N*-Me-D, *N*-Me-E, *N*-Me-Q, *N*-Me-N, *N*-Me-S and *N*-Me-V
$Z_1$ is an amino acid residue selected from I, L and V

**SEQ ID NO: 3**
IETAV

**SEQ ID NO: 4**
IETDV

**SEQ ID NO: 5**

$X_5X_4X_3X_2X_1$

wherein

$X_5$ is any amino acid residue,
$X_4$ is an amino acid residue selected from E, Q, A, N and S,
$X_3$ is an amino acid residue selected from S and T,
$X_2$ is an amino acid residue selected from A, D, E, Q, N, S, V, *N*-Me-A, *N*-Me-D, *N*-Me-E, *N*-Me-Q, *N*-Me-N, *N*-Me-S and *N*-Me-V
$X_1$ is an amino acid residue selected from I, L and V

**SEQ ID NO: 6**

$Z_5Z_4Z_3Z_2Z_1$

wherein

$Z_5$ is any amino acid residue,
$Z_4$ is an amino acid residue selected from E, Q, A, N and S,
$Z_3$ is an amino acid residue selected from S and T,
$Z_2$ is an amino acid residue selected from A, D, E, Q, N, S, V, *N*-Me-A, *N*-Me-D, *N*-Me-E, *N*-Me-Q, *N*-Me-N, *N*-Me-S and *N*-Me-V
$Z_1$ is an amino acid residue selected from I, L and V

## Claims

1. A dimeric ligand of PSD-95 comprising a first peptide ($P_1$) and a second peptide ($P_2$), wherein

   $P_1$ and $P_2$ individually comprise at least two proteinogenic or non-proteinogenic amino acid residues,
   both $P_1$ and $P_2$ are conjugated to a first linker $L_1$ via their N-termini,
   $L_1$ comprises polyethylene glycol (PEG) wherein at least one oxygen atom of said PEG is substituted with a nitrogen atom to give *N*PEG,

   an albumin binding moiety is linked to the nitrogen atom of the *N*PEG by an amide bond, or via an optional linker L2, wherein $L_2$ comprises a nitrogen atom,
   the albumin binding moiety is a fatty acid (FA),
   and wherein said dimeric ligand has the generic structure of formula (II), wherein $L_2$ is optional:

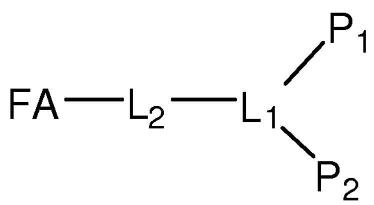

Formula (II)

   or a pharmaceutically acceptable salt or prodrug thereof.

2. The dimeric ligand according to any one of the preceding claims, wherein the second linker $L_2$ comprises one or more moieties selected from the group consisting of γ-Glu, γ-butyric acid (GABA), 5-amino valeric acid (5-Ava), proteinogenic amino acids, non-proteinogenic amino acids, and any dimeric ligand having the general formula $H_2N$-[Q]-COOH, wherein Q is any suitable atom or atoms.

3. The dimeric ligand according to any one of the preceding claims, wherein said dimeric ligand has the generic structure of formula (III) or (IV):

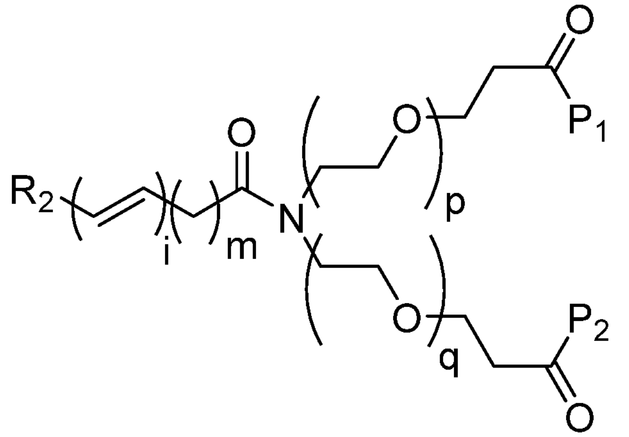

Formula (III)

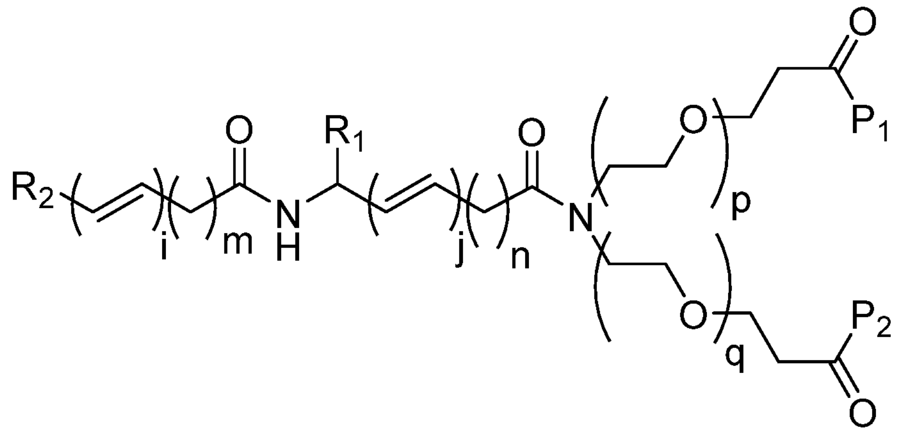

Formula (IV)

wherein

$R_1$ and $R_2$ individually are selected from the group consisting of H and COOH,
n is an integer 0 to 48,
m is an integer 1 to 48,
p is an integer 0 to 28,
q is an integer 0 to 28,
i is an integer 0 to 12,
j is an integer 0 to 12
$P_1$ and $P_2$ are individually selected from peptides comprising at least two proteinogenic or non-proteinogenic amino acid residues.

**4.** The dimeric ligand according to any one of the preceding claims, wherein the fatty acid is a $C_4$-$C_{22}$ fatty acid.

**5.** The dimeric ligand according to any one of the preceding claims, wherein the fatty acid is selected from the group consisting of caprylic acid, capric acid, lauric acid, myristic acid, palmitic acid, stearic acid, arachidic acid, behenic acid, lignoceric acid,cerotic acid, myristoleic acid, palmitoleic acid, sapienic acid, oleic acid, elaidic acid, vaccenic acid, linoleic acid, linoelaidic acid, $\alpha$-linolenic acid, arachidonic acid, eicosapentaenoic acid, erucic acid and docosahexaenoic acid.

**6.** The dimeric ligand according to any one of the preceding claims, wherein

$P_1$ comprises the amino acid sequence $X_4X_3X_2X_1$ (SEQ ID NO: 1), and
$P_2$ comprises the amino acid sequence $Z_4Z_3Z_2Z_1$ (SEQ ID NO: 2),

wherein

$X_1$ and/or $Z_1$ is an amino acid residue selected from I, L and V,

$X_2$ and/or $Z_2$ is an amino acid residue selected from A, D, E, Q, N, S, V, *N*-Me-A, N-Me-D, *N*-Me-E, *N*-Me-Q, *N*-Me-N, *N*-Me-S and *N*-Me-V,
$X_3$ and/or $Z_3$ is an amino acid residue selected from S and T,
$X_4$ and/or $Z_4$ is an amino acid residue selected from E, Q, A, N and S,

wherein $X_1$ and $Z_1$ both individually represent the ultimate C-terminal amino acid residue comprising a free carboxylic acid.

**7.** The dimeric ligand according to any one of the preceding claims, wherein said dimeric ligand has the generic structure of formula (V) or (VI):

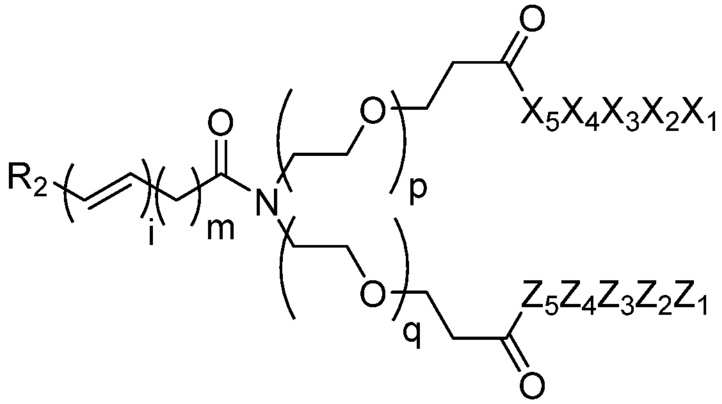

Formula (V)

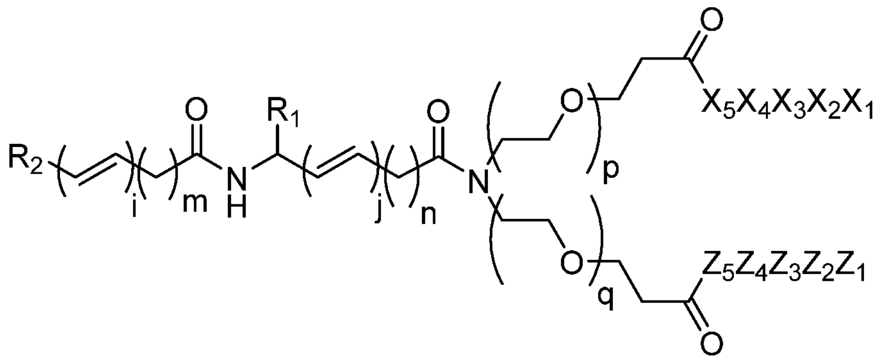

Formula (VI)

wherein

$R_1$ and $R_2$ individually are selected from the group consisting of H and COOH,
n is an integer 0 to 48,
m is an integer 1 to 48,
p is an integer 0 to 28,
q is an integer 0 to 28,
i is an integer 0 to 12,
j is an integer 0 to 12
$X_5$ and/or $Z_5$ are/is an optional proteinogenic or a non-proteinogenic amino acid residue, a peptide or a polypeptide,
$X_4$ and/or $Z_4$ is an amino acid residue selected from E, Q, A, N and S,
$X_3$ and/or $Z_3$ is an amino acid residue selected from S and T,
$X_2$ and/or $Z_2$ is an amino acid residue selected from A, D, E, Q, N, S, V, *N*-Me-A, *N*-Me-D, *N*-Me-E, *N*-Me-Q, *N*-Me-N, *N*-Me-S and *N*-Me-V
$X_1$ and/or $Z_1$ is an amino acid residue selected from I, L and V.

**8.** The dimeric ligand according to any one of the preceding claims, wherein $X_5$ is an amino acid residue selected from the group consisting of I, A, L and V.

**9.** The dimeric ligand according to any one of the preceding claims, wherein the dimeric ligand is selected from the group consisting of:

a) KBN41 **(5)** derivative

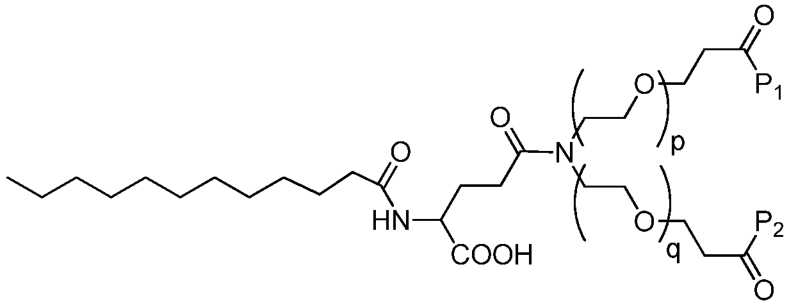

b) KBN42 (7) Derivative

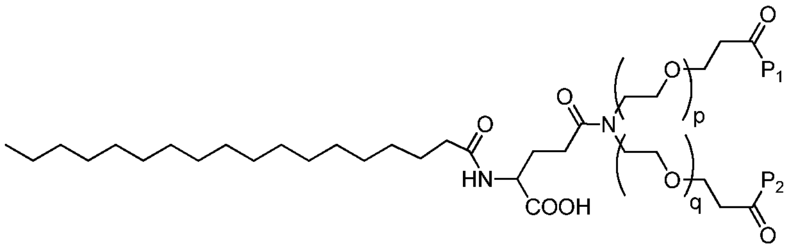

c) KBN43 **(6)** Derivative

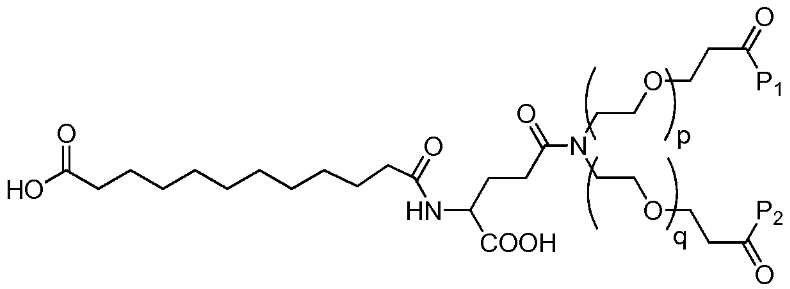

d) KBN44 **(8)** derivative

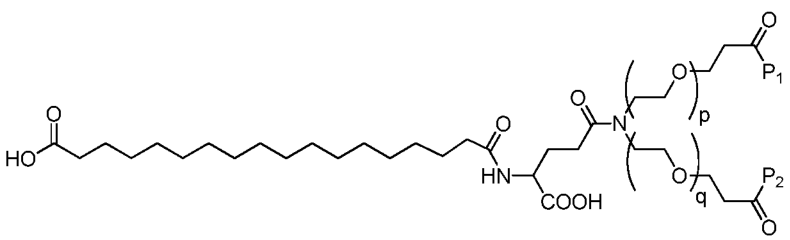

e) KBN45 **(9)** derivative

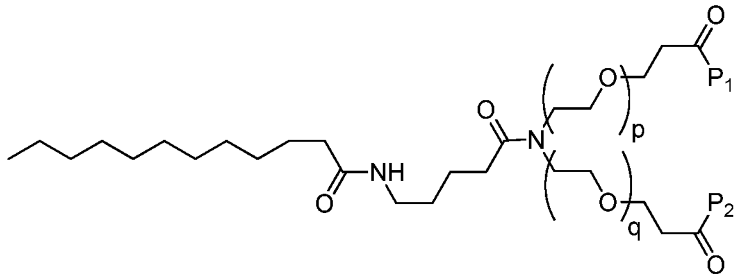

f) KBN46 **(11)** derivative

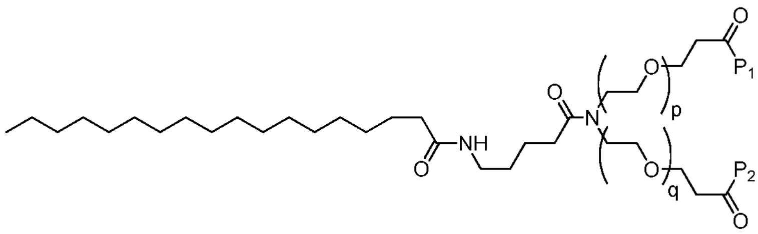

g) KBN47 **(10)** derivative

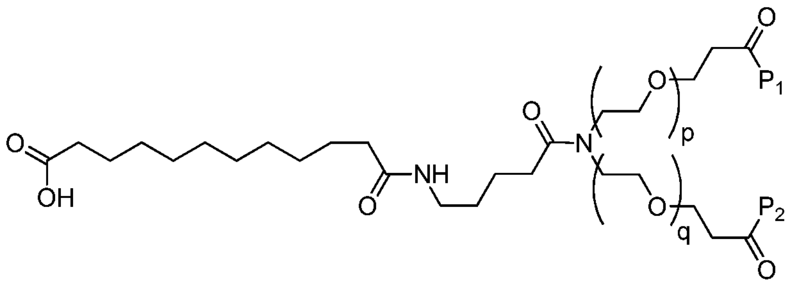

h) KBN48 **(12)** derivative

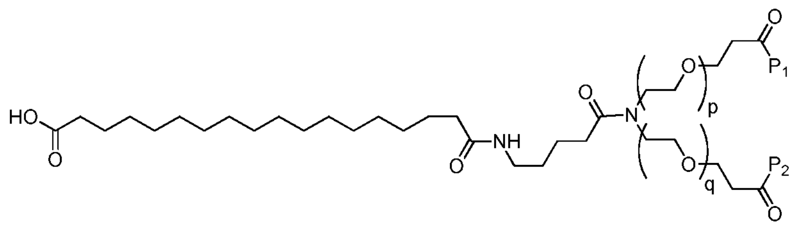

i) KBN52 **(1)** Derivative

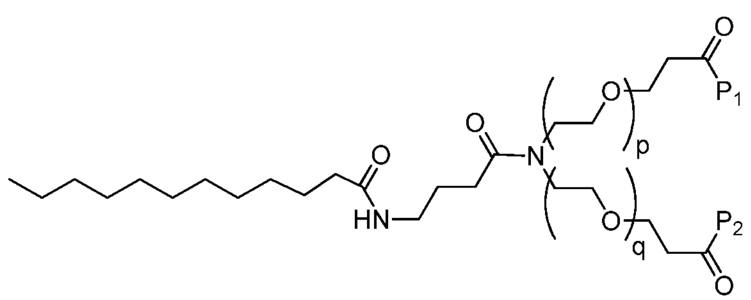

j) KBN53 **(3)** derivative

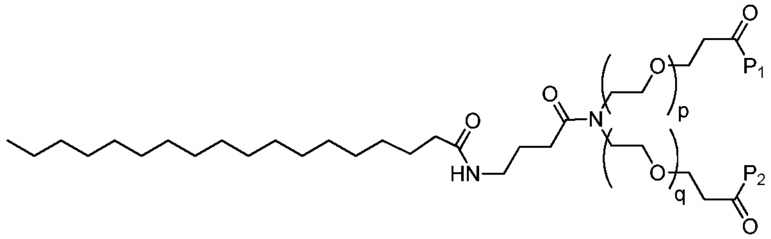

k) KBN54 **(2)** derivative

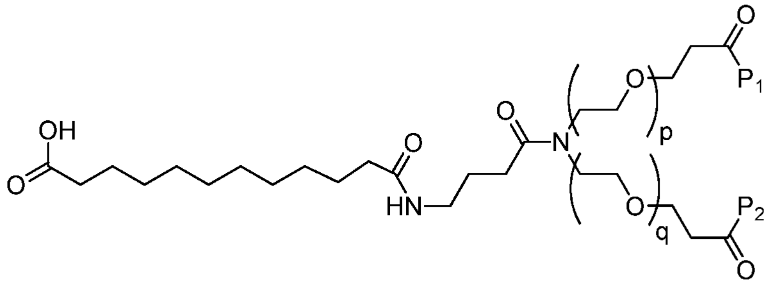

l) KBN55 **(4)** derivative

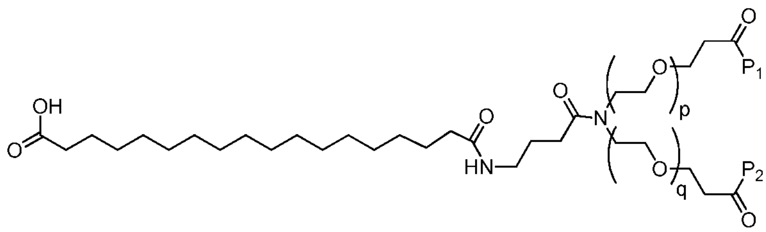

**10.** The dimeric ligand according to any one of the preceding claims, wherein the dimeric ligand is selected from the group consisting of:

a) KBN41 (**5**) derivative

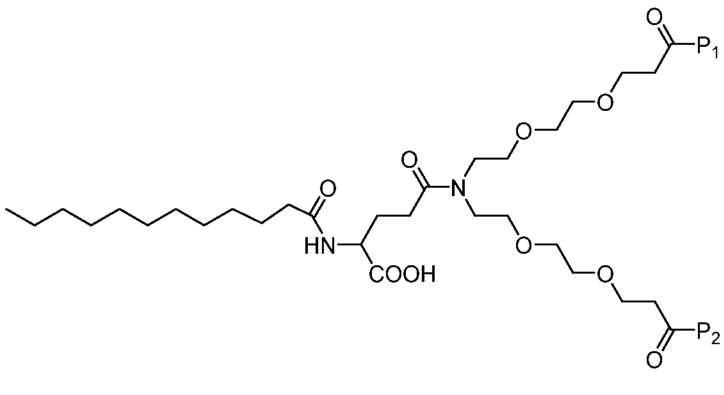

b) KBN42 (**7**) Derivative

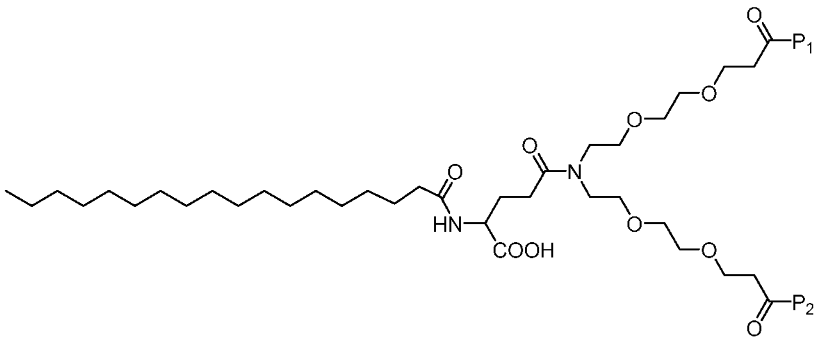

c) KBN43 (**6**) derivative

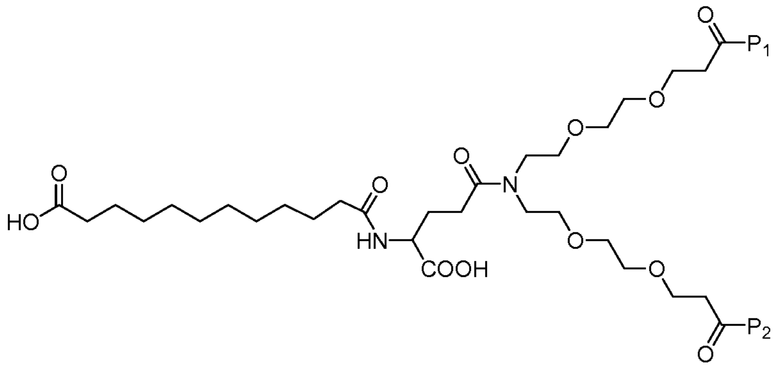

d) KBN44 (**8**) derivative

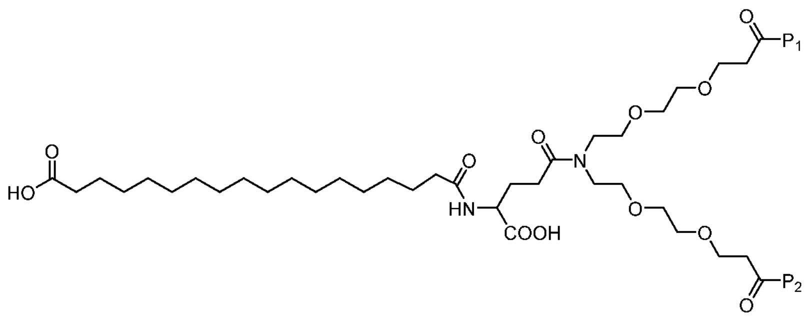

e) KBN45 (**9**) derivative

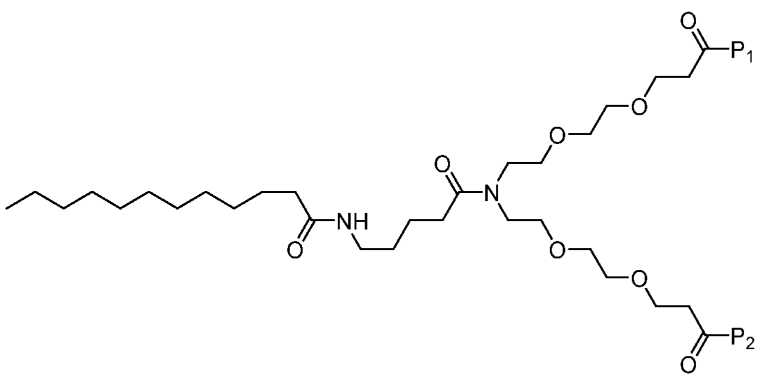

f) KBN46 (**11**) derivative

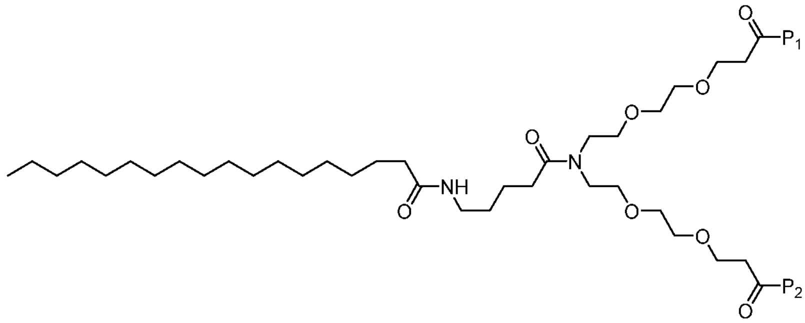

g) KBN47 (**10**) derivative

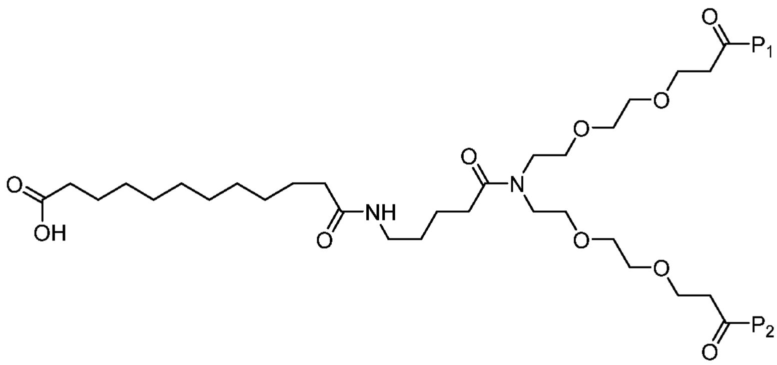

h) KBN48 (**12**) derivative

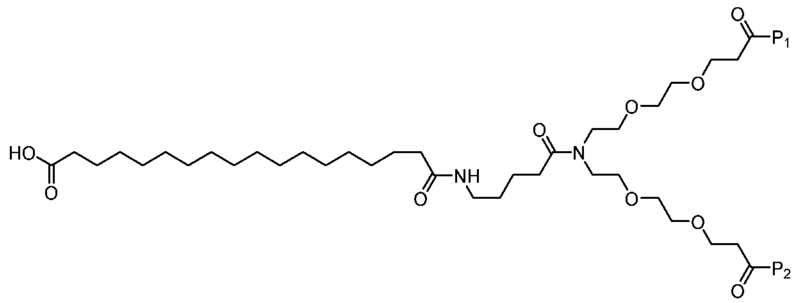

i) KBN52 (**1**) derivative

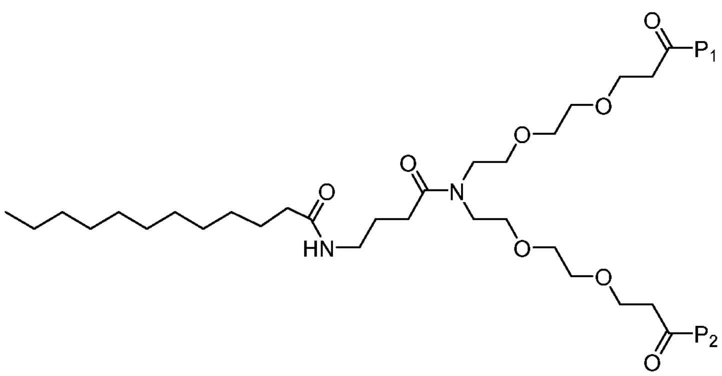

j) KBN53 (3) derivative

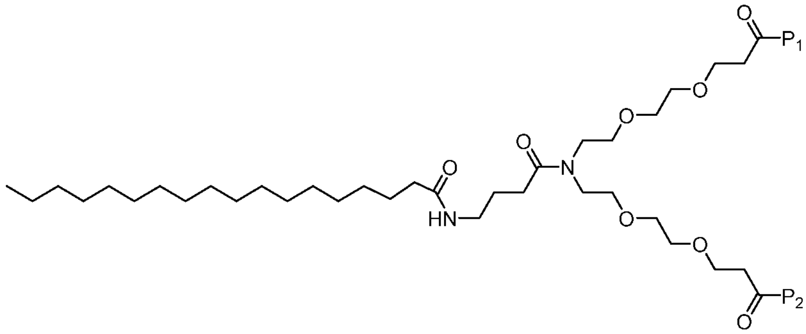

k) KBN54 (**2**) derivative

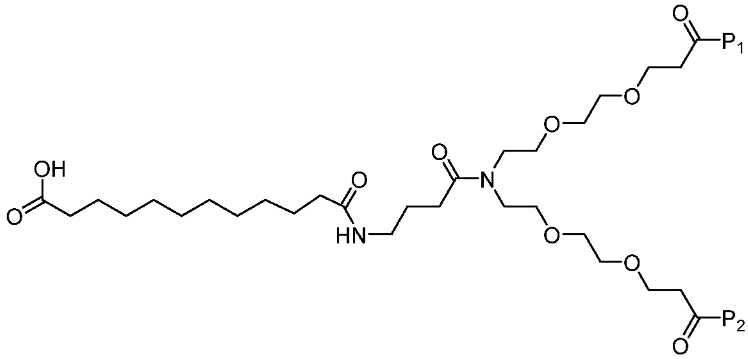

l) KBN55 (**4**) derivative

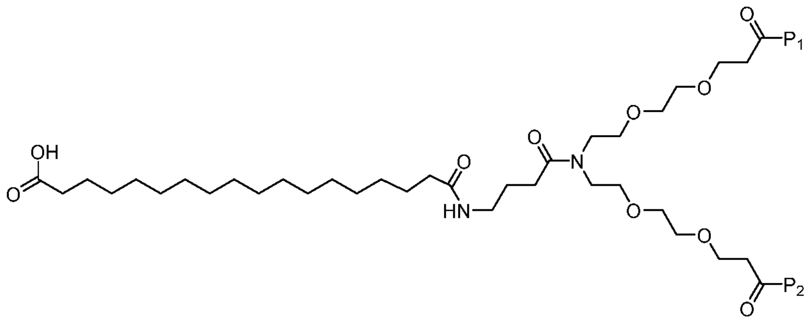

**11.** The dimeric ligand according to any one of the preceding claims, wherein the dimeric ligand is selected from the group consisting of:

a) KBN41 (**5**)

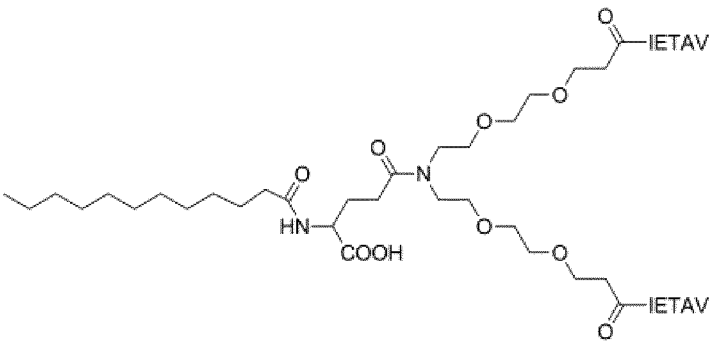

b) KBN42 (**7**)

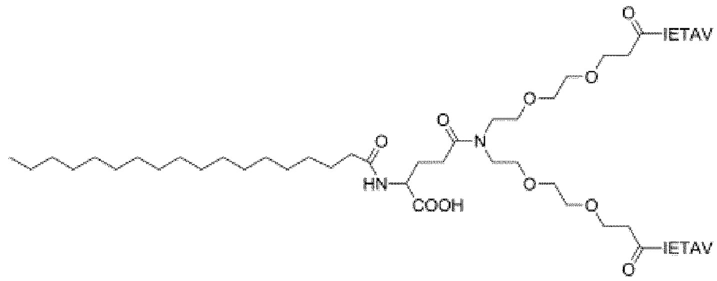

c) KBN43 (**6**)

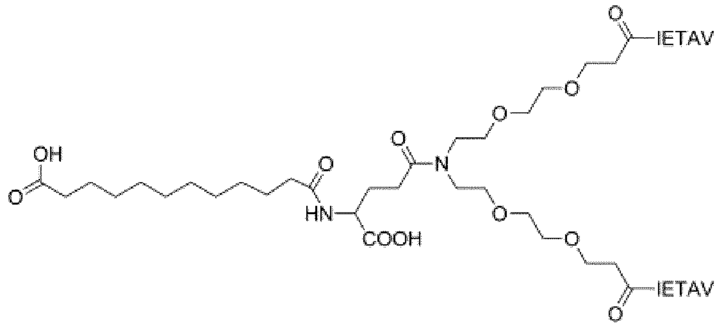

d) KBN44 (**8**)

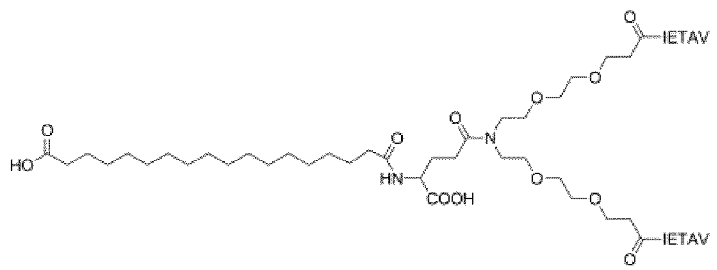

e) KBN45 (**9**)

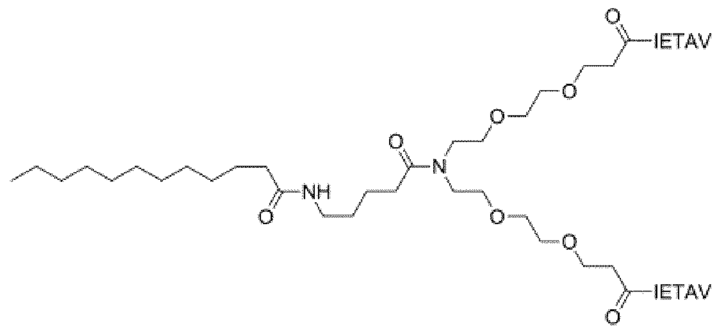

f) KBN46 (**11**)

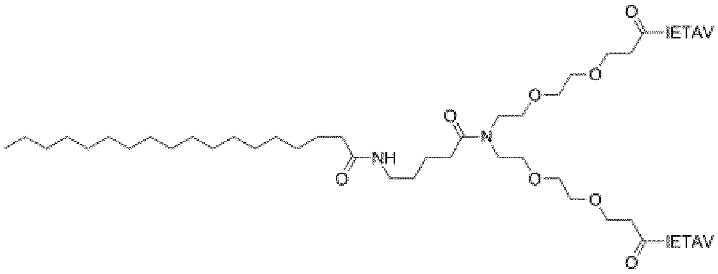

g) KBN47 (**10**)

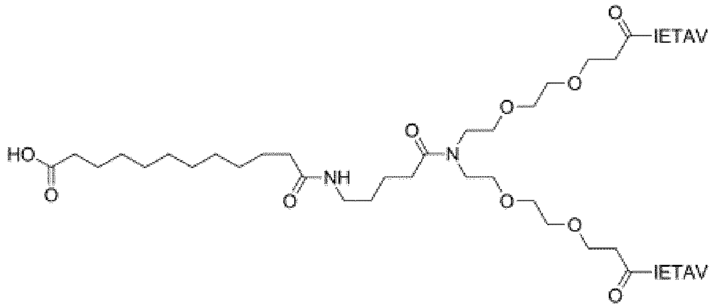

h) KBN48 (**12**)

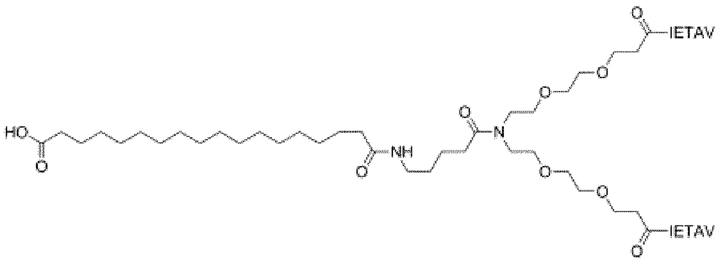

j) KBN52 (**1**)

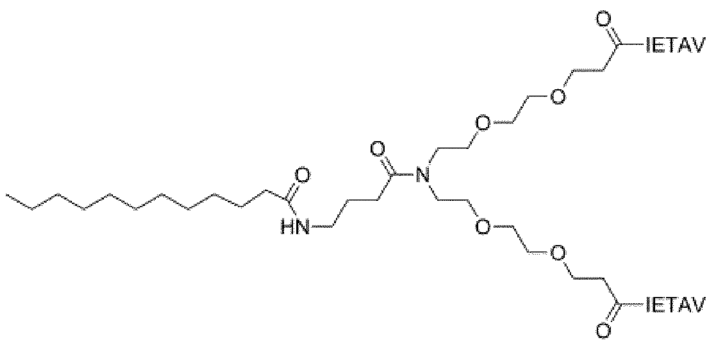

k) KBN53 (**3**)

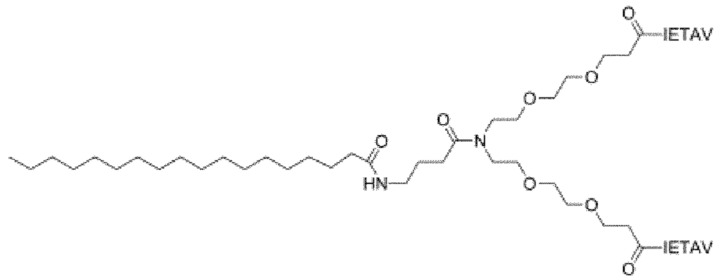

l) KBN54 (**2**)

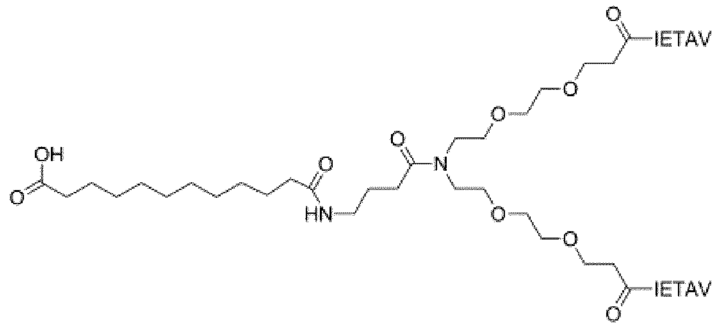

m) KBN55 (**4**)

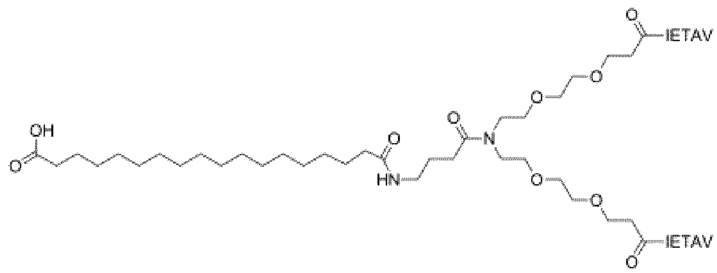

n) KBN63 (**15**)

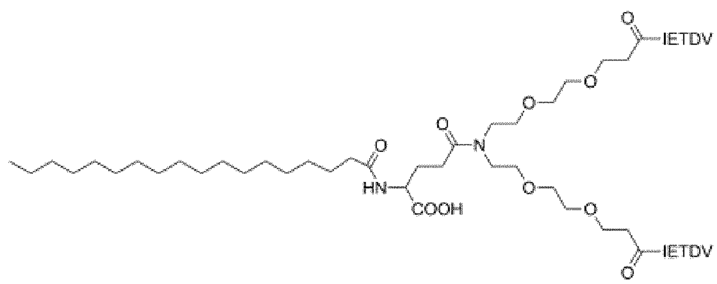

o) KBN64 (**13**)

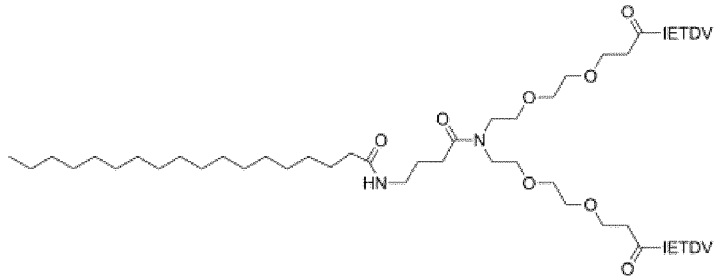

p) KBN65 (**14**)

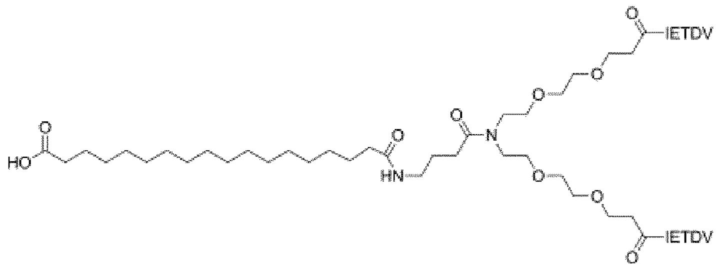

**12.** A dimeric ligand according to any one of the preceding claims for use as a medicament.

**13.** A dimeric ligand according to any one of claims 1 to 13 for use in the treatment or prophylaxis of pain and/or an excitotoxic-related disease.

**14.** The dimeric ligand according to claim 13, wherein the disease is ischemic or traumatic injury to/in/of the CNS.

**15.** A method of manufacturing the dimeric ligand according to any one of claims 1 to 14, said method comprising the steps of:

a) preparing a Ns-*N*PEG diacid linker,
b) preparing a peptide using Fmoc-based solid-phase peptide synthesis,
c) dimerizing said peptide with said Ns-*N*PEG diacid linker, and
d) coupling a fatty acid to the linker

**Patentansprüche**

**1.** Dimerer Ligand von PSD-95, der ein erstes Peptid ($P_1$) und ein zweites Peptid ($P_2$) aufweist, wobei

$P_1$ und $P_2$ individuell mindestens zwei proteinogene oder nicht proteinogene Aminosäurerückstände umfassen, sowohl $P_1$ als auch $P_2$ an einem ersten Linker $L_1$ über ihre N-Termini konjugiert sind,
$L_1$ Polyethylenglycol (PEG) umfasst, wobei mindestens ein Sauerstoffatom des PEG mit einem Stickstoffatom substituiert ist, um *N*PEG zu ergeben,

eine Albumin-Bindungseinheit über eine Amidbindung oder über einen optionalen Linker $L_2$ mit dem Stickstoffatom des *N*PEG verbunden ist, wobei $L_2$ ein Stickstoffatom umfasst,
die Albumin-Bindegruppe eine Fettsäure (FA) ist,
und wobei der dimere Ligand die generische Struktur der Formel (II) aufweist, wobei $L_2$ wahlweise:

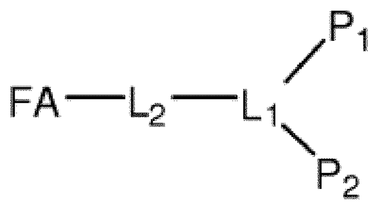

Formel (II)

ist oder ein pharmazeutisch annehmbares Salz oder Pro-Pharmakon davon ist.

**2.** Dimerer Ligand nach einem der vorhergehenden Ansprüche, wobei der zweite Linker $L_2$ eine oder mehrere Einheiten umfasst, die ausgewählt sind aus der Gruppe bestehend aus γ-Glu, γ-Buttersäure (GABA), 5-Aminovaleriansäure (5-Ava), proteinogenen Aminosäuren, nicht-proteinogenen Aminosäuren und einem beliebigen dimeren Liganden, der die allgemeine Formel $H_2N$-[Q]-COOH aufweist, wobei Q ein beliebiges geeignetes Atom oder Atome ist/sind.

**3.** Dimerer Ligand nach einem der vorhergehenden Ansprüche, wobei der dimere Ligand die generische Struktur der Formel (III) oder (IV) aufweist:

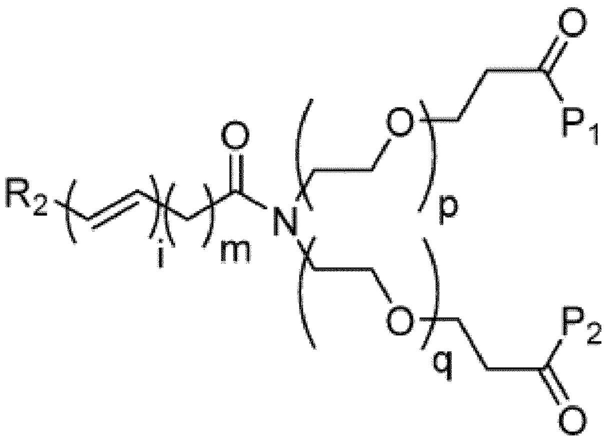

Formel (III)

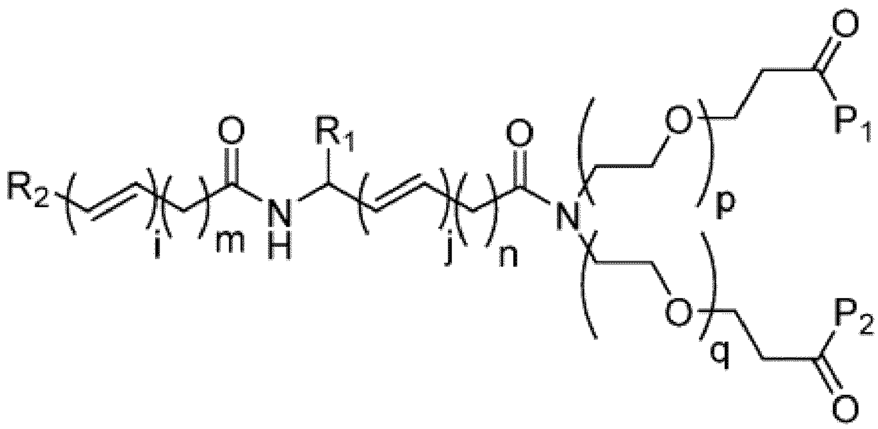

Formel (IV)

wobei $R_1$ und $R_2$ individuell ausgewählt sind aus der Gruppe bestehend aus H und COOH,

n eine Ganzzahl von 0 bis 48 ist,
m eine Ganzzahl von 1 bis 48 ist,
p eine Ganzzahl von 0 bis 28 ist,
q eine Ganzzahl von 0 bis 28 ist,

i eine Ganzzahl von 0 bis 12 ist,
j eine Ganzzahl von 0 bis 12 ist
$P_1$ und $P_2$ individuell ausgewählt sind aus Peptiden, die mindestens zwei proteinogene oder nicht-proteinogene Aminosäurerückstände umfassen.

**4.** Dimerer Ligand nach einem der vorhergehenden Ansprüche, wobei die Fettsäure eine $C_4$-$C_{22}$-Fettsäure ist.

**5.** Dimerer Ligand nach einem beliebigen der vorhergehenden Ansprüche, wobei die Fettsäure ausgewählt ist aus der Gruppe bestehend aus Caprylsäure, Caprinsäure, Laurinsäure, Myristinsäure, Palmitinsäure, Stearinsäure, Arachinsäure, Behensäure, Lignocerinsäure, Cerotinsäure, Myristoleinsäure, Palmitoleinsäure, Sapiensäure, Ölsäure, Elaidinsäure, Vaccensäure, Linolsäure, Linoelaidinsäure, α-Linolensäure, Arachidonsäure, Eicosapentaensäure, Erucasäure und Docosahexaensäure.

**6.** Dimerer Ligand nach einem der vorhergehenden Ansprüche, wobei

$P_1$ die Aminosäuresequenz $X_4X_3X_2X_1$ (SEQ ID NO: 1) umfasst, und
$P_2$ die Aminosäuresequenz $Z_4Z_3Z_2Z_1$ (SEQ ID NO: 2) umfasst,

wobei

$X_1$ und/oder $Z_1$ ein Aminosäurerückstand ist, der ausgewählt ist aus I, L und V,
$X_2$ und/oder $Z_2$ Aminosäurerückstand ist, der ausgewählt ist aus A, D, E, Q, N, S, V, *N*-Me-A, *N*-Me-D, *N*-Me-E, *N*-Me-Q, *N*-Me-N, *N*-Me-S und *N*-Me-V,
$X_3$ und/oder $Z_3$ ein Aminosäurerückstand ist, der ausgewählt ist aus S und T,
$X_4$ und/oder $Z_4$ ein Aminosäurerückstand ist, der ausgewählt ist aus E, Q, A, N und S,

wobei $X_1$ und $Z_1$ beide individuell den letzten C-Terminal-Aminosäurerückstand darstellen, der eine freie Carbonsäure umfasst.

**7.** Dimerer Ligand nach einem der vorhergehenden Ansprüche, wobei der dimere Ligand die generische Struktur der Formel (V) oder (VI) aufweist:

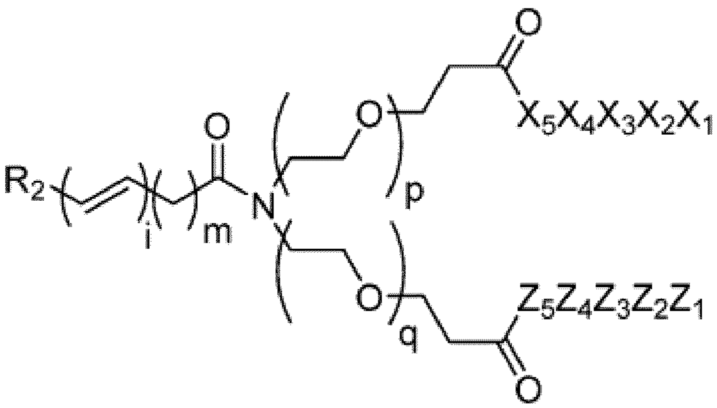

Formel (V)

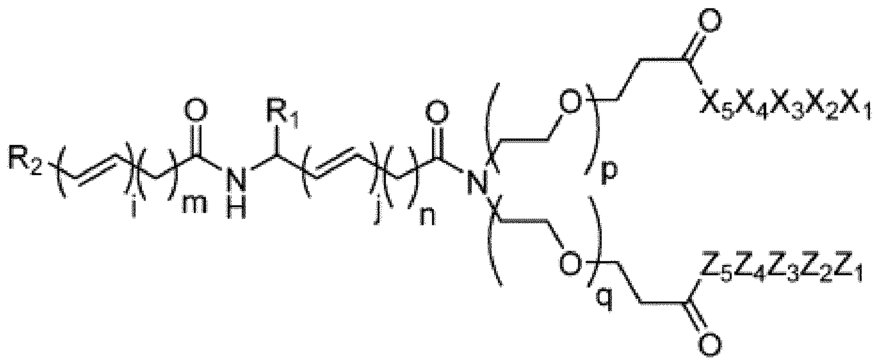

Formel

(VI)

wobei

$R_1$ und $R_2$ individuell ausgewählt sind aus der Gruppe bestehend aus H und COOH,
n eine Ganzzahl von 0 bis 48 ist,
m eine Ganzzahl von 1 bis 48 ist,
p eine Ganzzahl von 0 bis 28 ist,
q eine Ganzzahl von 0 bis 28 ist,
i eine Ganzzahl von 0 bis 12 ist,
j eine Ganzzahl von 0 bis 12 ist
$X_5$ und/oder $Z_5$ wahlweise ein proteinogener oder nicht proteinogener Rückstand, ein Peptid oder ein Polypeptid ist,
$X_4$ und/oder $Z_4$ ein Aminosäurerückstand ist, der ausgewählt ist aus E, Q, A, N und S,
$X_3$ und/oder $Z_3$ ein Aminosäurerückstand ist, der ausgewählt ist aus S und T,
$X_2$ und/oder $Z_2$ ein Aminosäurerückstand ist, der ausgewählt ist aus A, D, E, Q, N, S, V, *N*-Me-A, *N*-Me-D, *N*-Me-E, *N*-Me-Q, *N*-Me-N, *N*-Me-S und *N*-Me-V
$X_1$ und/oder $Z_1$ ein Aminosäurerückstand ist, der ausgewählt ist aus I, L und V.

**8.** Dimerer Ligand nach einem der vorhergehenden Ansprüche, wobei $X_5$ ein Aminosäurerückstand ist, der ausgewählt ist aus der Gruppe bestehend aus I, A, L und V.

**9.** Dimerer Ligand nach einem der vorhergehenden Ansprüche, wobei der dimere Ligand ausgewählt ist aus der Gruppe bestehend aus:

a) KBN41 **(5)** -Derivat

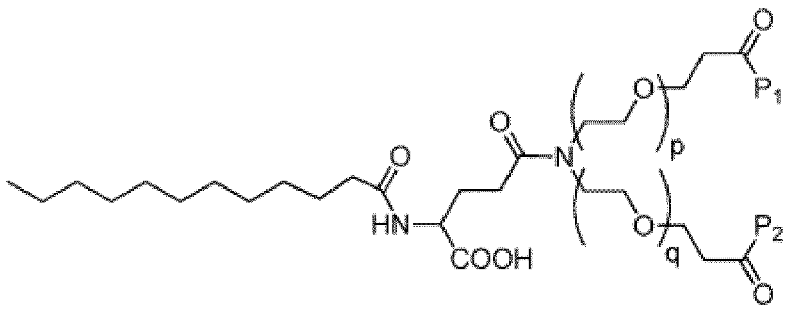

b) KBN42 **(7)** -Derivat

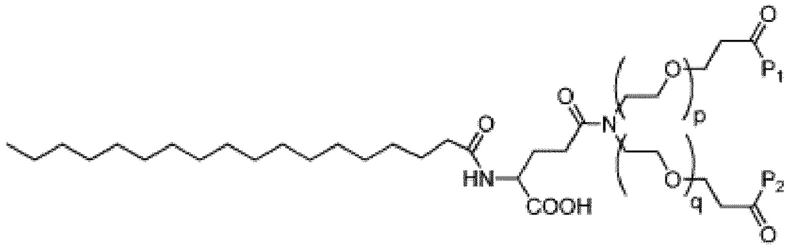

c) KBN43 **(6)** -Derivat

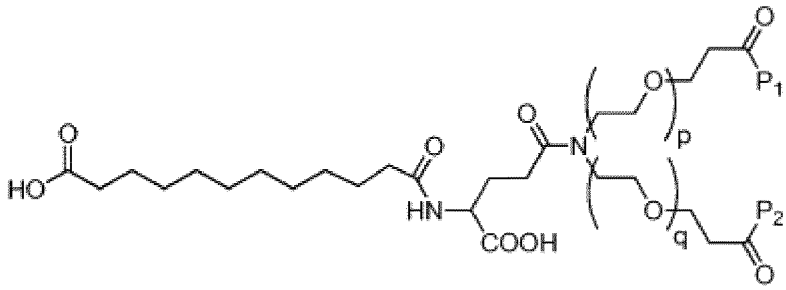

d) KBN44 **(8)** -Derivat

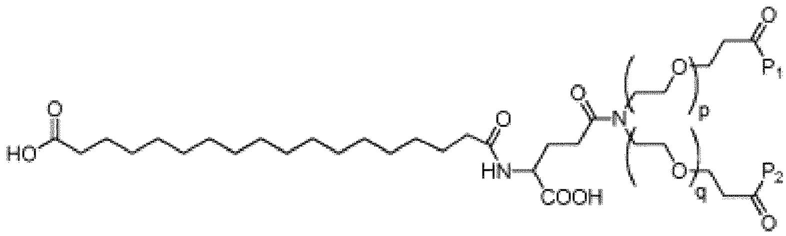

e) KBN45 **(9)** -Derivat

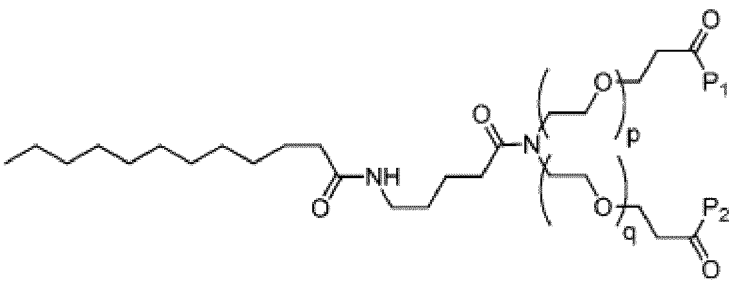

f) KBN46 **(11)** -Derivat

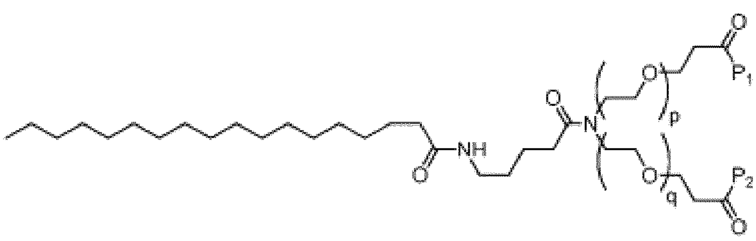

g) KBN47 **(10)** -Derivat

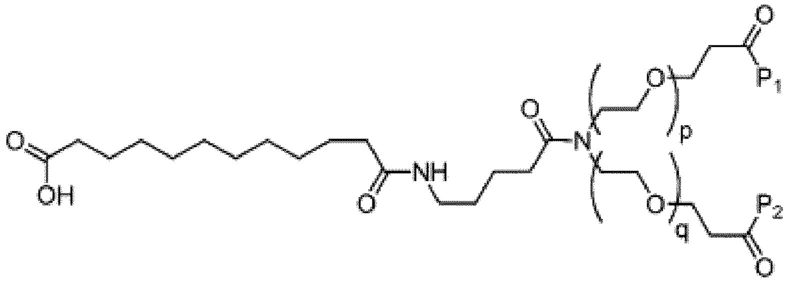

h) **KBN48 (12)** -Derivat

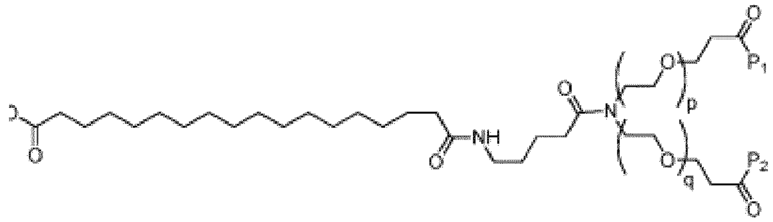

i) KBN52 **(1)** -Derivat

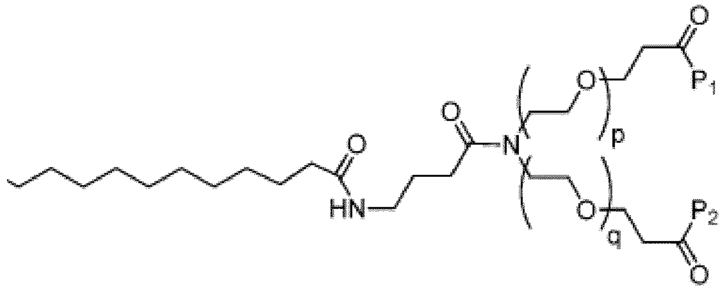

j) KBN53 **(3)** -Derivat

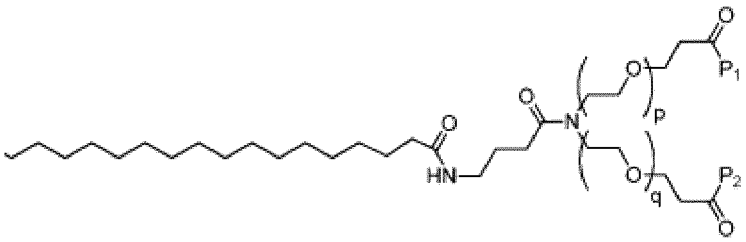

**k) KBN54 (2)** -Derivat

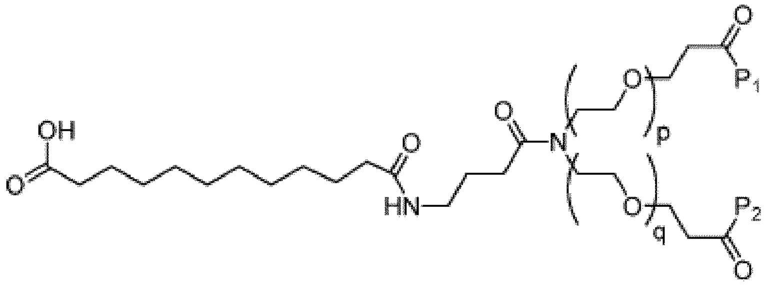

l) KBN55 **(4)** -Derivat

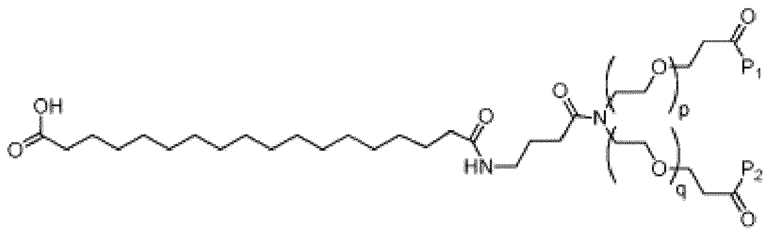

**10.** Dimerer Ligand nach einem der vorhergehenden Ansprüche, wobei der dimere Ligand ausgewählt ist aus der Gruppe bestehend aus:

a) KBN41 **(5)** -Derivat

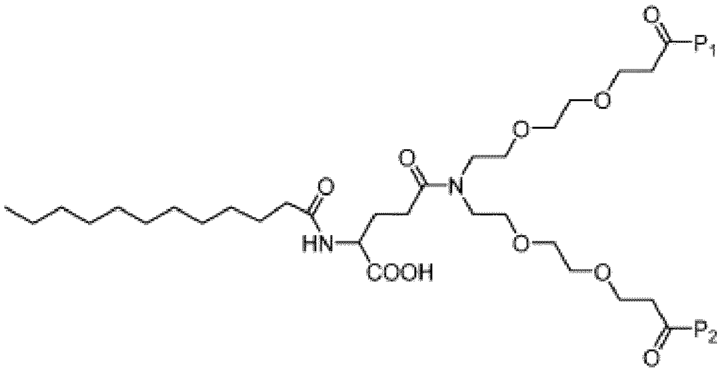

b) KBN42 **(7)** -Derivat

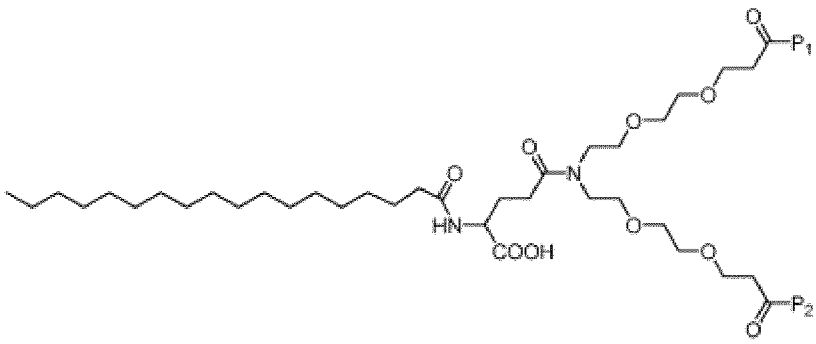

c) KBN43 **(6)** -Derivat

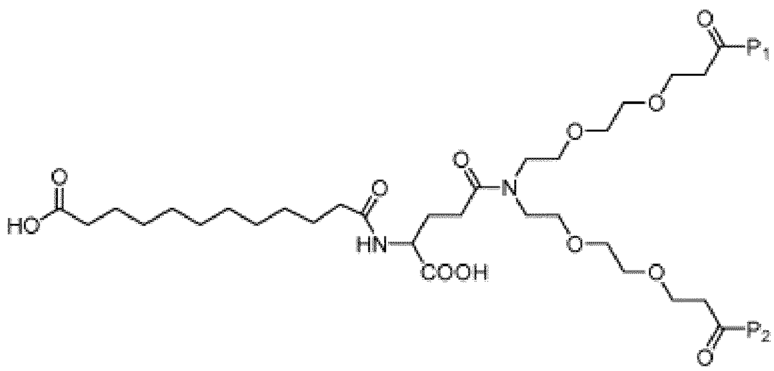

d) KBN44 **(8)** -Derivat

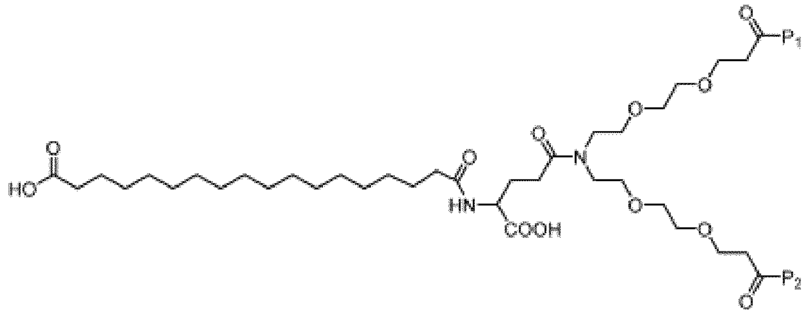

**e)** KBN45 **(9)** -Derivat

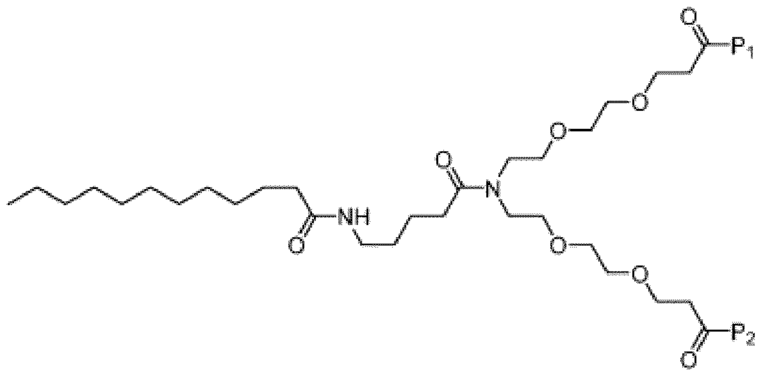

f) KBN46 **(11)** -Derivat

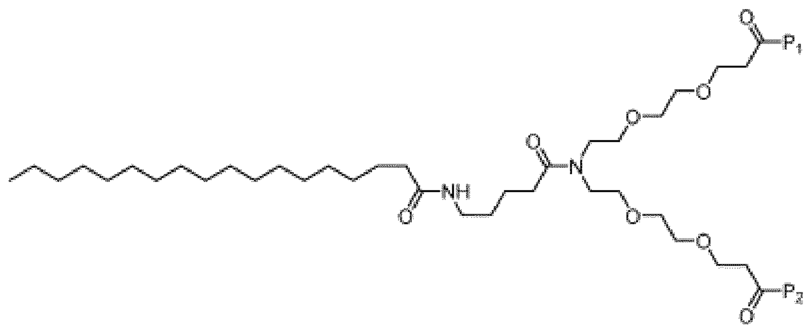

g) KBN47 **(10)** -Derivat

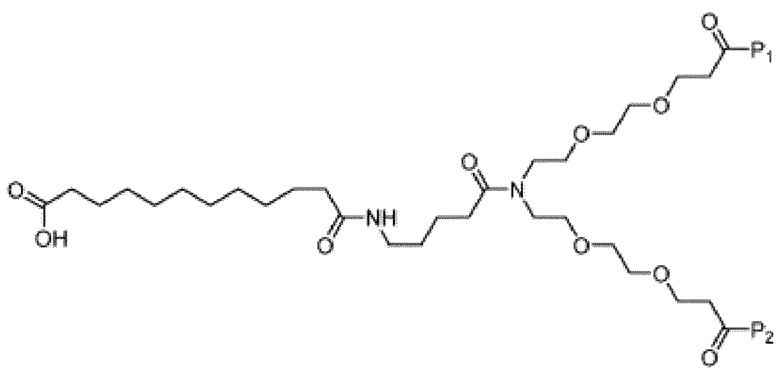

h) KBN48 **(12)** -Derivat

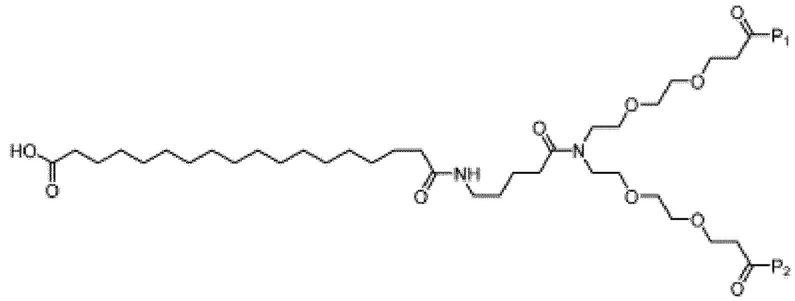

i) KBN52 **(1)** -Derivat

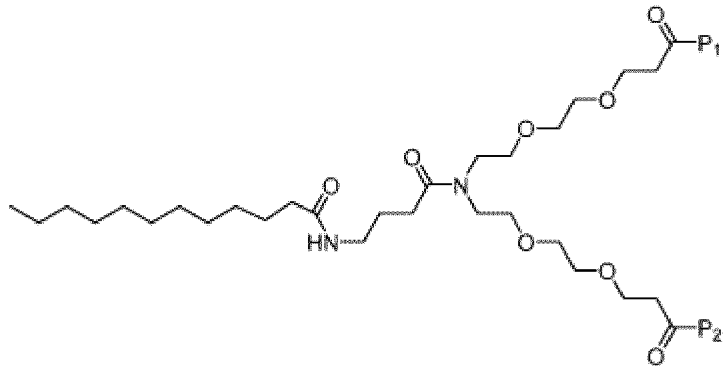

j) KBN53 **(3)** -Derivat

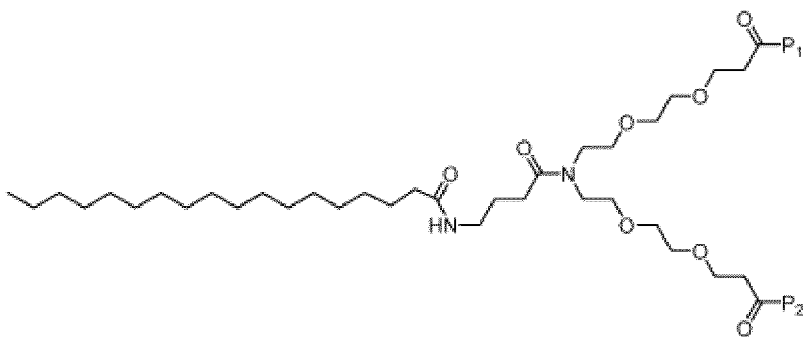

k) KBN54 **(2)** -Derivat

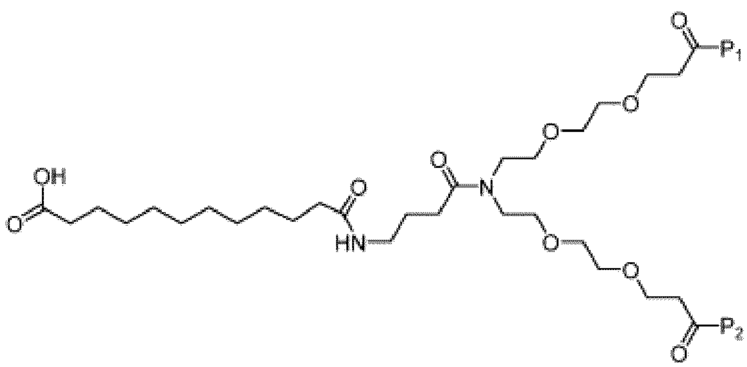

l) KBN55 **(4)** -Derivat

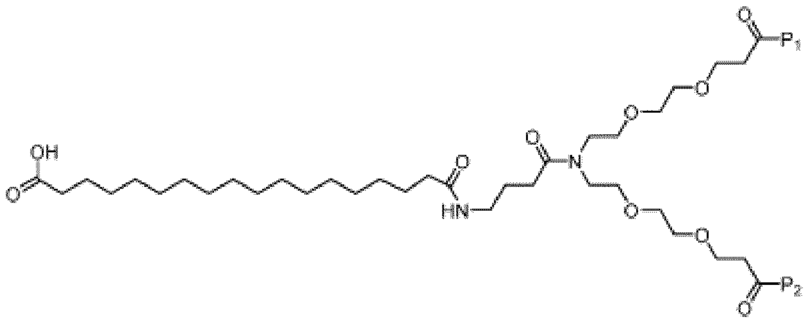

**11.** Dimerer Ligand nach einem der vorhergehenden Ansprüche, wobei der dimere Ligand ausgewählt ist aus der Gruppe bestehend aus:

a) KBN41 **(5)**

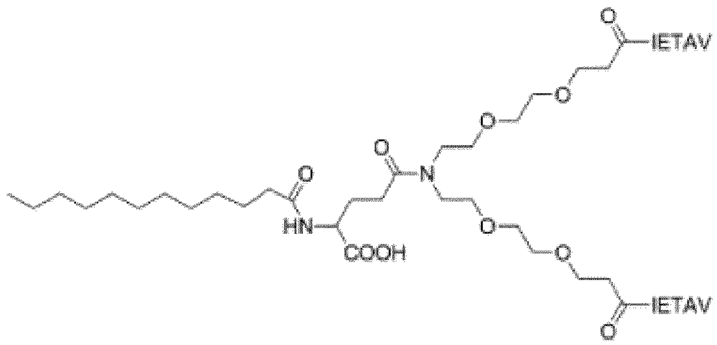

b) KBN42 **(7)**

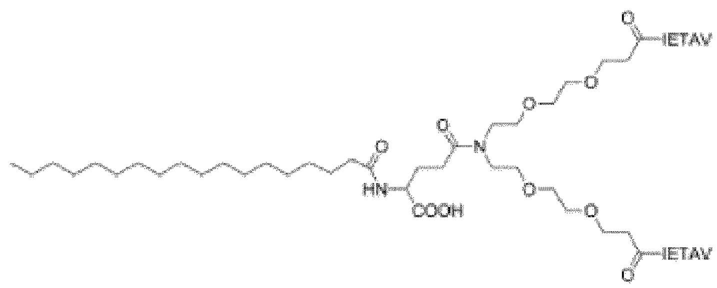

c) KBN43 **(6)**

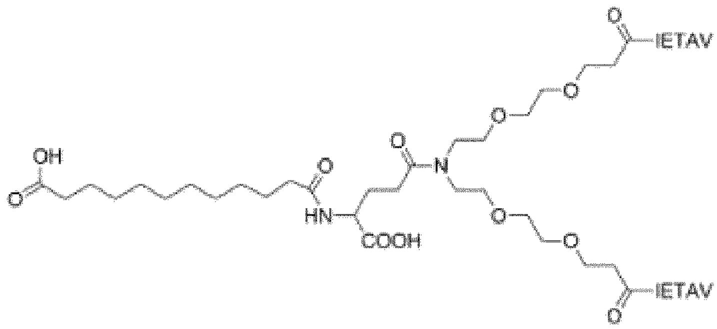

d) KBN44 **(8)**

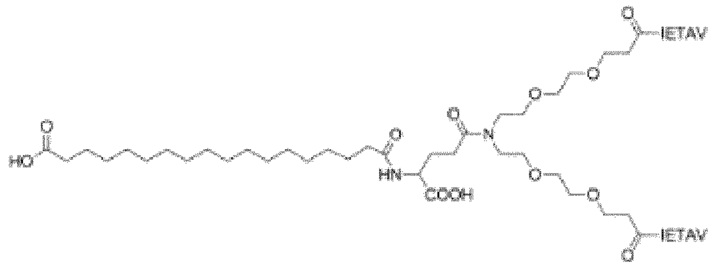

e) KBN45 **(9)**

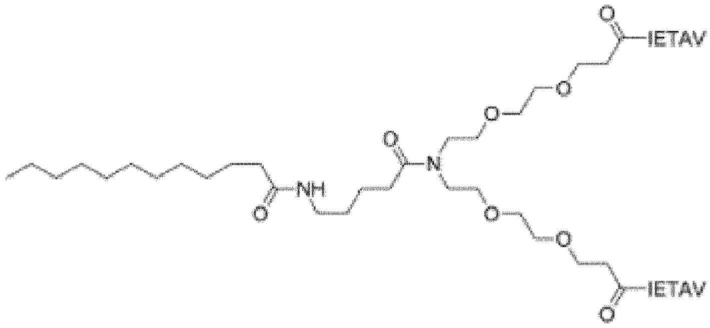

f) KBN46 **(11)**

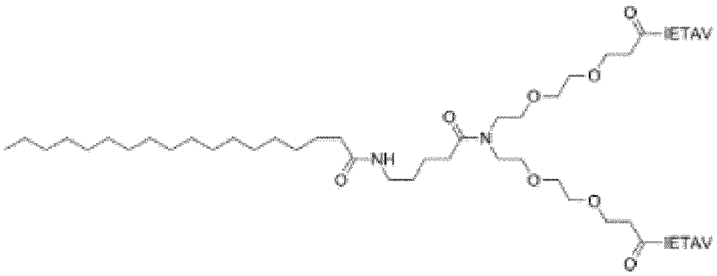

g) KBN47 **(10)**

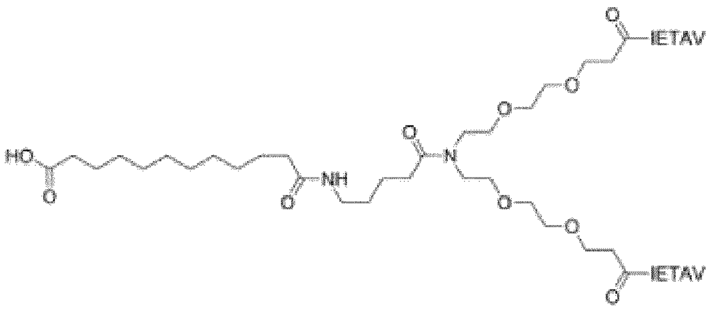

h) KBN48 **(12)**

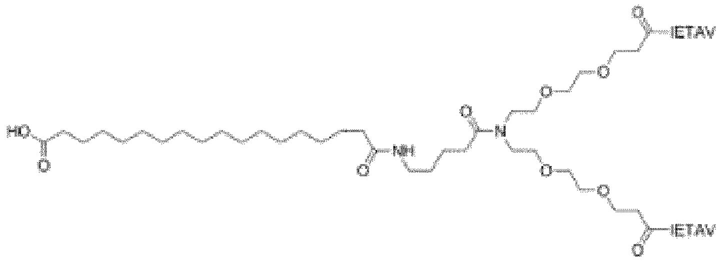

j) KBN52 **(1)**

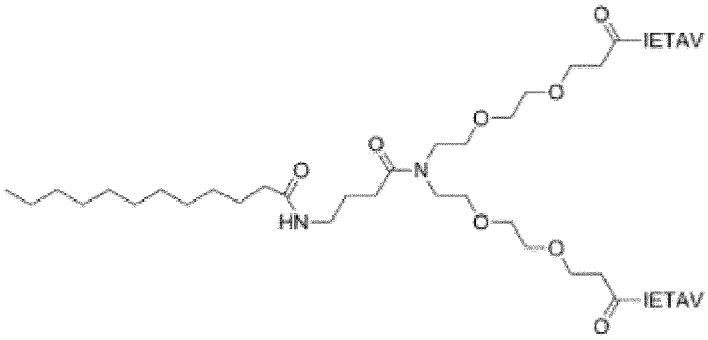

k) KBN53 **(3)**

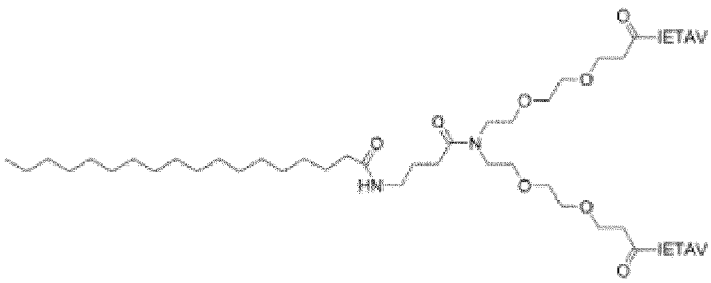

l) KBN54 **(2)**

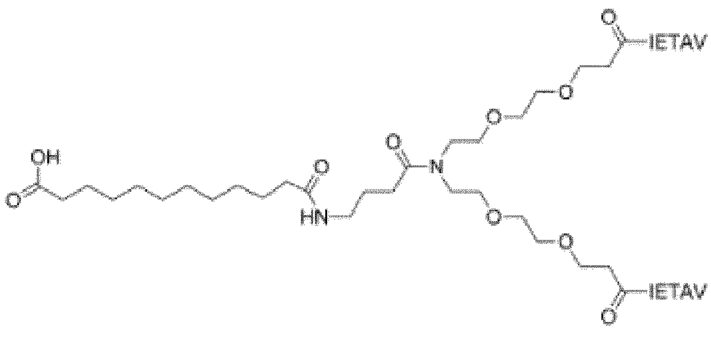

**m) KBN55 (4)**

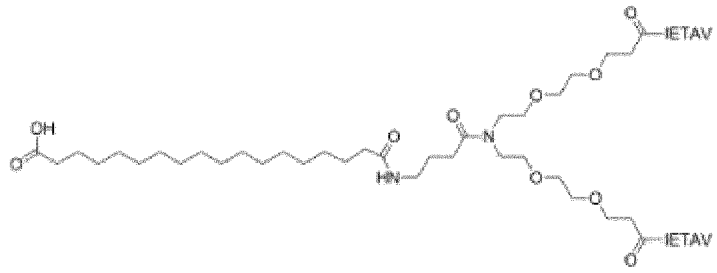

n) KBN63 **(15)**

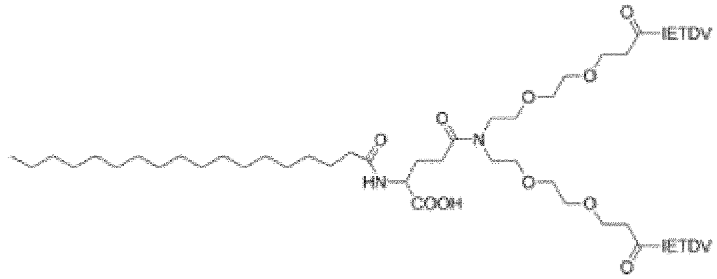

o) KBN64 **(13)**

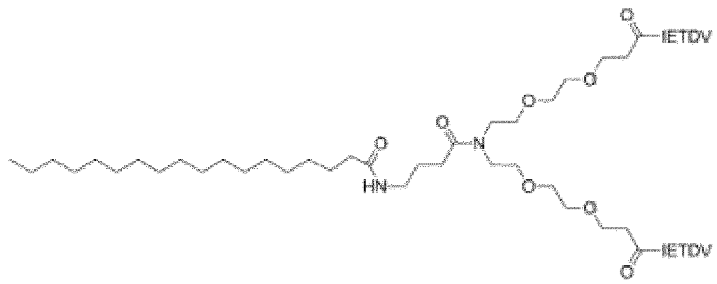

p) KBN65 **(14)**

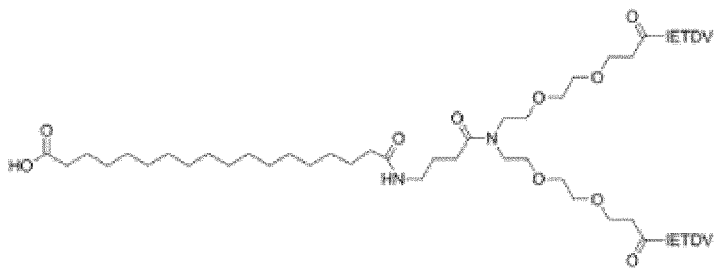

**12.** Dimerer Ligand nach einem der vorhergehenden Ansprüche zur Verwendung als Medikament.

**13.** Dimerer Ligand nach einem der vorhergehenden Ansprüche 1 bis 13 zur Verwendung bei der Behandlung oder Prophylaxe von Schmerzen und/oder einer Excitotoxizität zugehörigen Erkrankung.

**14.** Dimerer Ligand nach Anspruch 13, wobei die Erkrankung eine ischämische oder traumatische Verletzung am/im/des ZNS ist.

**15.** Verfahren zur Herstellung des dimeren Ligands nach einem der Ansprüche 1 bis 14, wobei das Verfahren die folgenden Schritte umfasst:

a) Herstellen eines Ns-*N*PEG-Disäure-Linkers,

b) Herstellen eines Peptids unter Verwendung einer Fmocbasierter Festphasenpeptidsynthese,
c) Dimerisierung des Peptids mit dem Ns-*N*PEG-Disäure-Linkers,
d) Koppeln einer Fettsäure an den Linker.

## Revendications

**1.** Ligand dimère de PSD-95 comprenant un premier peptide ($P_1$) et un second peptide ($P_2$), dans lequel

$P_1$ et $P_2$ comprennent individuellement au moins deux résidus d'acides aminés protéinogènes ou non protéinogènes,
$P_1$ et $P_2$ sont tous deux conjugués à un premier lieur $L_1$ par l'intermédiaire de leurs N-terminaux,
$L_1$ comprend du polyéthylène glycol (PEG) dans lequel au moins un atome d'oxygène dudit PEG est substitué par un atome d'azote pour donner un *N*PEG,

un groupe de liaison à l'albumine est lié à l'atome d'azote du *N*PEG par une liaison amide, ou par l'intermédiaire d'un lieur facultatif $L_2$, dans lequel $L_2$ comprend un atome d'azote,
le groupe de liaison à l'albumine est un acide gras (FA),
et dans lequel ledit ligand dimère a la structure générique de formule (II), dans lequel $L_2$ est facultatif :

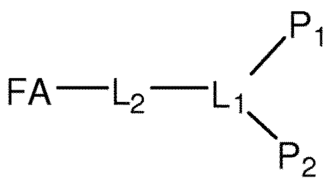

Formule (II)

ou un sel pharmaceutiquement acceptable ou un promédicament de celui-ci.

**2.** Ligand dimère selon l'une quelconque des revendications précédentes, dans lequel le second lieur $L_2$ comprend un ou plusieurs groupes choisis dans le groupe constitué du γ-Glu, de l'acide γ-butyrique (GABA), de l'acide 5-amino valérique (5-Ava), des acides aminés protéinogènes, des acides aminés non protéinogènes et de tout ligand dimère ayant la formule générale $H_2N$-[Q]-COOH, dans lequel Q représente un atome ou des atomes approprié(s).

**3.** Ligand dimère selon l'une quelconque des revendications précédentes, dans lequel ledit ligand dimère a la structure générique de formule (III) ou (IV) :

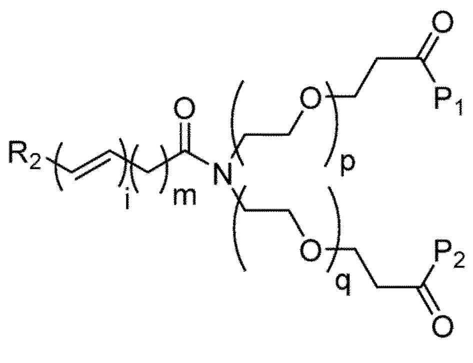

Formule (III)

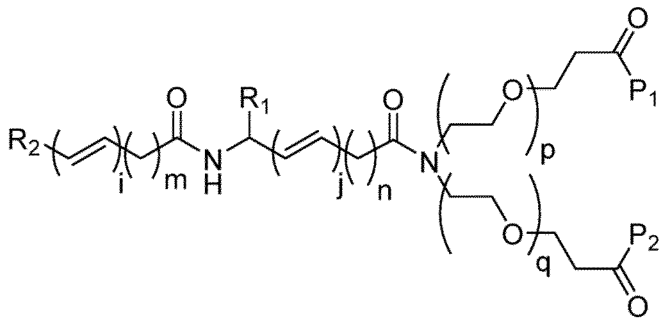

Formule (IV)

dans lequel

$R_1$ et $R_2$ sont individuellement choisis dans le groupe constitué de H et de COOH,
n représente un nombre entier de 0 à 48,
m représente un nombre entier de 1 à 48,
p représente un nombre entier de 0 à 28,
q représente un nombre entier de 0 à 28,
i représente un nombre entier de 0 à 12,
j représente un nombre entier de 0 à 12
$P_1$ et $P_2$ sont individuellement choisis parmi des peptides comprenant au moins deux résidus d'acides aminés protéinogènes ou non protéinogènes.

**4.** Ligand dimère selon l'une quelconque des revendications précédentes, dans lequel l'acide gras est un acide gras en $C_4$-$C_{22}$.

**5.** Ligand dimère selon l'une quelconque des revendications précédentes, dans lequel l'acide gras est choisi dans le groupe constitué de l'acide caprylique, l'acide caprique, l'acide laurique, l'acide myristique, l'acide palmitique, l'acide stéarique, l'acide arachidique, l'acide béhénique, l'acide lignocérique, l'acide cérotique, l'acide myristoléique, l'acide palmitoléique, l'acide sapienique, l'acide oléique, l'acide élaïdique, l'acide vaccénique, l'acide linoléique, l'acide linoélaïdique, l'acide $\alpha$-linolénique, l'acide arachidonique, l'acide eicosapentaénoïque, l'acide érucique et l'acide docosahexaénoïque.

**6.** Ligand dimère selon l'une quelconque des revendications précédentes, dans lequel

$P_1$ comprend la séquence d'acides aminés $X_4X_3X_2X_1$ (SEQ ID NO: 1), et
$P_2$ comprend la séquence d'acides aminés $Z_4Z_3Z_2Z_1$ (SEQ ID NO: 2), dans lequel
$X_1$ et/ou $Z_1$ représente un résidu d'acide aminé choisi parmi I, L et V,
$X_2$ et/ou $Z_2$ représente un résidu d'acide aminé choisi parmi A, D, E, Q, N, S, V, *N*-Me-A, *N*-Me-D, *N*-Me-E, *N*-Me-Q, *N*-Me-N, N-Me-S et *N*-Me-V,
$X_3$ et/ou $Z_3$ représente un résidu d'acide aminé choisi parmi S et T,
$X_4$ et/ou $Z_4$ représente un résidu d'acide aminé choisi parmi E, Q, A, N et S,

dans lequel $X_1$ et $Z_1$ représentent tous deux individuellement le résidu d'acide aminé C-terminal final comprenant un acide carboxylique libre.

**7.** Ligand dimère selon l'une quelconque des revendications précédentes, dans lequel ledit ligand dimère a la structure générique de formule (V) ou (VI) :

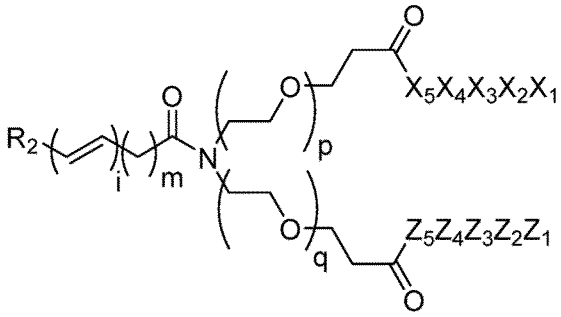

Formule (V)

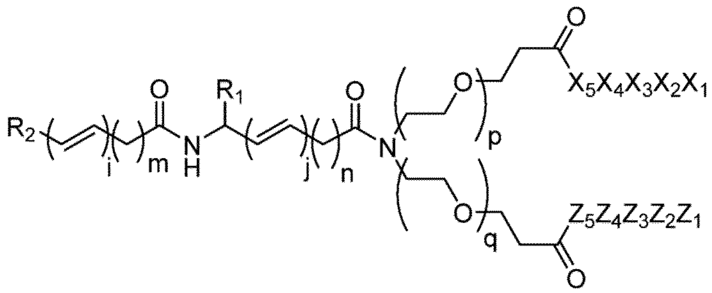

Formule (VI)

dans lequel

$R_1$ et $R_2$ sont individuellement choisis dans le groupe constitué de H et de COOH,
n représente un nombre entier de 0 à 48,
m représente un nombre entier de 1 à 48,
p représente un nombre entier de 0 à 28,
q représente un nombre entier de 0 à 28,
i représente un nombre entier de 0 à 12,
j représente un nombre entier de 0 à 12
$X_5$ et/ou $Z_5$ représente(nt) un résidu d'acide aminé protéinogène ou non protéinogène facultatif, un peptide ou un polypeptide, $X_4$ et/ou $Z_4$ représente un résidu d'acide aminé choisi parmi E, Q, A, N et S,
$X_3$ et/ou $Z_3$ représente un résidu d'acide aminé choisi parmi S et T,
$X_2$ et/ou $Z_2$ représente un résidu d'acide aminé choisi parmi A, D, E, Q, N, S, V, *N*-Me-A, *N*-Me-D, *N*-Me-E, *N*-Me-Q, *N*-Me-N, N-Me-S et *N*-Me-V
$X_1$ et/ou $Z_1$ représente un résidu d'acide aminé choisi parmi I, L et V.

**8.** Ligand dimère selon l'une quelconque des revendications précédentes, dans lequel $X_5$ représente un résidu d'acide aminé choisi dans le groupe constitué de I, A, L et V.

**9.** Ligand dimère selon l'une quelconque des revendications précédentes, dans lequel le ligand dimère est choisi dans le groupe constitué de :

a) Dérivé KBN41 **(5)**

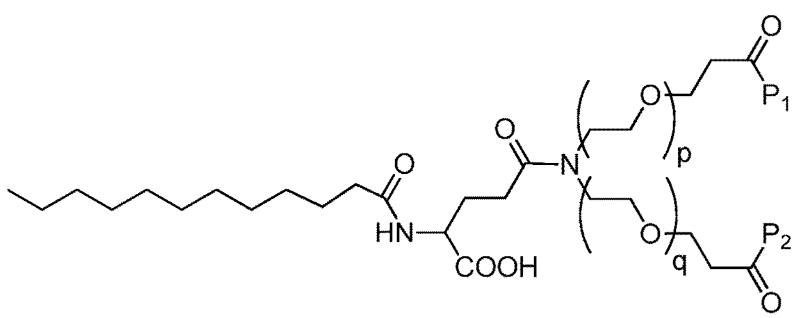

b) Dérivé KBN42 **(7)**

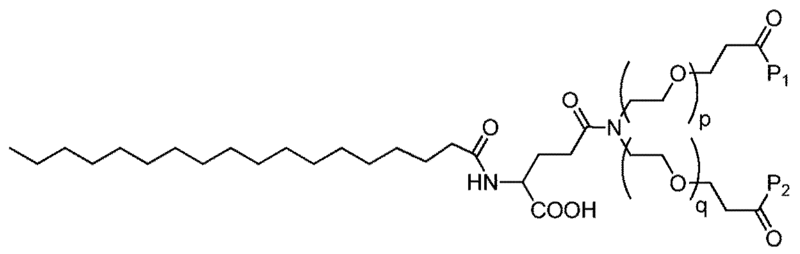

c) Dérivé KBN43 **(6)**

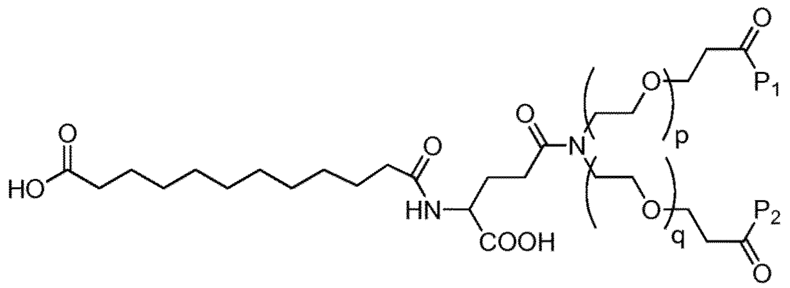

d) Dérivé KBN44 (8)

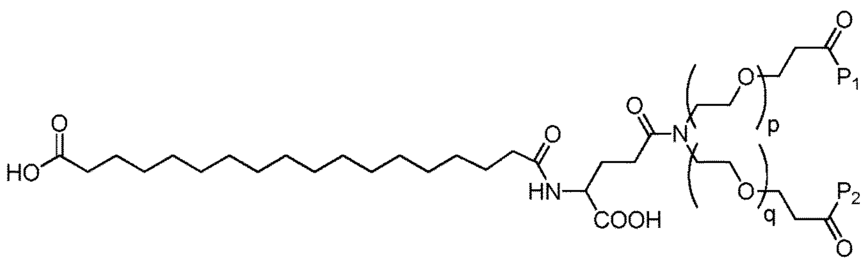

e) Dérivé KBN45 **(9)**

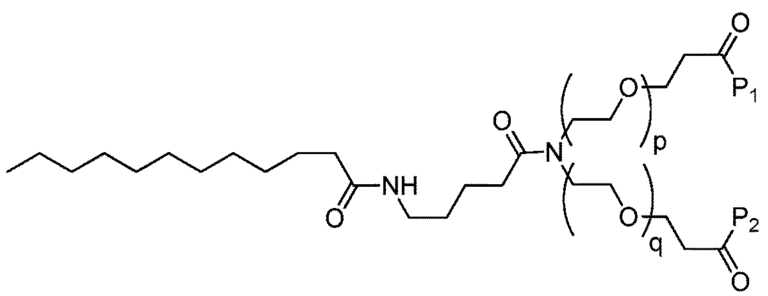

f) Dérivé KBN46 (11)

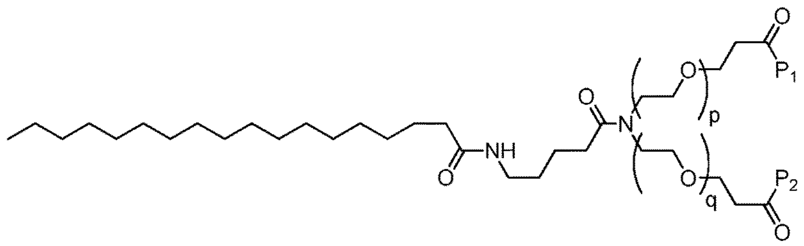

g) Dérivé KBN47 (10)

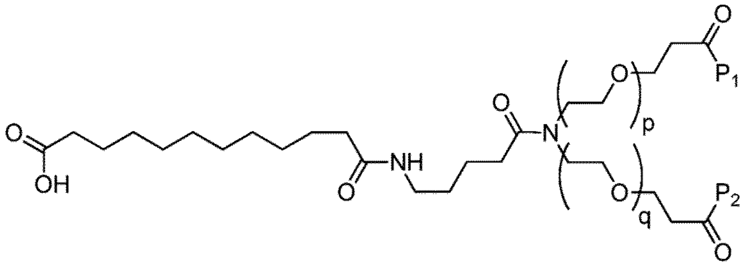

h) Dérivé KBN48 **(12)**

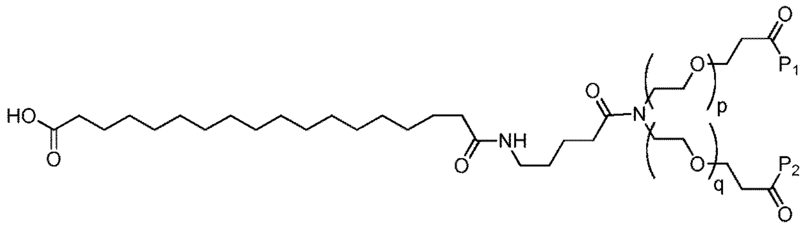

i) Dérivé KBN52 (1)

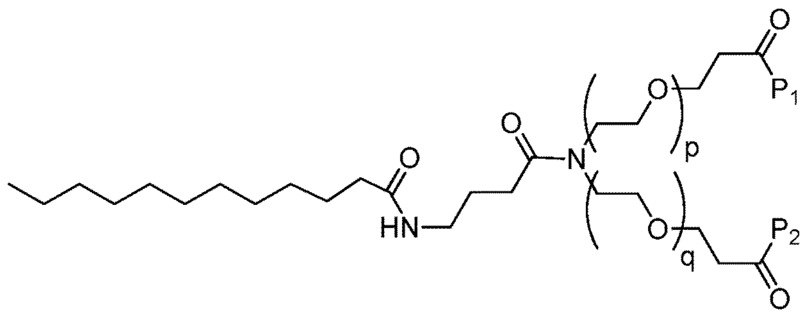

j) Dérivé KBN53 (3)

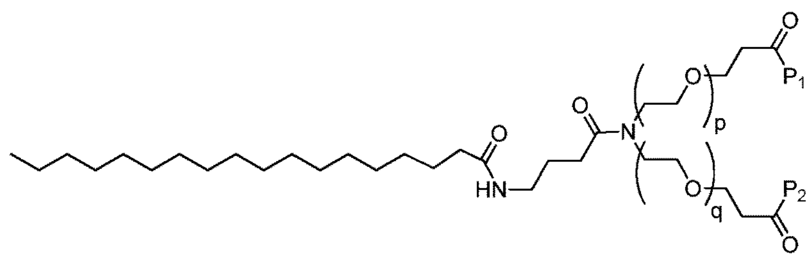

k) Dérivé KBN54 (2)

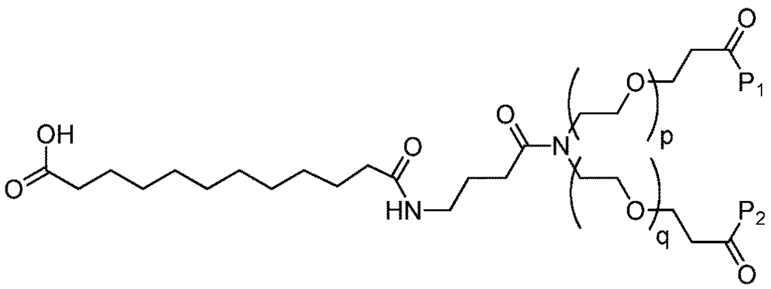

l) Dérivé KBN55 **(4)**

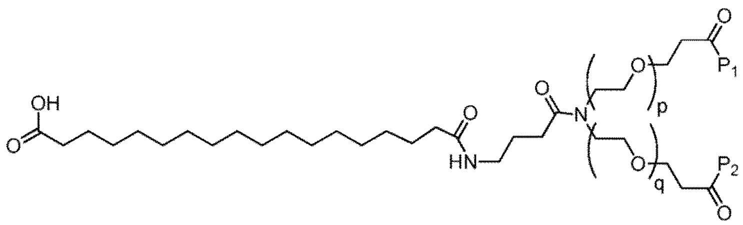

**10.** Ligand dimère selon l'une quelconque des revendications précédentes, dans lequel le ligand dimère est choisi dans le groupe constitué de :

a) Dérivé KBN41 **(5)**

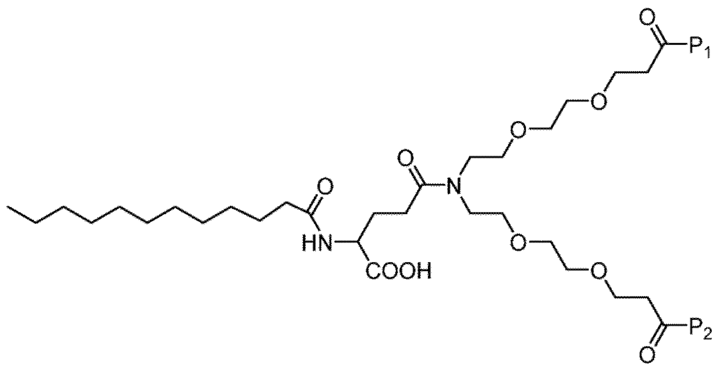

b) Dérivé KBN42 **(7)**

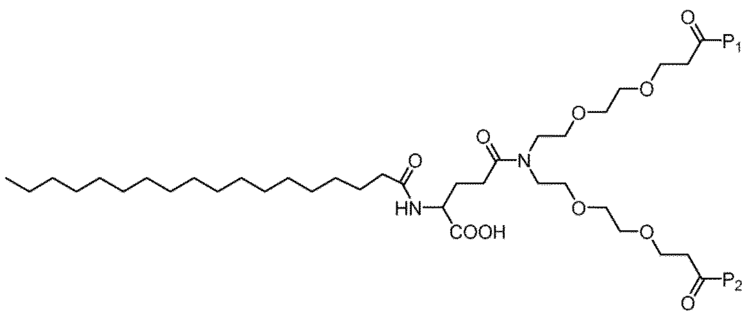

c) Dérivé KBN43 **(6)**

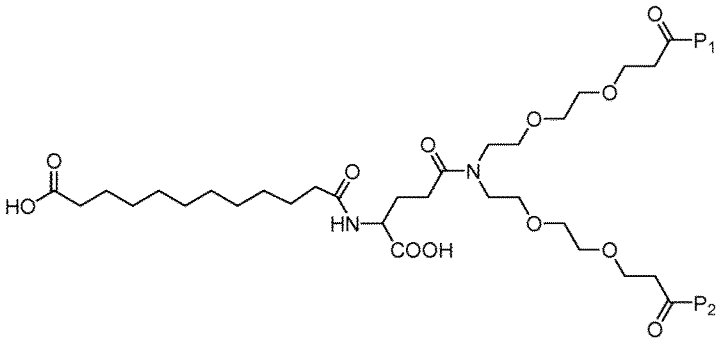

d) Dérivé KBN44 **(8)**

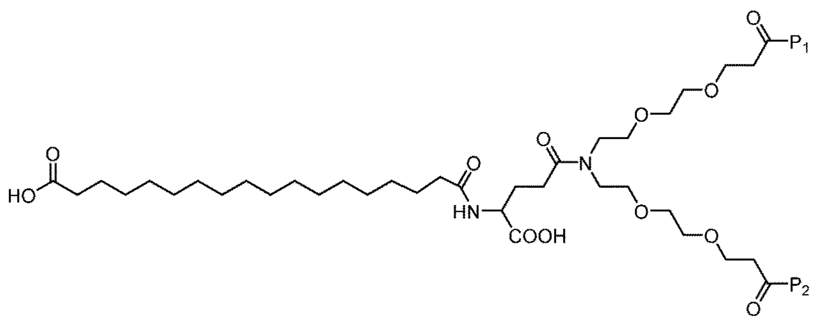

e) Dérivé KBN45 **(9)**

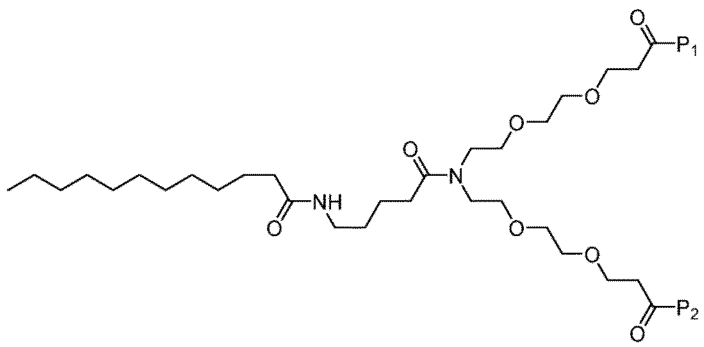

f) Dérivé KBN46 **(11)**

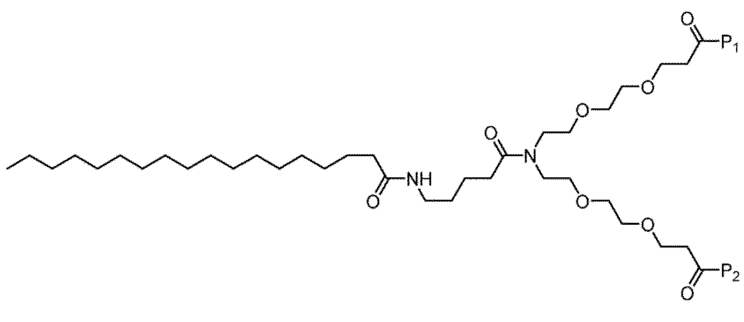

g) Dérivé KBN47 **(10)**

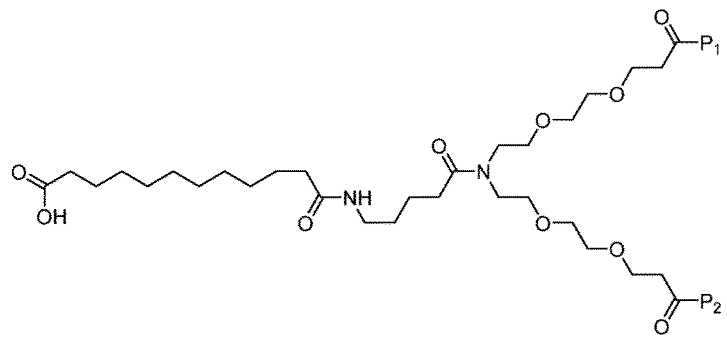

h) Dérivé KBN48 **(12)**

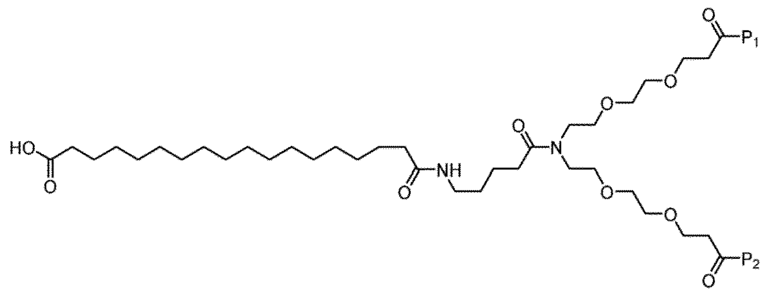

i) Dérivé KBN52 **(1)**

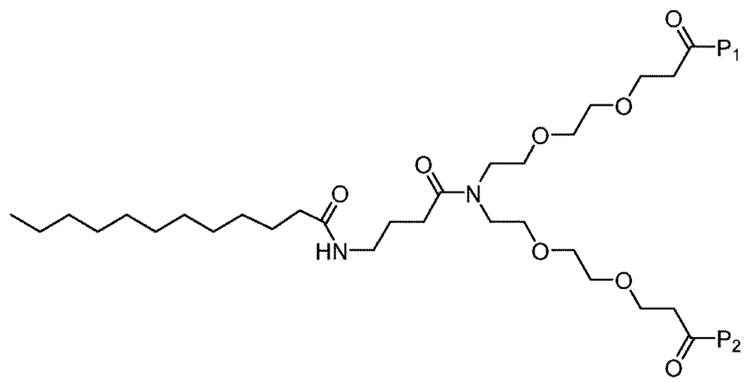

j) Dérivé KBN53 **(3)**

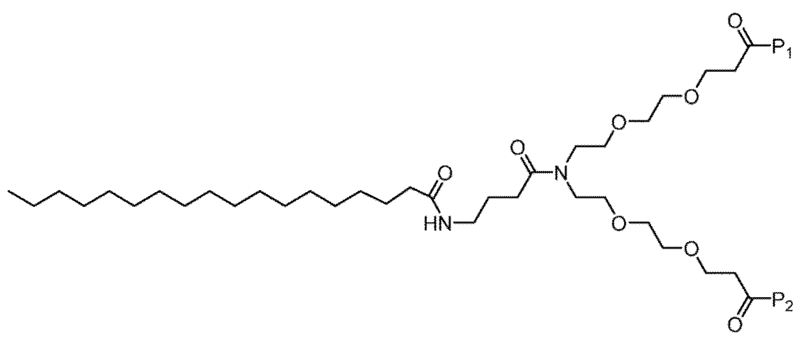

k) Dérivé KBN54 **(2)**

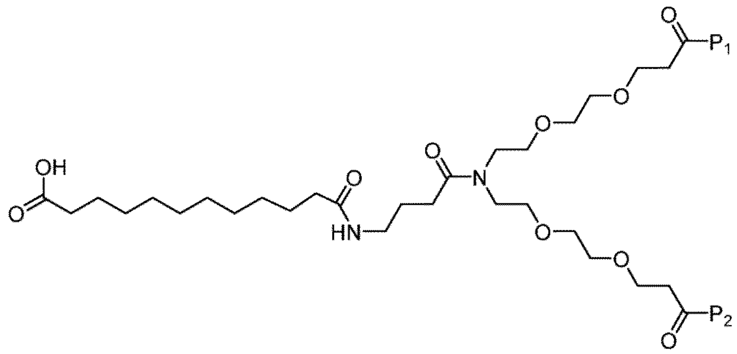

l) Dérivé KBN55 **(4)**

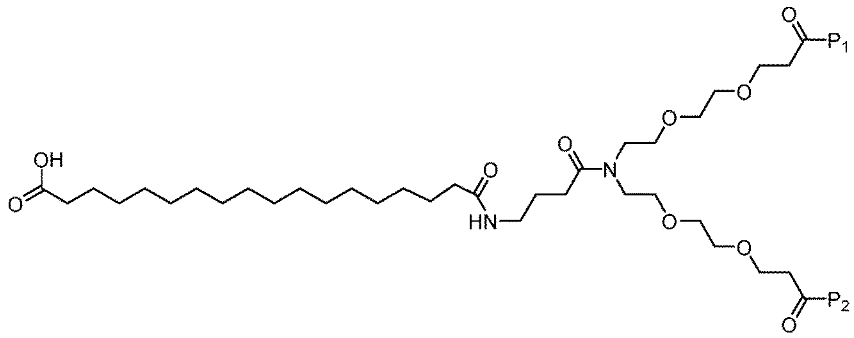

**11.** Ligand dimère selon l'une quelconque des revendications précédentes, dans lequel le ligand dimère est choisi dans le groupe constitué de :

a) KBN41 **(5)**

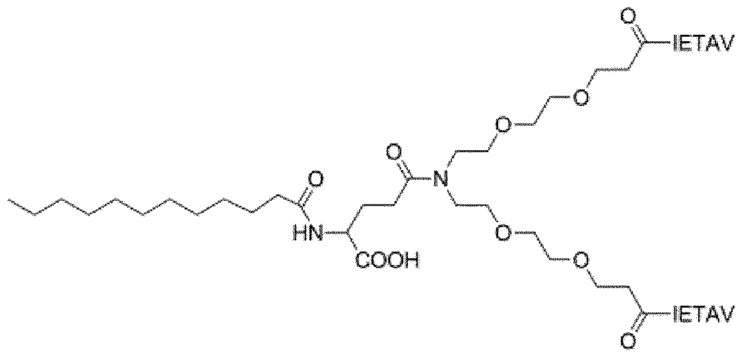

b) KBN42 **(7)**

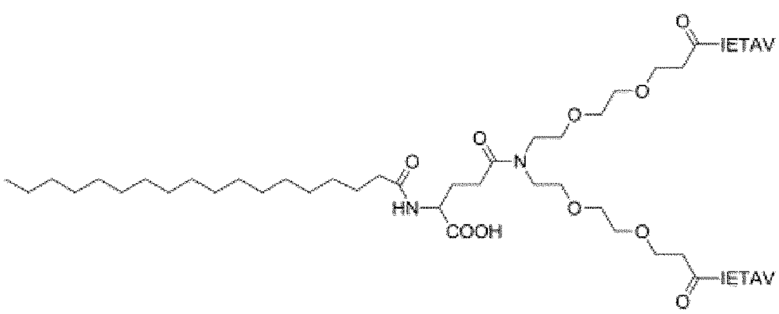

c) KBN43 **(6)**

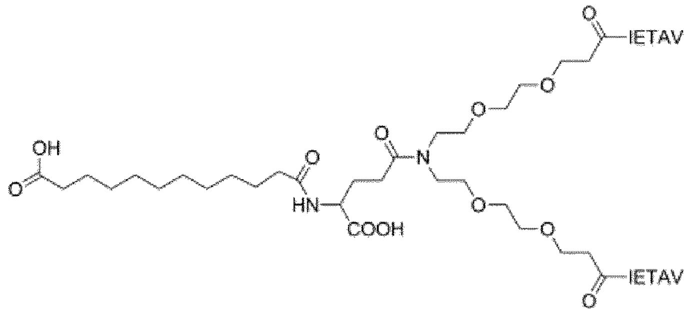

d) KBN44 **(8)**

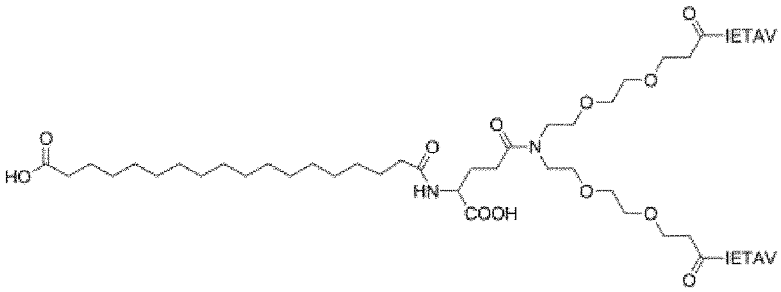

e) KBN45 **(9)**

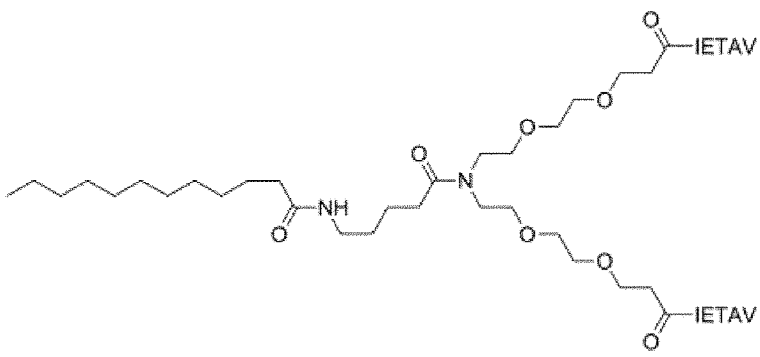

f) KBN46 **(11)**

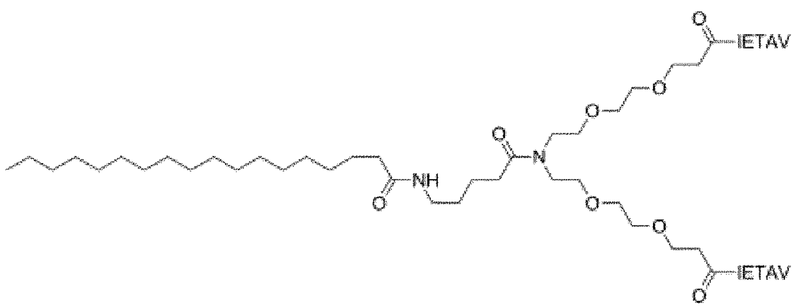

g) KBN47 **(10)**

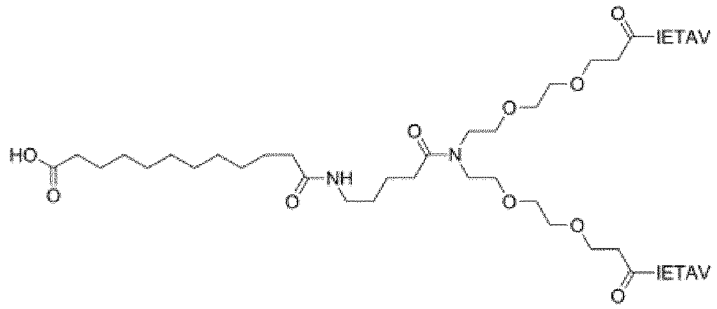

h) KBN48 **(12)**

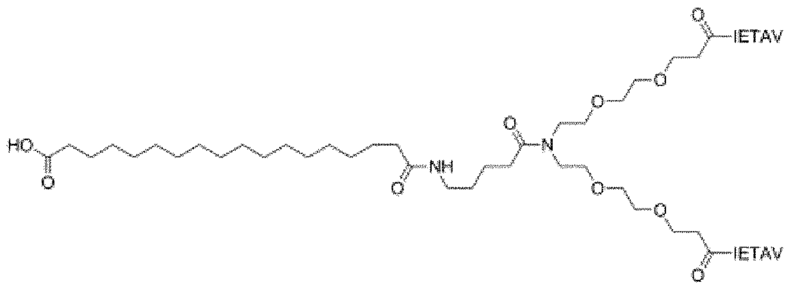

j) KBN52 **(1)**

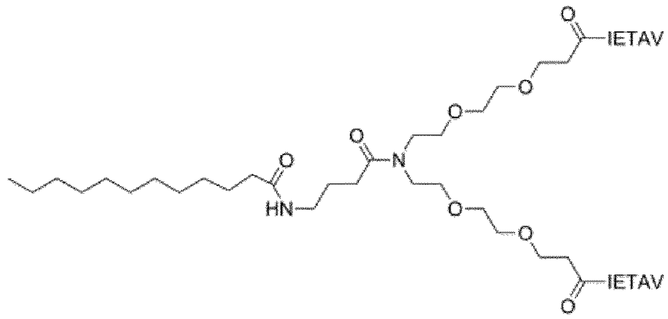

k) KBN53 **(3)**

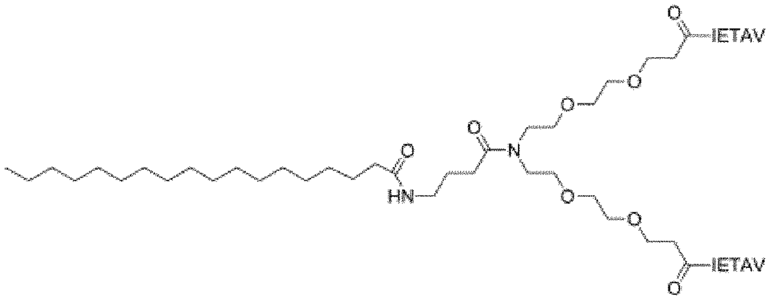

l) KBN54 **(2)**

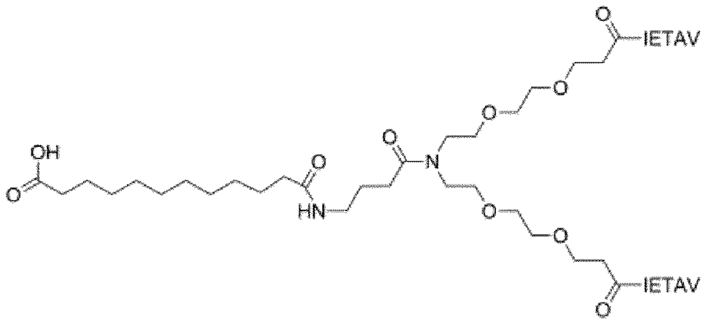

m) KBN55 **(4)**

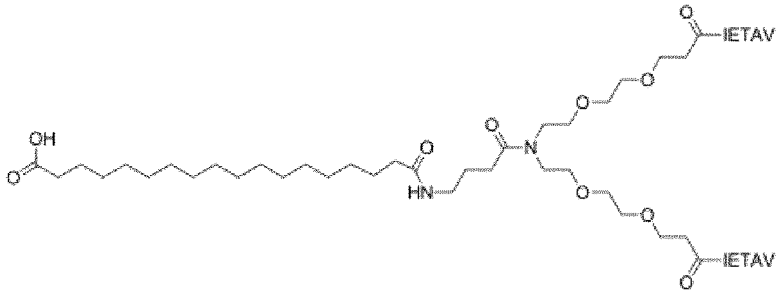

n) KBN63 **(15)**

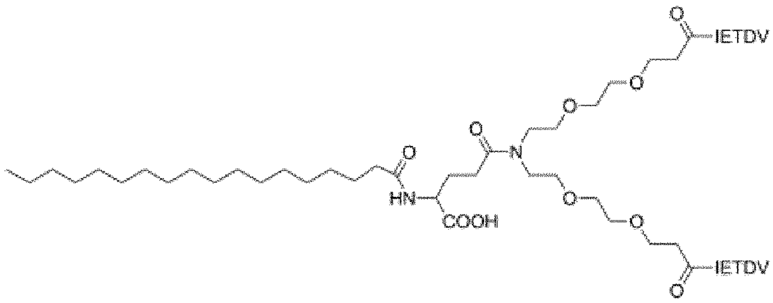

o) KBN64 **(13)**

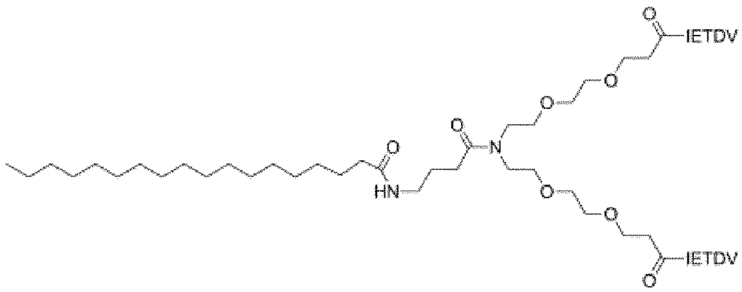

p) KBN65 **(14)**

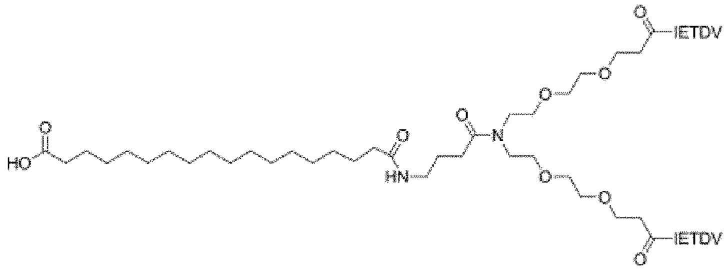

**12.** Ligand dimère selon l'une quelconque des revendications précédentes, destiné à être utilisé comme médicament.

**13.** Ligand dimère selon l'une quelconque des revendications 1 à 13, destiné à être utilisé dans le traitement ou la prophylaxie de la douleur et/ou d'une maladie liée à l'excitotoxicité.

**14.** Ligand dimère selon la revendication 13, dans lequel la maladie est une lésion ischémique ou traumatique au niveau du/dans le/du SNC.

**15.** Procédé de fabrication du ligand dimère selon l'une quelconque des revendications 1 à 14, ledit procédé comprenant les étapes de :

a) préparation d'un lieur diacide Ns-*N*PEG,
b) préparation d'un peptide en utilisant une synthèse peptidique en phase solide à base de Fmoc,
c) dimérisation dudit peptide avec ledit lieur diacide Ns-*N*PEG, et
d) couplage d'un acide gras au lieur

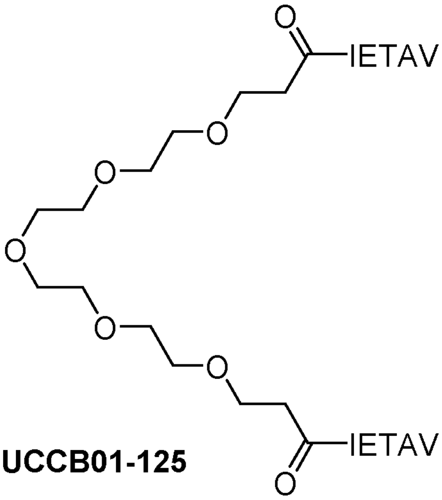

**UCCB01-125**

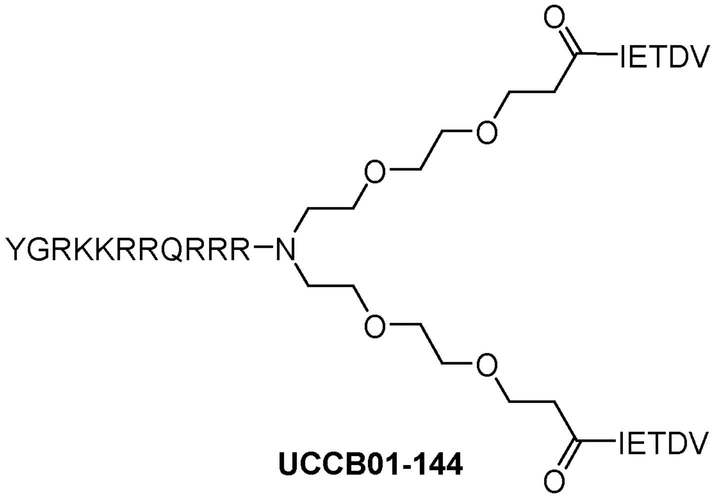

**UCCB01-144**

Fig. 1

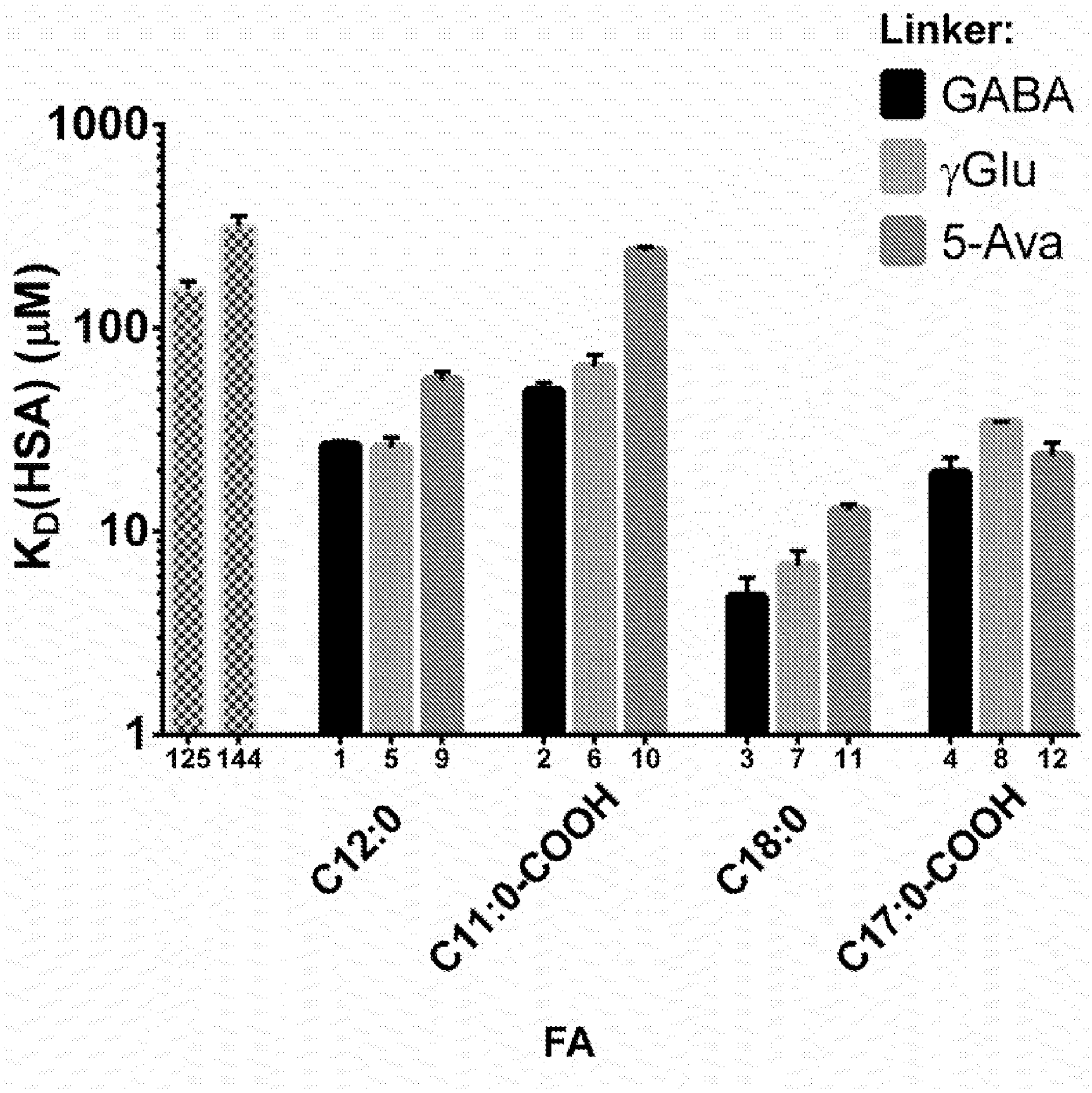
Linker:
GABA
γGlu
5-Ava
1000
100
10
1
K_D(HSA) (μM)
125
144
1
5
9
2
6
10
3
7
11
4
8
12
C12:0
C11:0-COOH
C18:0
C17:0-COOH
FA

Fig. 2

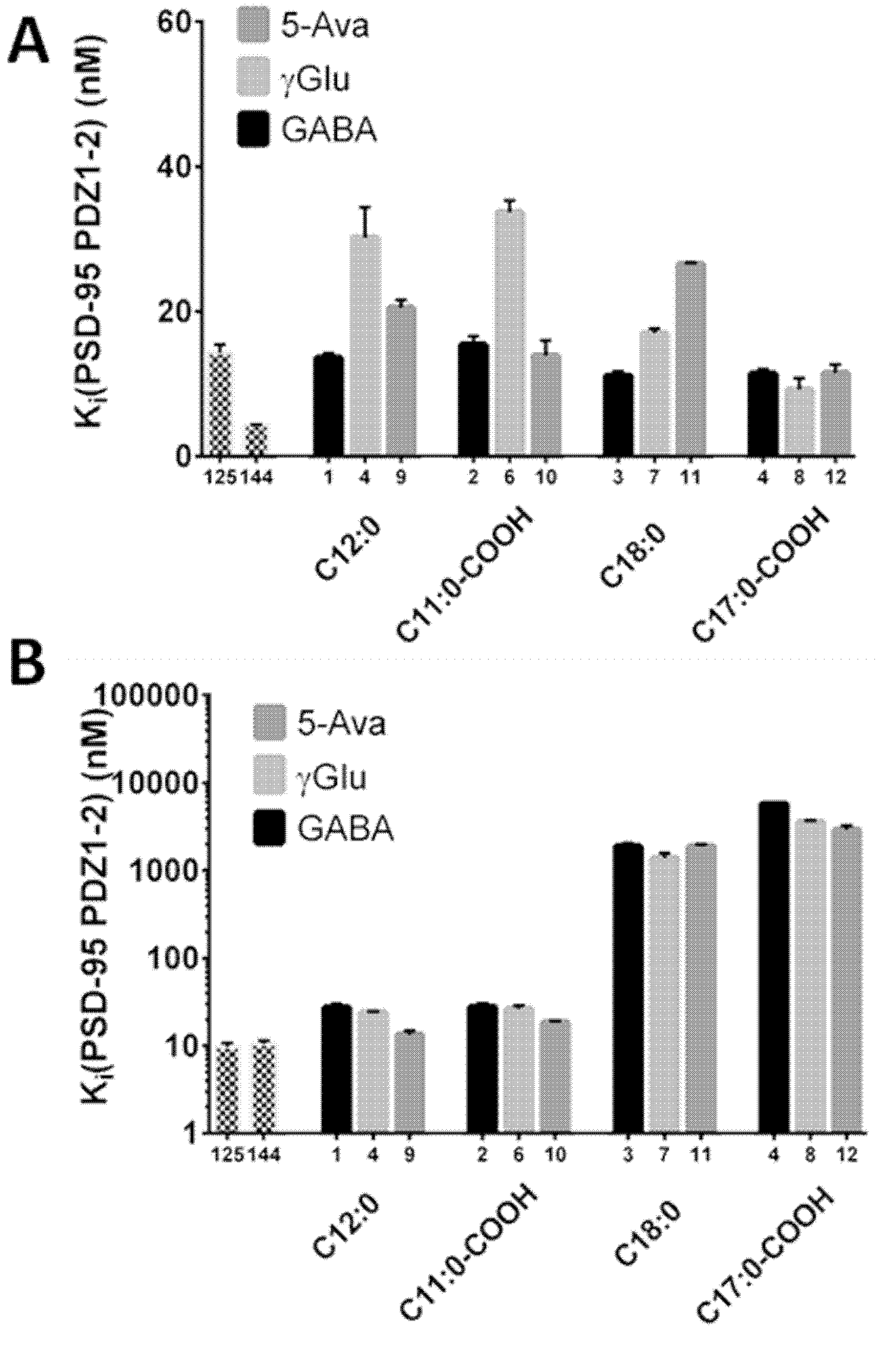
A
60
40
20
0
5-Ava
γGlu
GABA
$K_i$(PSD-95 PDZ1-2) (nM)
125 144
1 4 9
2 6 10
3 7 11
4 8 12
C12:0
C11:0-COOH
C18:0
C17:0-COOH
B
100000
10000
1000
100
10
1
5-Ava
γGlu
GABA
$K_i$(PSD-95 PDZ1-2) (nM)
125 144
1 4 9
2 6 10
3 7 11
4 8 12
C12:0
C11:0-COOH
C18:0
C17:0-COOH

Fig. 3

C

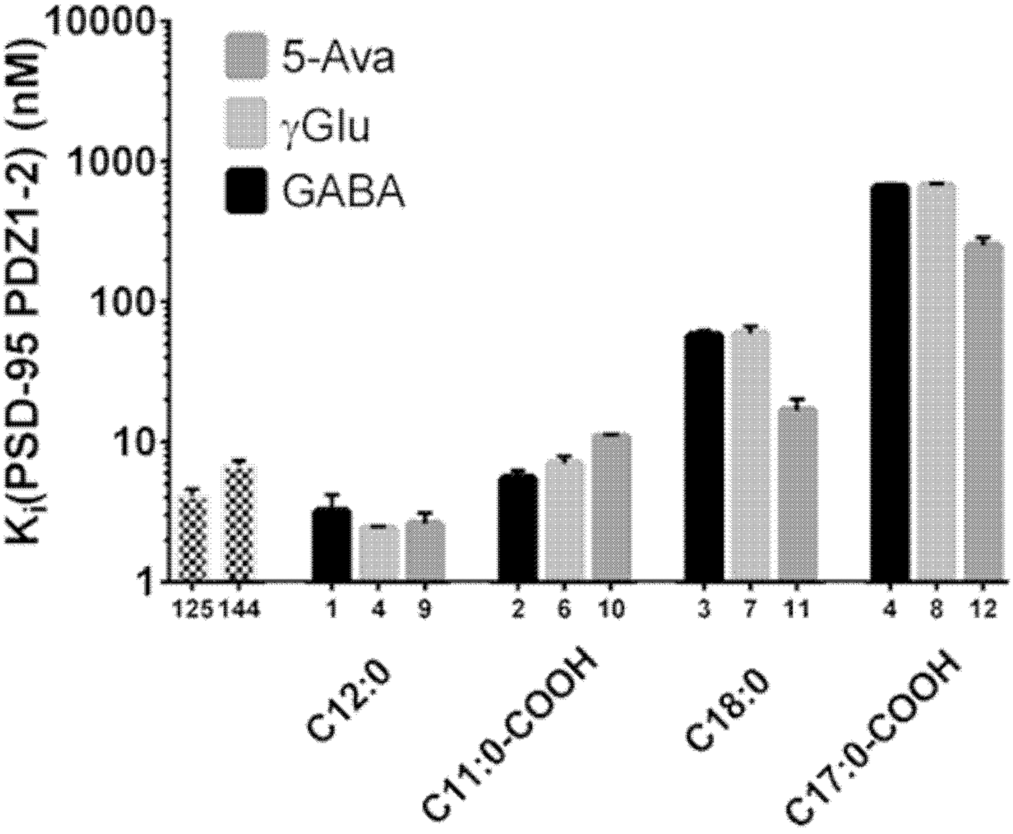
10000
1000
100
10
1
$K_i$(PSD-95 PDZ1-2) (nM)
5-Ava
γGlu
GABA
125
144
1
4
9
2
6
10
3
7
11
4
8
12
C12:0
C11:0-COOH
C18:0
C17:0-COOH

Fig. 3, cont.

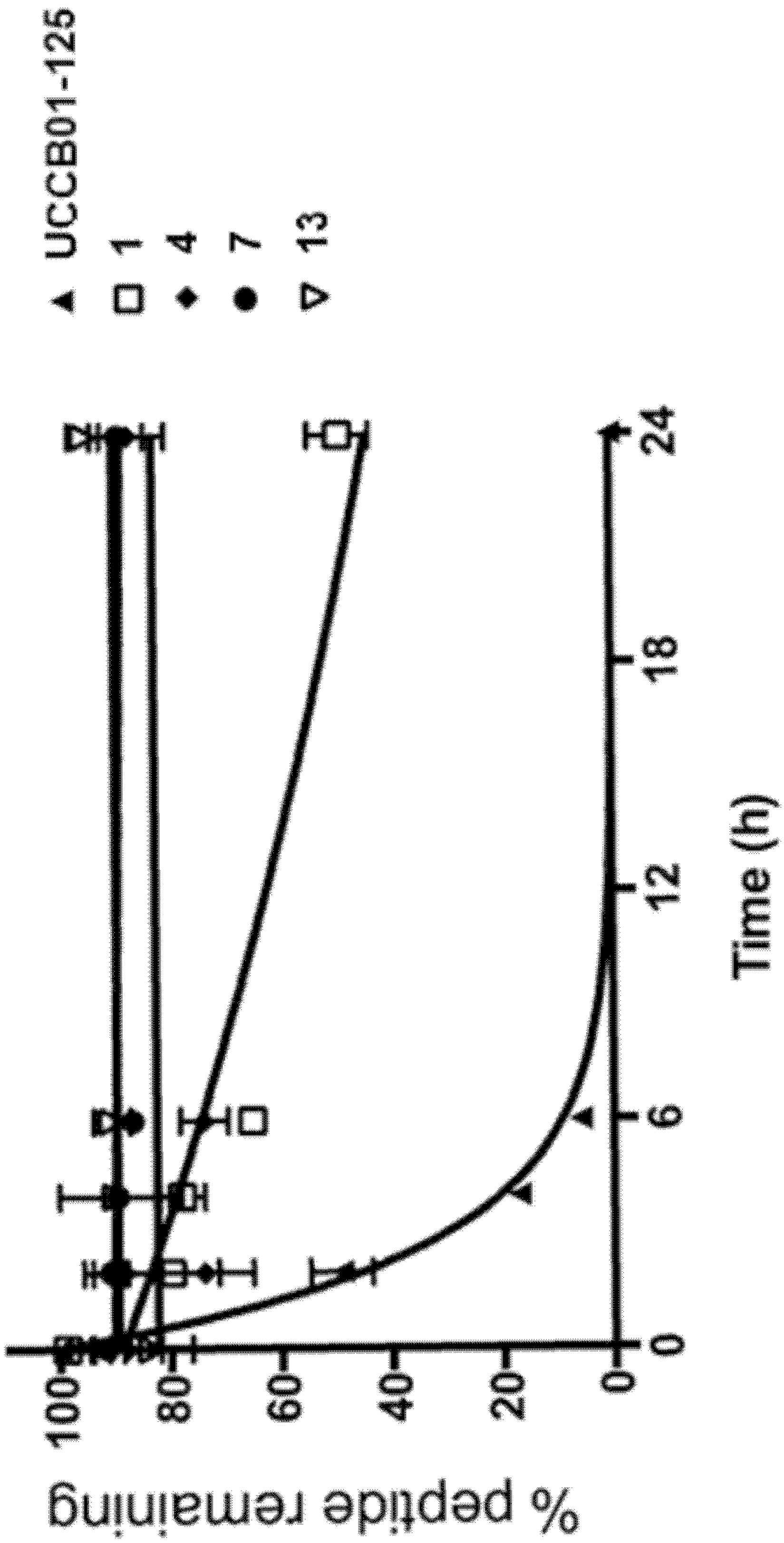
UCCB01-125
1
4
7
13
% peptide remaining
100
80
60
40
20
0
0
6
12
18
24
Time (h)

Fig. 4

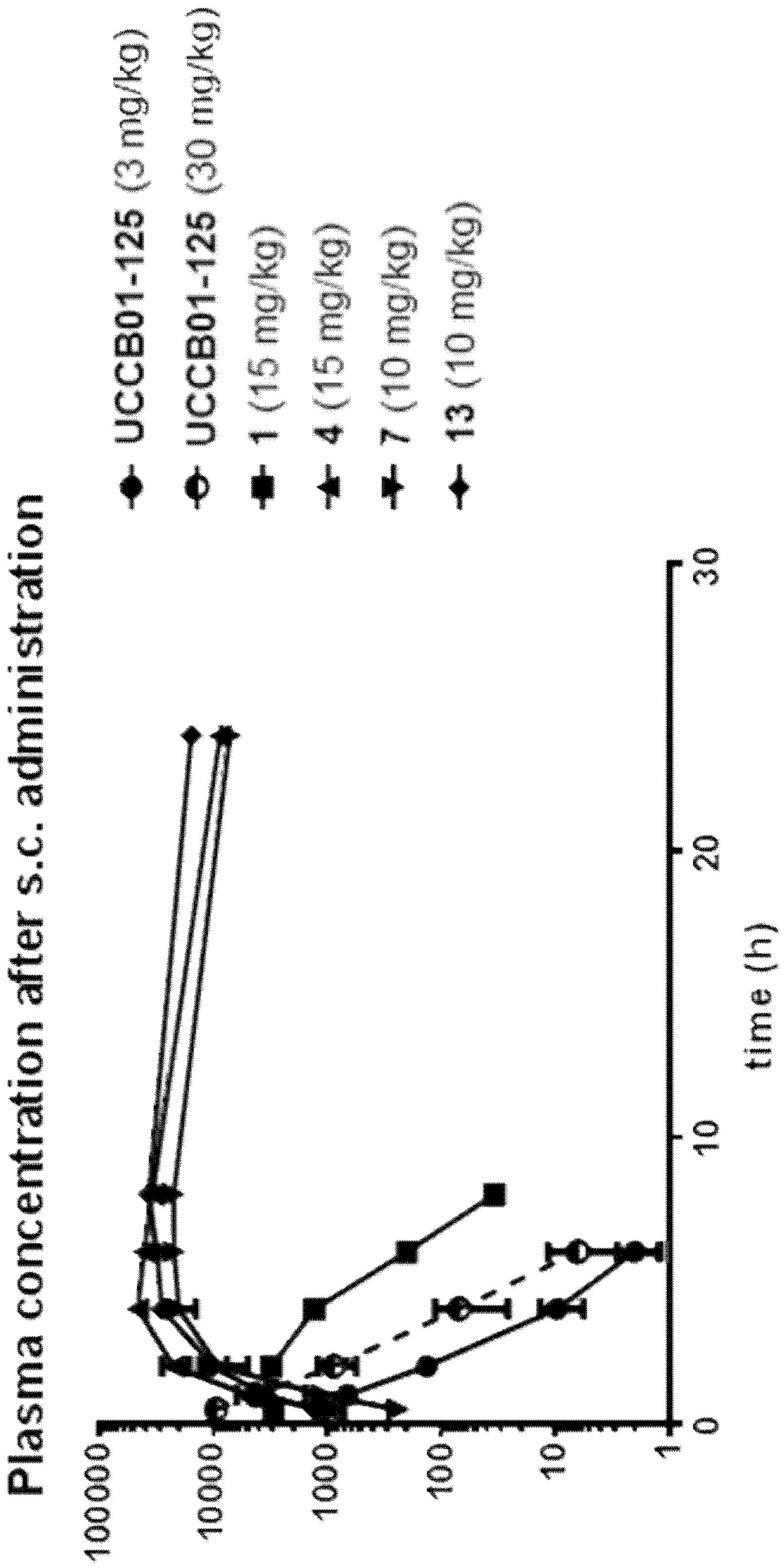
Plasma concentration after s.c. administration
c (ng/mL)
100000
10000
1000
100
10
1
0
10
20
30
time (h)
UCCB01-125 (3 mg/kg)
UCCB01-125 (30 mg/kg)
1 (15 mg/kg)
4 (15 mg/kg)
7 (10 mg/kg)
13 (10 mg/kg)

Fig. 5

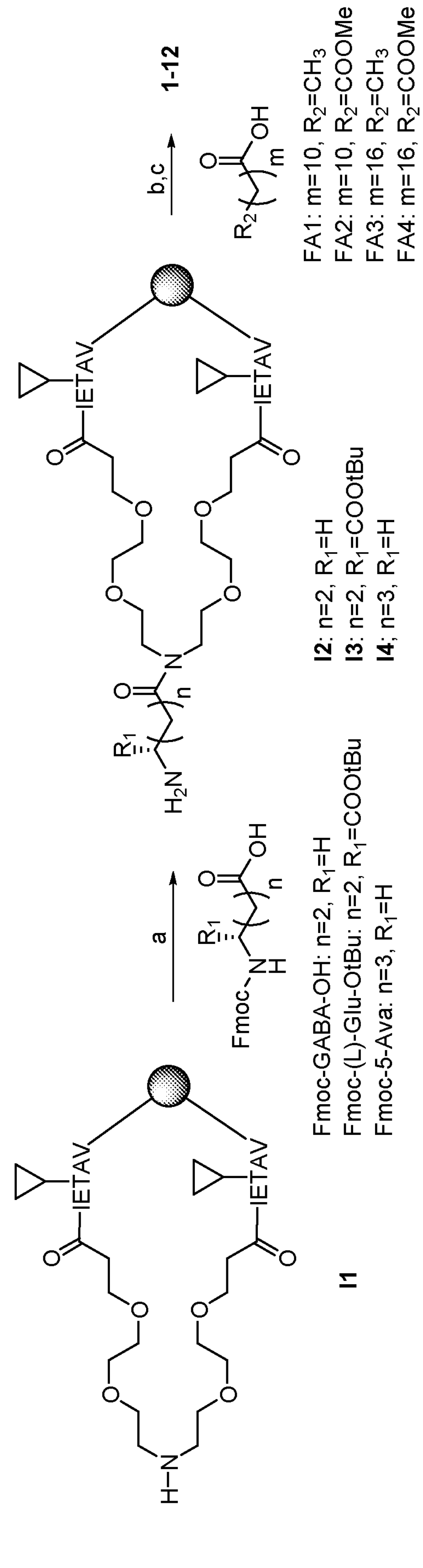
I1
a
Fmoc-GABA-OH: n=2, $R_1$=H
Fmoc-(L)-Glu-OtBu: n=2, $R_1$=COOtBu
Fmoc-5-Ava: n=3, $R_1$=H
**I2**: n=2, $R_1$=H
**I3**: n=2, $R_1$=COOtBu
**I4**, n=3, $R_1$=H
b,c
1-12
FA1: m=10, $R_2$=$CH_3$
FA2: m=10, $R_2$=COOMe
FA3: m=16, $R_2$=$CH_3$
FA4: m=16, $R_2$=COOMe

Fig. 6

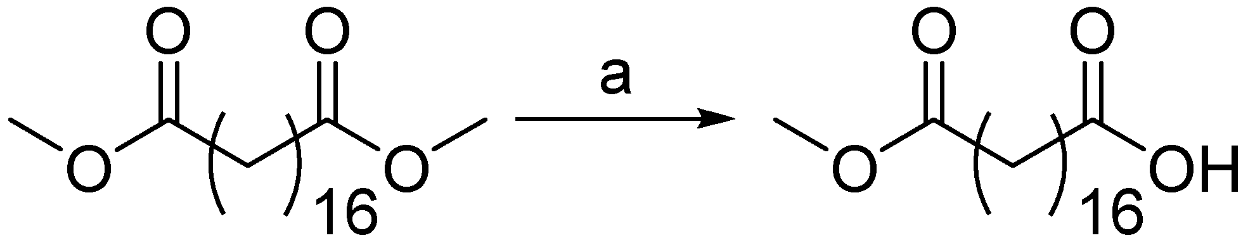


**FA4**

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010004003 A
- WO 2012156308 A


### Non-patent literature cited in the description


- **LIM et al.** *Journal of Controlled Release,* 2013, vol. 170 (2), 219-225
- **ANDREASEN et al.** *Neuropharmacol,* 2013, vol. 67, 193-200
- **BACH et al.** *PNAS USA,* 2012, vol. 109, 3317-3322
- **TANG et al.** *J Pharm Sci,* 2004, vol. 93, 2184-204
- **DENNIS et al.** *J Biol Chem,* 2002, vol. 277, 35035-43
- **CLAXTON et al.** *Clin Ther,* 2001, vol. 23, 1296-310
- **DYCHTER et al.** *J Infus Nurs,* 2012, vol. 35, 154-60
- **HAVELUND et al.** *Pharm Res,* 2004, vol. 21, 1498-504
- **ELSADEK et al.** *J Control Release,* 2012, vol. 157, 4-28
- **KRAGH-HANSEN et al.** *Biol Pharm Bull,* 2002, vol. 25, 695-704
- **SLEEP et al.** *Biochim Biophys Acta,* 2013, vol. 1830, 5526-34
- **LØBERG.** *APMIS,* 1993, vol. 101, 777-83
- **SIMARD et al.** *PNAS USA,* 2005, vol. 102, 17958-63
- **YAMASAKI et al.** *Biochim Biophys Acta,* 2013, vol. 1830, 5435-43
- **SUGIO et al.** *Protein Eng,* 1999, vol. 12, 439-46
- **SUDLOW et al.** *Mol Pharmacol,* 1975, vol. 11, 824-32
- **JONSSON et al.** *Protein Eng Des Sel,* 2008, vol. 21, 515-27
- **LIPOVSEK et al.** *Protein Eng Des Sel,* 2011, vol. 24, 3-9
- **JONASSEN et al.** *Pharm Res,* 2012, vol. 29, 2104-2114
- **BACH et al.** *Angew. Chem, Int. Ed,* 2009, vol. 48, 9685-9689
- **HACKETT et al.** *Adv Drug Deliv Rev,* 2013, vol. 65, 1331-1339
- **NIKOLOVSKA-COLESKA et al.** *Anal Biochem,* 2004, vol. 332, 261-273
- **BEREZHKOVSKIY et al.** *J Pharm Sci,* 2007, vol. 96, 249-257